(19) Europäisches Patentamt
European Patent Office
Office européen des brevets

(11) **EP 1 527 341 B1**

(12) **EUROPEAN PATENT SPECIFICATION**

(45) Date of publication and mention
of the grant of the patent:
**17.06.2009   Bulletin 2009/25**

(51) Int Cl.:
***G01N 33/543*** (2006.01)

(21) Application number: **02806542.3**

(22) Date of filing: **20.11.2002**

(86) International application number:
**PCT/US2002/037414**

(87) International publication number:
**WO 2003/072542 (04.09.2003 Gazette 2003/36)**

(54) **INTERFACIAL BIOMATERIALS**

GRENZFLÄCHEN-BIOMATERIALIEN

BIOMATERIAUX INTERFACIAUX

(84) Designated Contracting States:
**AT BE BG CH CY CZ DE DK EE ES FI FR GB GR IE IT LI LU MC NL PT SE SK TR**

(30) Priority:  **20.11.2001  US 331843 P**

(43) Date of publication of application:
**04.05.2005  Bulletin 2005/18**

(73) Proprietor: **Duke University
Durham, NC 27708 (US)**

(72) Inventors:
• **GRINSTAFF, Mark, W.
Durham, NC 27707 (US)**
• **KENAN, Daniel, J.
Chapel Hill, NC 27514 (US)**
• **WALSH, Elisabeth, B.
Durham, NC 27705 (US)**
• **MIDDLETON, Crystan
Carrboro, NC 27510 (US)**

(74) Representative: **Naylor, Kathryn May
Mathys & Squire LLP
120 Holborn
London
EC1N 2SQ (GB)**

(56) References cited:
**US-A- 5 019 393          US-A- 5 217 492
US-B1- 6 780 582**

• **HYNES G M ET AL: "Individual subunits of the eukaryotic cytosolic chaperonin mediate interactions with binding sites located on subdomains of beta-actin." THE JOURNAL OF BIOLOGICAL CHEMISTRY. 23 JUN 2000, vol. 275, no. 25, 23 June 2000 (2000-06-23), pages 18985-18994, XP002376183 ISSN: 0021-9258**
• **DILLOW A.K. ET AL: 'Adhesion of [alpha]5[beta]1 receptors to biomimetic substrates constructed from peptide amphiphiles' BIOMATERIALS vol. 22, 15 June 2001, pages 1493 - 1505, XP004245884**
• **ADEY N.B. ET AL: 'Characterization of phage that bind plastic from phage-displayed random peptide libraries' GENE vol. 156, 1995, pages 27 - 31**
• **BROWN S.: 'Metal-recognition by repeating polypeptides' NATURE BIOTECHNOLOGY vol. 15, March 1997, pages 269 - 272**
• **GEBHARDT K. ET AL: 'ADHESIVE PEPTIDES SELECTED BY PHAGE DISPLAY: CHARACTERIZATION, APPLICATIONS AND SIMILARITIES WITH FIBRINOGEN' PEPTIDE RESEARCH vol. 9, 1996, pages 269 - 278**
• **GASKIN DUNCAN J.H. ET AL: 'Identification of inorganic crystal-specific sequences using phage display combinatorial library of short peptides: A feasibility study' BIOTECHNOLOGY LETTERS vol. 22, 2000, page 1216**

SEQUENCE LISTING

<110> Duke University
Grinstaff, Mark W.
Kenan, Daniel J.
Walsh, Elisabeth B.

Middleton, Crystan

<120> INTERFACIAL BIOMATERIALS

<130> 180/143/2

<150> US 60/331,843
<151> 2001-11-20

<160> 117

<170> PatentIn version 3.1

<210> 1
<211> 13
<212> PRT
<213> synthetic construct

<400> 1

```
Phe Leu Ser Phe Val Phe Pro Ala Ser Ala Trp Gly Gly
1               5                   10
```

<210> 2
<211> 13
<212> PRT
<213> synthetic construct

<400> 2

```
Phe Tyr Met Pro Phe Gly Pro Thr Trp Trp Gln His Val
1               5                   10
```

<210> 3
<211> 13
<212> PRT
<213> synthetic construct

<400> 3

```
Leu Phe Ser Trp Phe Leu Pro Thr Asp Asn Tyr Pro Val
1               5                   10
```

<210> 4
<211> 13
<212> PRT
<213> synthetic construct

<400> 4

```
Phe Met Asp Ile Trp Ser Pro Trp His Leu Leu Gly Thr
1               5                   10
```

<210> 5

<211> 13
<212> PRT
<213> synthetic construct

<400> 5

```
        Phe Ser Ser Leu Phe Phe Pro His Trp Pro Ala Gln Leu
        1               5               10
```

<210> 6
<211> 19
<212> PRT
<213> synthetic construct

<400> 6

```
        Ser Cys Ala Met Ala Gln Trp Phe Cys Asp Arg Ala Glu Pro His His
        1               5               10              15

        Val Ile Ser
```

<210> 7
<211> 19
<212> PRT
<213> synthetic construct

<400> 7

```
        Ser Cys Asn Met Ser His Leu Thr Gly Val Ser Leu Cys Asp Ser Leu
        1               5               10              15

        Ala Thr Ser
```

<210> 8
<211> 19
<212> PRT
<213> synthetic construct

<400> 8

```
        Ser Cys Val Tyr Ser Phe Ile Asp Gly Ser Gly Cys Asn Ser His Ser
        1               5               10              15

        Leu Gly Ser
```

<210> 9
<211> 19
<212> PRT
<213> synthetic construct

<400> 9

```
        Ser Cys Ser Gly Phe His Leu Leu Cys Glu Ser Arg Ser Met Gln Arg
        1               5                   10                  15

        Glu Leu Ser
```

<210> 10
<211> 19
<212> PRT
<213> synthetic construct

<400> 10

```
        Ser Cys Gly Ile Leu Cys Ser Ala Phe Pro Phe Asn Asn His Gln Val
        1               5                   10                  15

        Gly Ala Ser
```

<210> 11
<211> 19
<212> PRT
<213> synthetic construct

<400> 11

```
        Ser Cys Cys Ser Met Phe Phe Lys Asn Val Ser Tyr Val Gly Ala Ser
        1               5                   10                  15

        Asn Pro Ser
```

<210> 12
<211> 19
<212> PRT
<213> synthetic construct

<400> 12

```
        Ser Cys Pro Ile Trp Lys Tyr Cys Asp Asp Tyr Ser Arg Ser Gly Ser
        1               5                   10                  15

        Ile Phe Ser
```

<210> 13
<211> 18
<212> PRT
<213> synthetic construct

<400> 13

```
        Ser Cys Leu Phe Asn Ser Met Lys Cys Leu Val Leu Ile Leu Cys Phe
        1               5                   10                  15

        Val Ser
```

<210> 14
<211> 19
<212> PRT
<213> synthetic construct

<400> 14

```
Ser Cys Tyr Val Asn Gly His Asn Ser Val Trp Val Val Val Phe Trp
1               5                   10                  15

Gly Val Ser
```

<210> 15
<211> 19
<212> PRT
<213> synthetic construct

<400> 15

```
Ser Cys Asp Phe Val Cys Asn Val Leu Phe Asn Val Asn His Gly Ser
1               5                   10                  15

Asn Met Ser
```

<210> 16
<211> 19
<212> PRT
<213> synthetic construct

<400> 16

```
Ser Cys Leu Asn Lys Phe Phe Val Leu Met Ser Val Gly Leu Arg Ser
1               5                   10                  15

Tyr Thr Ser
```

<210> 17
<211> 19
<212> PRT
<213> synthetic construct

<400> 17

```
Ser Cys Cys Asn His Asn Ser Thr Ser Val Lys Asp Val Gln Phe Pro
1               5                   10                  15

Thr Leu Ser
```

<210> 18
<211> 13
<212> PRT
<213> synthetic construct

<220>

<221> misc feature
<222> (13)..(13)
<223> residue 13 (leucine) can optionally have a polyethylene glycol mo iety attached

<400> 18

```
Phe Phe Pro Ser Ser Trp Tyr Ser His Leu Gly Val Leu
1               5                   10
```

<210> 19
<211> 13
<212> PRT
<213> synthetic construct

<400> 19

```
Phe Phe Gly Phe Asp Val Tyr Asp Met Ser Asn Ala Leu
1               5                   10
```

<210> 20
<211> 13
<212> PRT
<213> synthetic construct

<400> 20

```
Leu Ser Phe Ser Asp Phe Tyr Phe Ser Glu Gly Ser Glu
1               5                   10
```

<210> 21
<211> 13
<212> PRT
<213> synthetic construct

<400> 21

```
Phe Ser Tyr Ser Val Ser Tyr Ala His Pro Glu Gly Leu
1               5                   10
```

<210> 22
<211> 13
<212> PRT
<213> synthetic construct

<400> 22

```
Leu Pro His Leu Ile Gln Tyr Arg Val Leu Leu Val Ser
1               5                   10
```

<210> 23
<211> 19
<212> PRT
<213> synthetic construct

<400> 23

```
        Ser Cys Tyr Val Asn Gly His Asn Ser Val Trp Val Val Val Phe Trp
        1                   5                   10                  15

        Gly Val Ser
```

<210> 24
<211> 19
<212> PRT
<213> synthetic construct

<400> 24

```
        Ser Cys Phe Trp Phe Leu Arg Trp Ser Leu Phe Ile Val Leu Phe Thr
        1                   5                   10                  15

        Cys Cys Ser
```

<210> 25
<211> 19
<212> PRT
<213> synthetic construct

<400> 25

```
        Ser Cys Glu Ser Val Asp Cys Phe Ala Asp Ser Arg Met Ala Lys Val
        1                   5                   10                  15

        Ser Met Ser
```

<210> 26
<211> 19
<212> PRT
<213> synthetic construct

<400> 26

```
        Ser Cys Val Gly Phe Phe Cys Ile Thr Gly Ser Asp Val Ala Ser Val
        1                   5                   10                  15

        Asn Ser Ser
```

<210> 27
<211> 19
<212> PRT
<213> synthetic construct

<400> 27

```
Ser Cys Ser Asp Cys Leu Lys Ser Val Asp Phe Ile Pro Ser Ser Leu
1               5                   10                  15

Ala Ser Ser
```

<210> 28
<211> 19
<212> PRT
<213> synthetic construct

<400> 28

```
Ser Cys Ala Phe Asp Cys Pro Ser Ser Val Ala Arg Ser Pro Gly Glu
1               5                   10                  15

Trp Ser Ser
```

<210> 29
<211> 18
<212> PRT
<213> synthetic construct

<400> 29

```
Ser Cys Val Asp Val Met His Ala Asp Ser Pro Gly Pro Asp Gly Leu
1               5                   10                  15

Asn Ser
```

<210> 30
<211> 19
<212> PRT
<213> synthetic construct

<400> 30

```
Ser Cys Ser Ser Phe Glu Val Ser Glu Met Phe Thr Cys Ala Val Ser
1               5                   10                  15

Ser Tyr Ser
```

<210> 31
<211> 19
<212> PRT
<213> synthetic construct

<400> 31

```
Ser Cys Gly Leu Asn Phe Pro Leu Cys Ser Phe Val Asp Phe Ala Gln
1               5                   10                  15

Asp Ala Ser
```

<210> 32
<211> 19
<212> PRT
<213> synthetic construct

<400> 32

```
Ser Cys Met Leu Phe Ser Ser Val Phe Asp Cys Gly Met Leu Ile Ser
1               5                   10                  15

Asp Leu Ser
```

<210> 33
<211> 19
<212> PRT
<213> synthetic construct

<400> 33

```
Ser Cys Val Asp Tyr Val Met His Ala Asp Ser Pro Gly Pro Asp Gly
1               5                   10                  15

Leu Asn Ser
```

<210> 34
<211> 19
<212> PRT
<213> synthetic construct

<400> 34

```
Ser Cys Ser Glu Asn Phe Met Phe Asn Met Tyr Gly Thr Gly Val Cys
1               5                   10                  15

Thr Glu Ser
```

<210> 35
<211> 19
<212> PRT
<213> synthetic construct

<400> 35

```
Ser Cys Ser Ser Phe Glu Val Ser Glu Met Phe Thr Cys Ala Val Ser
1               5                   10                  15

Ser Tyr Ser
```

<210> 36
<211> 19
<212> PRT
<213> synthetic construct

<400> 36

Ser Cys Gly Leu Asn Phe Pro Leu Cys Ser Phe Val Asp Phe Ala Gln
1               5                   10                  15

Asp Ala Ser


<210> 37
<211> 19
<212> PRT
<213> synthetic construct


<400> 37


Ser Cys Asn Ser Phe Met Phe Ile Asn Gly Ser Phe Lys Glu Thr Gly
1               5                   10                  15

Gly Cys Ser


<210> 38
<211> 19
<212> PRT
<213> synthetic construct


<400> 38


Ser Cys Phe Gly Asn Leu Gly Asn Leu Ile Tyr Thr Cys Asp Arg Leu
1               5                   10                  15

Met Pro Ser


<210> 39
<211> 19
<212> PRT
<213> synthetic construct


<400> 39


Ser Cys Ser Phe Phe Met Pro Trp Cys Asn Phe Leu Asn Gly Glu Met
1               5                   10                  15

Ala Val Ser


<210> 40
<211> 19
<212> PRT
<213> synthetic construct


<400> 40


Ser Cys Phe Gly Asn Val Phe Cys Val Tyr Asn Gln Phe Ala Ala Gly
1               5                   10                  15

Leu Phe Ser

<210> 41
<211> 19
<212> PRT
<213> synthetic construct

<400> 41

Ser Cys Cys Phe Ile Asn Ser Asn Phe Ser Val Met Asn His Ser Leu
1               5                   10                  15

Phe Lys Ser


<210> 42
<211> 19
<212> PRT
<213> synthetic construct

<400> 42

Ser Cys Asp Tyr Phe Ser Phe Leu Glu Cys Phe Ser Asn Gly Trp Ser

    1               5                   10                  15

Gly Ala Ser


<210> 43
<211> 19
<212> PRT
<213> synthetic construct

<400> 43

Ser Cys Trp Met Gly Leu Phe Glu Cys Pro Asp Ala Trp Leu His Asp
1               5                   10                  15

Trp Asp Ser


<210> 44
<211> 19
<212> PRT
<213> synthetic construct

<400> 44

Ser Cys Phe Trp Tyr Ser Trp Leu Cys Ser Ala Ser Ser Ser Asp Ala
1               5                   10                  15

Leu Ile Ser


<210> 45
<211> 19
<212> PRT

<213> synthetic construct

<400> 45

```
Ser Cys Phe Gly Asn Phe Leu Ser Phe Gly Phe Asn Cys Glu Ser Ala
1               5                   10                  15

Leu Gly Ser
```

<210> 46
<211> 19
<212> PRT
<213> synthetic construct

<400> 46

```
Ser Cys Leu Tyr Cys His Leu Asn Asn Gln Phe Leu Ser Trp Val Ser
1               5                   10                  15

Gly Asn Ser
```

<210> 47
<211> 19
<212> PRT
<213> synthetic construct

<400> 47

```
Ser Cys Phe Gly Phe Ser Asp Cys Leu Ser Trp Phe Val Gln Pro Ser
1               5                   10                  15

Thr Ala Ser
```

<210> 48
<211> 19
<212> PRT
<213> synthetic construct

<400> 48

```
Ser Cys Asn His Leu Gly Phe Phe Ser Ser Phe Cys Asp Arg Leu Val
1               5                   10                  15

Glu Asn Ser
```

<210> 49
<211> 19
<212> PRT
<213> synthetic construct

<400> 49

Ser Cys Gly Tyr Phe Cys Ser Phe Tyr Asn Tyr Leu Asp Ile Gly Thr
1                   5                   10                  15

Ala Ser Ser

<210> 50
<211> 19
<212> PRT
<213> synthetic construct

<400> 50

Ser Cys Asn Ser Ser Ser Tyr Ser Trp Tyr Cys Trp Phe Gly Gly Ser
1                   5                   10                  15

Ser Pro Ser

<210> 51
<211> 13
<212> PRT
<213> synthetic construct

<400> 51

Cys Phe Val Leu Asn Cys His Leu Val Leu Asp Arg Pro
1                   5                   10

<210> 52
<211> 19
<212> PRT
<213> synthetic construct

<400> 52

Ser Cys Phe Gly Asn Phe Leu Ser Phe Gly Phe Asn Cys Glu Tyr Ala
1                   5                   10                  15

Leu Gly Ser

<210> 53
<211> 13
<212> PRT
<213> synthetic construct

<400> 53

Asp Gly Phe Phe Ile Leu Tyr Lys Asn Pro Asp Val Leu
1                   5                   10

<210> 54
<211> 7

<212> PRT
<213> synthetic construct

<400> 54

Asn His Gln Asn Gln Thr Asn
1                   5

<210> 55
<211> 7
<212> PRT
<213> synthetic construct

<400> 55

Ala Thr His Met Val Gly Ser
1                   5

<210> 56
<211> 7
<212> PRT
<213> synthetic construct

<400> 56

Gly Ile Asn Pro Asn Phe Ile
1                   5

<210> 57
<211> 7
<212> PRT
<213> synthetic construct

<400> 57

Thr Ala Ile Ser Gly His Phe
1                   5

<210> 58
<211> 13
<212> PRT
<213> synthetic construct

<400> 58

Leu Tyr Gly Thr Pro Glu Tyr Ala Val Gln Pro Leu Arg
1                   5                   10

<210> 59
<211> 13
<212> PRT
<213> synthetic construct

<400> 59

```
                    Cys Phe Leu Thr Gln Asp Tyr Cys Val Leu Ala Gly Lys
                    1               5                   10
```

<210> 60
<211> 13
<212> PRT
<213> synthetic construct

<400> 60

```
                    Asp Gly Phe Phe Ile Leu Tyr Lys Asn Pro Asp Val Leu
                    1               5                   10
```

<210> 61
<211> 13
<212> PRT
<213> synthetic construct

<400> 61

```
                    Val Leu His Leu Asp Ser Tyr Gly Pro Ser Val Pro Leu
                    1               5                   10
```

<210> 62
<211> 13
<212> PRT
<213> synthetic construct

<400> 62

```
                    Val Leu His Leu Asp Ser Tyr Gly Pro Ser Val Pro Leu
                    1               5                   10
```

<210> 63
<211> 13
<212> PRT
<213> synthetic construct

<400> 63

```
                    Val Val Asp Ser Thr Gly Tyr Leu Arg Pro Val Ser Thr
                    1               5                   10
```

<210> 64
<211> 13
<212> PRT
<213> synthetic construct

<400> 64

```
                        Val Leu Gln Asn Ala Thr Asn Val Ala Pro Phe Val Thr
                        1               5               10
```

<210> 65
<211> 13
<212> PRT
<213> synthetic construct

<400> 65

```
                        Trp Trp Ser Ser Met Pro Tyr Val Gly Asp Tyr Thr Ser
                        1               5               10
```

<210> 66
<211> 13
<212> PRT
<213> synthetic construct

<400> 66

```
                        Phe Gly His Gly Trp Leu Asn Thr Leu Asn Leu Gly Trp
                        1               5               10
```

<210> 67
<211> 13
<212> PRT
<213> synthetic construct

<400> 67

```
                        Phe Ser Pro Phe Ser Ala Asn Leu Trp Tyr Asp Met Phe
                        1               5               10
```

<210> 68
<211> 13
<212> PRT
<213> synthetic construct

<400> 68

```
                        Val Phe Val Pro Phe Gly Asn Trp Leu Ser Thr Ser Val
                        1               5               10
```

<210> 69
<211> 13
<212> PRT
<213> synthetic construct

<400> 69

```
                        Phe Trp Asn Val Asn Tyr Asn Pro Trp Gly Trp Asn Tyr
                        1               5               10
```

<210> 70
<211> 13
<212> PRT
<213> synthetic construct

<400> 70

```
Phe Tyr Trp Asp Arg Leu Asn Val Gly Trp Gly Leu Leu
1               5                   10
```

<210> 71
<211> 13
<212> PRT
<213> synthetic construct

<400> 71

```
Leu Tyr Ser Thr Met Tyr Pro Gly Met Ser Trp Leu Val
1               5                   10
```

<210> 72
<211> 16
<212> PRT
<213> synthetic construct

<400> 72

```
Arg Gly Asp Phe Leu Ser Phe Val Phe Pro Ala Ser Ala Trp Gly Gly
1               5                   10                  15
```

<210> 73
<211> 22
<212> PRT
<213> synthetic construct

<400> 73

```
Arg Gly Asp Ser Cys Ser Asp Cys Leu Lys Ser Val Asp Phe Ile Pro
1               5                   10                  15

Ser Ser Leu Ala Ser Ser
                20
```

<210> 74
<211> 6
<212> PRT
<213> synthetic construct

<400> 74

```
Gly Gly Trp Ser His Trp
1               5
```

<210> 75
<211> 3
<212> PRT
<213> synthetic construct

<400> 75

```
Arg Gly Asp
1
```

<210> 76
<211> 5
<212> PRT
<213> synthetic construct

<400> 76

```
Tyr Ile Gly Ser Arg
1               5
```

<210> 77
<211> 4
<212> PRT
<213> synthetic construct

<400> 77

```
Gly Arg Gly Asp
1
```

<210> 78
<211> 6
<212> PRT
<213> synthetic construct

<400> 78

```
Gly Tyr Ile Gly Ser Arg
1               5
```

<210> 79
<211> 5
<212> PRT
<213> synthetic construct

<400> 79

```
Pro Asp Ser Gly Arg
1               5
```

<210> 80
<211> 5
<212> PRT

<213> synthetic construct

<400> 80

```
                              Ile Lys Val Ala Val
                              1                   5
```

<210> 81
<211> 5
<212> PRT
<213> synthetic construct

<400> 81

```
                              Gly Arg Gly Asp Tyr
                              1                   5
```

<210> 82
<211> 7
<212> PRT
<213> synthetic construct

<400> 82

```
                              Gly Tyr Ile Gly Ser Arg Tyr
                              1                   5
```

<210> 83
<211> 4
<212> PRT
<213> synthetic construct

<400> 83

```
                              Arg Gly Asp Tyr
                              1
```

<210> 84
<211> 6
<212> PRT
<213> synthetic construct

<400> 84

```
                              Tyr Ile Gly Ser Arg Tyr
                              1                   5
```

<210> 85
<211> 4
<212> PRT
<213> synthetic construct

<400> 85

```
                      Arg Glu Asp Val
                      1
```

<210> 86
<211> 5
<212> PRT
<213> synthetic construct

<400> 86

```
                      Gly Arg Glu Asp Val
                      1                   5
```

<210> 87
<211> 4
<212> PRT
<213> synthetic construct

<400> 87

```
                      Arg Gly Asp Phe
                      1
```

<210> 88
<211> 5
<212> PRT
<213> synthetic construct

<400> 88

```
              Gly Arg Gly Asp Phe
              1               5
```

<210> 89
<211> 13
<212> PRT
<213> synthetic construct

<400> 89

```
              Cys Gly Phe Glu Cys Val Arg Gln Cys Pro Glu Arg Cys
              1               5                   10
```

<210> 90
<211> 4
<212> PRT
<213> synthetic construct

<400> 90

Lys Arg Ser Arg
1

<210> 91
<211> 7
<212> PRT
<213> synthetic construct

<400> 91

Lys Arg Ser Arg Gly Gly Gly
1                 5

<210> 92
<211> 7
<212> PRT
<213> synthetic construct

<400> 92

Ala Ser Ser Leu Asn Ile Ala
1                 5

<210> 93
<211> 6
<212> PRT
<213> synthetic construct

<400> 93

Lys Gln Ala Gly Asp Val
1                 5

<210> 94
<211> 5
<212> PRT
<213> synthetic construct

<400> 94

Tyr Ile Gly Ser Arg
1                 5

<210> 95
<211> 8
<212> PRT
<213> synthetic construct

<400> 95

Cys Arg Arg Gly Asp Trp Leu Cys
1                 5

<210> 96
<211> 4
<212> PRT
<213> synthetic construct

<400> 96

Arg Gly Asp Ser
1

<210> 97
<211> 4
<212> PRT
<213> synthetic construct

<400> 97

Lys Arg Ser Lys
1

<210> 98
<211> 7
<212> PRT
<213> synthetic construct

<400> 98

Lys Arg Ser Arg Gly Gly Gly
1                   5

<210> 99
<211> 70
<212> DNA
<213> synthetic construct

<220>
<221> misc_feature
<222> (1)..(70)

<223> N is A, G, C, or T
K isA, G, C, or T

<400> 99

agtgtgtgcc tcgagcnnkn nknnknnknn knnktatnnk nnknnknnkn nknnktctag    60
actgtgcagt                                                           70

<210> 100
<211> 39
<212> DNA
<213> synthetic construct

<220>
<221> misc_feature

<222> (1)..(39)
<223> N is A, C, G, or T
K is A, C, G, or T

<400> 100
nnknnknnkn nknnknnkta tnnknnknnk nnknnknnk 39

<210> 101
<211> 19
<212> PRT
<213> synthetic construct

<400> 101

```
Ser Cys Ser Val Tyr Asp His Lys Ile Gly Arg Asp Ser Phe Tyr Ser
1               5                   10                  15

Gly Cys Ser
```

<210> 102
<211> 19
<212> PRT
<213> synthetic construct

<400> 102

```
Phe Leu Ser Phe Val Phe Pro Ala Ser Ala Trp Gly Gly Ser Ser Gly
1               5                   10                  15

Arg Gly Asp
```

<210> 103
<211> 22
<212> PRT
<213> synthetic construct

<400> 103

```
Ser Cys Ser Asp Cys Leu Lys Ser Val Asp Phe Ile Pro Ser Ser Leu
1               5                   10                  15

Ala Ser Ser Arg Gly Asp
              20
```

<210> 104
<211> 13
<212> PRT
<213> synthetic construct

<220>
<221> misc_feature
<222> (13)..(13)
<223> residue 13 (glycine) can optionally have a polyethylene glycol mo iety attached

<400> 104

```
Phe Phe Pro Tyr Ser His Leu Gly Val Leu Ser Ser Gly
1               5               10
```

<210> 105
<211> 12
<212> PRT
<213> synthetic construct

<400> 105

```
Met Ala Ser Met Thr Gly Gly Gln Tyr Met Gly His
1               5               10
```

<210> 106
<211> 12
<212> PRT
<213> synthetic construct

<400> 106

```
Met Ala Ser Met Thr Gly Gly Gln Trp Met Gly His
```

```
                    1               5                   10
```

<210> 107
<211> 19
<212> PRT
<213> synthetic construct

<400> 107

```
Ser Cys Phe Tyr Gln Asn Val Ile Ser Ser Ser Phe Ala Gly Asn Pro
1               5               10                  15

Trp Glu Cys
```

<210> 108
<211> 19
<212> PRT
<213> synthetic construct

<400> 108

```
Ser Cys Asn Met Leu Leu Asn Ser Leu Pro Leu Pro Ser Glu Asp Trp
1               5               10                  15

Ser Ala Cys
```

<210> 109
<211> 19

<212> PRT
<213> synthetic construct

<400> 109

```
Ser Cys Pro Phe Thr His Ser Leu Ala Leu Asn Thr Asp Arg Ala Ser
1               5                   10                  15

Pro Gly Cys
```

<210> 110
<211> 19
<212> PRT
<213> synthetic construct

<400> 110

```
Ser Cys Phe Glu Ser Asp Phe Pro Asn Val Arg His His Val Leu Lys
1               5                   10                  15

Gln Ser Cys
```

<210> 111
<211> 19
<212> PRT
<213> synthetic construct

<400> 111

```
Ser Cys Val Phe Asp Ser Lys His Phe Ser Pro Thr His Ser Pro His
1               5                   10                  15

Asp Val Cys
```

<210> 112
<211> 19
<212> PRT
<213> synthetic construct

<400> 112

```
Ser Cys Gly Asp His Met Thr Asp Lys Asn Met Pro Asn Ser Gly Ile
1               5                   10                  15

Ser Gly Cys
```

<210> 113
<211> 12
<212> PRT
<213> synthetic construct

<400> 113

```
Met Ala Ser Met Thr Gly Gly Gln Trp Met Gly His
1               5                   10
```

<210> 114
<211> 19
<212> PRT
<213> synthetic construct

<400> 114

```
Ser Cys Asp Phe Phe Asn Arg His Gly Tyr Asn Ser Gly Cys Glu His
1               5                   10                  15

Ser Val Cys
```

<210> 115
<211> 19
<212> PRT
<213> synthetic construct

<400> 115

```
Ser Cys Gly Asp His Met Thr Asp Lys Asn Met Pro Asn Ser Gly Ile
1               5                   10                  15

Ser Gly Cys
```

<210> 116
<211> 19
<212> PRT
<213> synthetic construct

<400> 116

```
Ser Cys Tyr Tyr Asn Gly Leu Val Val His His Ser Asn Ser Gly His
1               5                   10                  15

Lys Asp Cys
```

<210> 117
<211> 17
<212> PRT
<213> synthetic construct

<400> 117

```
Cys Gly Ser Ser Leu Val Gly Leu His Ser Tyr Trp Ser Ser Pro Phe
1               5                   10                  15

Phe
```

**Description**

Field of the Invention

The present Invention generally relates to Interfacial biomaterials that mediate interaction between a non-biological substrate and a biological substrate, and methods for preparing and using the same. More particularly, the present invention relates to binding agents that create a binding interface between substrates via specific binding of each substrate.

Also disclosed herein are binding agents that create a non-binding Interface between substrates via specific binding to a non-biological substrate and substantially no binding to a biological substrate.

|  | Table of Abbreviations | |
|---|---|---|
| AFM | - | atomic force microscope |
| Ang1 | - | Angiopoltin-1 |
| BAP | - | bacterial alkaline phosphatase |
| BNHS | - | biotin N-hydroxysuccinimide ester |
| BSA | - | bovine serum albumin |
| DMSO | - | dimethyl sulfoxide |
| DWI | - | diffusion-weighted imaging |
| ELISA | - | enzyme-linked immunosorbent assay |
| ExFms | - | purified extracellular domain of the Fms receptor |
| ExTek | - | purified extracellular domain of the Tie2 receptor |
| FMOC | - | N-9-fluorenylmethyloxycarbonyl |
| fMRI | - | functional MR imaging |
| FTIR | - | Fourier Transform Infrared spectroscopy |
| GFP | - | green fluorescent protein |
| GST | - | glutathione-S-transferase |
| HPLC | - | high performance liquid chromatography |
| HRP | - | horseradish peroxidase |
| IFBM | - | interfacial biomaterial |
| IgG | - | immunoglobulin type G |
| ITO | - | indium tin oxide |
| I.U.B. | - | International Union of Biochemists |
| Ka | - | association constant |
| MRS | - | proton magnetic resonance spectroscopy |
| MTI | - | magnetization transfer imaging |
| NIH | - | National Institutes of Health |
| *pIII* | - | M13 phage gene encoding coat protein |
| PIII | - | M13 phage coat protein |
| PBS | - | phosphate buffered saline |
| PBS-T | - | PBS + 1% TRITON-X® detergent |
| PEG | - | polyethylene glycol |
| PELL | - | pellethane |
| PEPT | - | polyethylene terephthalate |
| PET | - | positron emission tomography |
| PFU | - | plaque-forming unit |
| PGA | - | polyglycolic acid |
| PHEMA | - | 2-hydroxyethyl methacrylate |
| PLA | - | polylactate |
| PMMA | - | polymethylmethacrylate |
| PPACK | - | D-phenylalanyl-L-prolyl-L-arginine |

(continued)

Table of Abbreviations

|  |  |  |
|---|---|---|
|  |  | chloromethylketone |
| TNF | - | tumor necrosis factor |
| scFv | - | single chain fragment variable antibody |
| SPECT | - | single photon emission computed tomography |
| SPR | - | surface plasmon resonance |
| TG1 | - | a strain of *E. coli* cells |
| TSAR | - | totally synthetic affinity reagents |
| VEGF | - | vascular endothelial growth factor |

Amino Acid Abbreviations and Corresponding mRNA Codons

| Amino Acid | 3-Letter | 1-Letter | mRNA Codons |
|---|---|---|---|
| Alanine | Ala | A | GCA GCC GCG GCU |
| Arginine | Arg | R | AGA AGG CGA CGC CGG CGU |
| Asparagine | Asn | N | AAC AAU |
| Aspartic Acid | Asp | D | GAC GAU |
| Cysteine | Cys | C | UGC UGU |
| Glutamic Acid | Glu | E | GAA GAG |
| Glutamine | Gln | Q | CAA CAG |
| Glycine | Gly | G | GGA GGC GGG GGU |
| Histidine | His | H | CAC CAU |
| Isoleucine | Ile | I | AUA AUC AUU |
| Leucine | Leu | L | UUA UUG CUA CUC CUG CUU |
| Lysine | Lys | K | AAA AAG |
| Methionine | Met | M | AUG |
| Proline | Pro | P | CCA CCC CCG CCU |
| Phenylalanine | Phe | F | UUC UUU |
| Serine | Ser | S | ACG AGU UCA UCC UCG UCU |
| Threonine | Thr | T | ACA ACC ACG ACU |
| Tryptophan | Trp | W | UGG |
| Tyrosine | Tyr | Y | UAC UAU |
| Valine | Val | V | GUA GUC GUG GUU |

Background of the Invention

The remarkable specificity of binding and function displayed by organic molecules has motivated efforts to employ these binding and functional activities in new ways. Molecular display technologies have facilitated these efforts by permitting rapid identification of specific binding agents for almost any target molecule. In particular, phage display of peptides and proteins (including antibodies) have led to the discovery of natural and designer binding sites.

Phage display systems use highly diverse libraries constructed by fusing degenerate sequences of DNA to a gene encoding a phage coat protein, such that the encoded variable protein sequence is displayed on the phage coat. Individual phage with desired binding specificities are isolated by binding to an immobilized or selectable target molecule. The peptides or proteins that confer binding are identified by sequencing the DNA within selected phage.

Peptides and proteins having unique binding and functional properties can be used as therapeutic agents (Raum *et al.*, 2001), as templates for molecular design, including drug design (Ballinger *et al.*, 1999; Bolin *et al.*, 2000; Wolfe *et al.*, 2000; Mourez *et al.*, 2001; Rudgers & Palzkill, 2001), as homing molecules for drug delivery (Arap *et al.*, 1998; Nilsson *et al.*, 2000; Ruoslahti, 2000), and as compositions to promote cellular attachment in cases of tissue healing or repair (*e.g.,* U.S. Patent Nos. 5,856,308; 5,635,482; and 5,292,362).

Phage display has also been used to select peptides that bind to inorganic surfaces with high specificity. Semiconductor surface-binding peptides that also bind a second molecule are suggested for assembly of electronic structures. See Whaley *et al.*, 2000.

Recent interest has developed in compositions that mimic recognition and functional capabilities of biological molecules to mediate interactions involving non-biological materials. For example, peptides can be used to coat prosthetic devices to thereby promote attachment of endothelial cells following implantation. See U.S. Patent Nos. 6,280,760; 6,140,127; 4,960,423; and 4,378,224.

Prior to the disclosure of the present Invention, preparation of peptide-coated surfaces and devices has been accomplished by non-specific adsorption, by coupling of the peptide to a derivatized surface, or by coupling of the peptide to a linker molecule covalently attached to the surface. These procedures are relatively tedious and time-consuming, and they generally require multiple steps for effective association of the peptide and the substrate. However, the potential benefits of non-biological surfaces and devices that include a biological coat are clear.

Thus, there exists a long-felt need In the art to develop an efficient and widely applicable method for promoting specific interactions between non-biological substrates and biological substrates. In addition, there exists a continuing need to develop methods for directing Interactions among molecules and/or cells, particularly in the context of diagnostic and therapeutic treatments.

To meet this need, the present invention relates to interfacial biomaterials that can mediate selective interactions between biological and non-biological substrates, novel binding agents that can specifically bind a target non-biologiCal substrate and/or a target biological substrate, and methods for making and using the same.

Summary of Invention

The present invention relates to an interfacial biomaterial comprising a plurality of binding agents wherein each binding agent comprises a first ligand that specifically binds a non-biological substrate and a second ligand that specifically binds a biological substrate, and wherein the plurality of binding agents comprise an interface between the non-biological substrate and the biological substrate.

Accordingly, in a first aspect, the present invention provides an interfacial biomaterials comprising a plurality of binding agents, wherein each binding agent comprises a first ligand that specifically binds a target non-biological substrate, and a second ligand that specifically binds a target biological substrate, wherein each binding agent comprises two or more binding specificities, wherein the plurality of binding agents are capable of defining an interface between the target non-biological substrate and the target biological substrate, and wherein the first ligand comprises a peptide and the second ligand comprises a peptide.

Also disclosed herein is an interfacial biomaterial comprising a plurality of binding agents wherein each binding agent comprises first and second ligands that specifically bind a biological substrate, and wherein the plurality of binding agents comprise an interface between the biological substrates. In one embodiment, the first and second ligands bind the same biological substrate. In another embodiment, the first and second ligands bind different biological substrates.

Also disclosed herein is an interfacial biomaterial comprising a plurality of binding agents, wherein each binding agent comprises a ligand that specifically binds a target non-biological substrate and a non-binding domain that substantially lacks binding to a target biological substrate.

The interfacial "biomaterial can comprise a plurality of identical or non-identical binding agents. When the interfacial biomaterial comprises a plurality of non-identical binding agents, each of the plurality of non-identical binding agents comprises in one embodiment an identical ligand that specifically binds a non-biological substrate.

The present invention further provides a patterned interfacial biomaterial, wherein the binding agents are spatially restricted within the interface.

Representative non-biological substrates include but are not limited to a non-biological substrate comprising a synthetic polymer, plastic, metal, a metal oxide, a non-metal oxide, silicone, a ceramic material, a drug, or a drug carrier. In one embodiment, a synthetic polymer comprises polyglycolic acid. In another embodiment, a synthetic polymer comprises a nylon suture. In one embodiment, a plastic comprises polycarbonate, in another embodiment polystyrene, and in yet another embodiment polyurethane. In one embodiment, a metal comprises titanium. In another embodiment, a metal comprises stainless steel.

Representative biological substrates include but are not limited to a tissue, a cell, or a macromolecule. In one embodiment, a target biological substrate comprises collagen. In another embodiment, a biological substrate comprises a Tie2 receptor.

Also provided are methods for preparing an interfacial biomaterial as defined in the appendant claims. Thus, in one embodiment of the invention, the method comprises: (a) applying to a non-biological substrate a plurality of binding agents, wherein each of the plurality of binding agents comprises a first ligand that specifically binds to the non-biological substrate and a second ligand that specifically binds a target biological substrate, and wherein the applying Is free of coupling; (b) contacting the non-biological substrate, wherein the plurality of binding agents are bound to the non-biological substrate, with a sample comprising the target biological substrate; and (c) allowing a time sufficient for binding of the target biological substrate to the plurality of binding agents, wherein an interfacial biomaterial is prepared. In accordance with the disclosed invention, the contacting can comprise contacting *in vitro*, *ex vivo*, or *in vivo*.

In another embodiment of the invention, an interfacial biomaterial comprises a biological array. In one embodiment, a method for preparing an interfacial biomaterial comprises: (a) providing a non-biological substrate having a plurality of positions; (b) applying to each of the plurality of positions a binding agent comprising a first ligand that specifically binds the non-biological substrate and a second ligand that specifically binds a target biological substrate, wherein the applying is free of coupling; (c) contacting the non-biological substrate, wherein a plurality of binding agents are bound to the non-biological substrate, with a sample comprising the target biological substrate; and (d) allowing a time sufficient for binding of the target biological substrate to the plurality of binding agents, whereby a biological array is prepared. In one embodiment, a method for applying the plurality of binding agents comprises dip-pen printing.

Also disclosed herein is, a method for preparing an interfacial biomaterial comprises: (a) applying to a non-biological substrate a plurality of binding agents, wherein each of the plurality of binding agents comprises a ligand that specifically binds to the non-biological substrate and a non-binding domain that shows substantially no binding to a target biological substrate, and wherein the applying is free of coupling; and (b) contacting the non-biological substrate, wherein the plurality of binding agents are bound to the non-biological substrate, with a sample comprising the target biological substrate, whereby an interfacial biomaterial is prepared.

Also disclosed herein are methods for preparing binding agents. In one instance, the method comprises: (a) panning a library of diverse molecules over a target non-biological substrate, whereby a first ligand that specifically binds a target non-biological substrate is identified; and (b) linking the first ligand to a second ligand, wherein the second ligand specifically binds a target biological substrate, whereby a binding agent is prepared. The method can further comprise panning a ligand over a target biological substrate, whereby a ligand that specifically binds a target biological substrate is identified.

In another instance, a method for preparing a binding agent comprises: (a) panning a library of diverse molecules over a target non-biological substrate, whereby a ligand that specifically binds a target non-biological substrate is identified; and (b) linking the ligand to a non-binding domain, wherein the non-binding domain shows substantially no binding to a target biological substrate, whereby a binding agent is prepared. The method can further comprise panning a ligand over a target biological substrate, whereby a non-binding domain that shows substantially no binding to a target biological substrate is identified.

Also disclosed are binding agents produced by the method. In one embodiment of the Invention, a binding agent further comprises a linker that links the first ligand and the second ligand. Also disclosed herein is a linker that links the first ligand and non-binding domain.

In the invention, the first ligand comprises a peptide. Also disclosed herein are single chain antibodies that specifically bind a non-biological substrate.

Representative plastic-binding ligands are set forth as SEQ ID NOs:1-23 and 66-71, and representative metal-binding ligands are set forth as SEQ ID NOs:24-36 and 51-65. The second ligand or non-binding region comprises a peptide.

Also disclosed herein, e.g. for use in the invention, are synthetic peptides comprising polystyrene-binding, polyurethane-binding, polycarbonate-binding, polyglycolic acid-binding, titanium-binding, stainless steel-binding ligands. In one embodiment, the synthetic ligands used in the invention comprise less than about 20 amino acid residues. Representative polystyrene-binding peptide ligands are set forth as SEQ ID NOs:1-22, a representative polyurethane-binding ligand is set forth as SEQ ID NO:23, representative polycarbonate-binding ligands are set for as SEQ ID NOs: 68-71, representative titanium-binding peptide ligands are set forth as SEQ ID NOs:24-36, and representative stainless steel-binding ligands are set forth as SEQ ID NOs:51-65.

The disclosure further provides representative methods for using an interfacial biomaterial, including, but not limited to a method for cell culture, a method for implanting a device in a subject, a method for modulating an activity of a biological substrate, a method for preparing a non-fouling coating, a method for drug delivery, and a method for screening for screening a test substance for interaction with a biological substrate.

A method for cell culture, In accordance with the present invention, can comprise: (a) applying to a non-biological substrate a plurality of binding agents, wherein each of the plurality of binding agents comprises a first ligand that specifically binds the non-biologibal substrate and a second ligand that specifically binds cells, macromolecules or a combination thereof, wherein the applying is free of coupling; (b) contacting the non-biological substrate, wherein the plurality of binding agents are bound to the non-biological substrate, with cells; (c) allowing a time sufficient for binding of the cells to the plurality of binding agents; and (d) culturing the cells.

The present invention also provides interfacial biomaterials for use in methods for implanting a device in a subject. Such a method may comprise: (a) applying to an implant a plurality of binding agents, wherein each of the plurality of binding agents comprises a first ligand that specifically binds the implant and a second ligand that specifically binds cells at an implant site, wherein the applying is free of coupling; and (b) placing the implant in a subject at the implant site. When implanted in a subject, a device so prepared can promote cell attachment to the device.

As also disclosed herein is a method for creating a lubricant interface comprising: applying to a first substrate a plurality of binding agents, wherein the applying is free of coupling, and wherein each of the plurality of binding agents comprises: (a) a ligand that specifically binds to the first substrate; and (b) a non-binding domain that shows substantially no binding to a second substrate. The first substrate can comprise a non-biological or a biological substrate.

Also disclosed herein is a method for implanting a device In a subject can comprise: (a) applying to the implant a plurality of binding agents, wherein each of the plurality of binding agents comprises a ligand that specifically binds the implant and a non-binding domain that shows substantially no binding to cells at an implant site, wherein the applying is free of coupling; and (b) placing the implant In a subject at the implant site. When implanted in a subject, a device so prepared can provide a lubricating activity at the implant site.

As also disclosed herein, a method for preparing an interfacial biomaterial comprising a boundary lubricant can also comprise:

(a) administering to a subject a plurality of binding agents, wherein each of the plurality of binding agents comprises a ligand that specifically binds a first biological substrate and a non-binding domain that shows substantially no binding to a second biological substrate; and (b) allowing a time sufficient for binding of the plurality of binding agents to the first biological substrate, whereby a lubricant interface is created.

The invention relates to a method for modulating an activity of a biological substrate, the method comprising:

(a) coating a biodegradable, non-biological substrate with a plurality of binding agents, wherein each of the plurality of binding agents comprises a first ligand that specifically binds the biodegradable, non-biological substrate and a second ligand that specifically binds the biological substrate, wherein the coating is free of coupling; (b) placing the coated biodegradable, non-biological substrate at a target site, wherein the biological substrate is present at the target site; and (c) allowing a time sufficient for binding of the biological substrate at the target site to the binding agents, wherein the binding modulates the activity of the biological substrate. In one embodiment, a biological substrate is a vascular endothelial cell. In another embodiment, biological substrate is a tumor vascular endothelial cell. In yet another embodiment, a biological substrate is a Tie2 receptor. In one embodiment, a target site is a wound site, and the modulating promotes wound healing. In another embodiment, a target site is an angiogenic site and the modulating inhibits angiogenesis, including, but not limited to tumor angiogenesis.

Also disclosed herein is a method for preparing a non-biological substrate with a non-fouling coating. The coating comprises a plurality of binding agents, wherein each of the plurality of binding agents comprises: (a) a ligand that specifically binds the non-biological substrate; and (b) a non-binding domain that shows substantially no binding to a fouling agent.

The present invention also relates to a method for drug delivery involving an interfacial biomaterial. The method comprises: (a) applying to a non-biological drug, or to a non-biologlcal carrier of the drug, a plurality of binding agents, wherein each of the plurality of binding agents comprises a first ligand that specifically binds the drug or the drug carrier and a second ligand that specifically binds a target cell; (b) administering the drug to a subject; and (c) allowing a sufficient time for binding of the plurality of binding agents to the target cell.

Also disclosed herein is a method for screening a test substance for interaction with a biological substrate. In one embodiment, the method comprises: (a) preparing a biological array comprising a plurality of biological substrates, wherein each of the plurality of biological substrates is specifically bound to one of a plurality of positions on a non-biological substrate; (b) contacting the biological array with a candidate substance; (c) allowing a time sufficient for binding of the candidate substance to the biological array; and (d) assaying an interaction between one or more of the biological substrates and the candidate substance, whereby an interacting molecule is identified.

Accordingly, it is an object of the present disclosure to provide interfacial biomaterials that can mediate direct binding and non-binding interactions between substrates. This object is achieved in whole or in part by the present disclosure .

An object of the invention having been stated above, other objects and advantages of the present disclosure will become apparent to those skilled in the art after a study of the following description and non-limiting Examples.

Detailed Description of the Invention

I. Definitions

While the following terms are believed to be well understood by one of ordinary skill in the art, the following definitions are set forth to facilitate explanation of the invention.

The term "ligand" as used herein refers to a molecule or other chemical entity having a capacity for binding to a substrate. A ligand can comprise a peptide, an oligomer, a nucleic acid (*e.g.,* an aptamer), a small molecule (*e.g.,* a chemical compound), an antibody or fragment thereof, a nucleic acid-protein fusion, a polymer, a polysaccharide, and/or any other affinity agent.

The term "non-binding domain" as used herein refers to a molecule, macromolecule, or other chemical entity that shows substantially no binding to a target substrate. A non-binding domain can comprise a peptide, an oligomer, a nucleic acid (*e.g.*, an aptamer), a small molecule (*e.g.,* a chemical compound), an antibody or fragment thereof, a nucleic acid-protein fusion, a polymer, a polysaccharide, and/or any other agent that shows substantially no binding to a target substrate.

The term "substrate" as used herein refers to a biological or non-biological composition used to prepare an interfacial biomaterial. Thus, the term "substrate" encompasses compositions having a capacity for binding to a ligand in the invention. Also disclosed are compositions showing substantially no binding to a non-binding domain of the disclosure,

The term "target" is typically used to qualify a description of a substrate as one of multiple substrates having different binding specificities. Thus, the term "target" generally refers to a substrate that is specifically bound by a ligand of the present invention, or to a substrate that shows substantially no binding to a non-binding domain of the present disclosure.

The term "binding" refers to an affinity between two molecules, for example, between a peptide and a substrate. As used herein, "binding" refers to a preferential binding of a peptide for a substrate In a mixture of molecules. The binding of a peptide to a substrate can be considered specific if the binding affinity is about $1 \times 10^4$ $M^{-1}$ to about $1 \times 10^6$ $M^{-1}$ or greater.

The phrase specifically (or selectively) binds", when referring to the binding capacity of a ligand, refers to a binding reaction that is determinative of the presence of the substrate in a heterogeneous population of other substrates. Specific binding excludes non-specific adsorption, covalent linkage via a chemical reaction, and coupling via a linking moiety.

The term "time sufficient for binding" generally refers to a temporal duration sufficient for specific binding of a ligand and a substrate.

The phases "substantially lack binding" or "substantially no binding", as used herein to describe binding of a ligand or non-binding domain to a substrate, refers to a level of binding that encompasses non-specific or background binding, but does not include specific binding.

The term "subject" as used herein refers to any invertebrate or vertebrate species. The methods of the present invention are particularly useful in the treatment and diagnosis of warm-blooded vertebrates. Thus, the invention concerns mammals and birds. More particularly, contemplated is the treatment and/or diagnosis of mammals such as humans, as well as those mammals of importance due to being endangered (such as Siberian tigers), of economical importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), and horses. Also contemplated is the treatment of birds, including the treatment of those kinds of birds that are endangered, kept in zoos, as well as fowl, and more particularly domesticated fowl, *e.g.*, poultry, such as turkeys, chickens, ducks, geese, guinea fowl, and the like, as they are also of economical importance to humans. Thus, contemplated is the treatment of livestock, including, but not limited to, domesticated swine (pigs and hogs), ruminants, horses, poultry, and the like.

The term "about", as used herein when referring to a measurable value such as a number of amino acids, etc. Is meant to encompass variations of in one embodiment $\pm 20\%$ or $\pm 10\%$, in another embodiment $\pm 5\%$, In another embodiment $\pm 1\%$, and in yet another embodiment $\pm 0.1\%$ from the specified amount, as such variations are appropriate to perform the disclosed methods.

## II. Interfacial Biomaterials

The present invention provides an interfacial biomaterial comprising a plurality of binding agents, as defined in the appendant claims. Each binding agent specifically binds a non-biological substrate and a biological substrate, to thereby create an interface between the non-biological substrate and the biological substrate. Also disclosed are binding agents and methods for making the same, as described further herein below.

The term "interfacial biomaterial" is used herein to broadly refer to a composition comprising a plurality of binding agents, wherein the plurality of binding agents creates a functional interface between two or more substrates. Each of the binding agents comprises two or more desired binding specificities, or a desired combination of binding specificities, including: (a) specific binding of at least one non-biological substrate; (b) and specific binding of at least one target biological substrate.

Several prior studies have described ligands having two or more binding specificities. For example, U.S. Patent No. 5,948,635 to Kay et al. discloses totally synthetic affinity reagents (TSARs) comprising bivalent fusion peptides. As defined therein, a bivalent peptide comprises two functional regions; a binding domain and an effector domain that is useful for enhancing expression and/or detection of the expressed TSAR. In contrast to the bivalent peptides described In U.S. Patent No. 5,948,635 to Kay et al., an interfacial biomaterial of the present invention comprises two or more binding domains and does not require an element for enhancing expression and/or detection of the interfacial biomaterial. In addition, U.S. Patent No. 5,948,di35 to Kay et al. does not disclose creation of an interfacial biomaterial comprising a plurality of binding agents, wherein the plurality of binding agents creates a functional interface between two or more substrates.

The term "functional interface" refers to an interface, wherein the functionality of the interface requires a plurality of binding agents. More particularly, a functional interface is not created by a binding reaction between a single binding agent and a substrate. For example, a binding interaction between a solid support, such as a purification column, and a molecule of interest does not comprise a functional interface in that the functionality of the interaction (purification) can comprise a single reagent and a single molecule of Interest.

Representative functional interfaces include coatings, wherein the plurality of binding agents comprises a binding interface, a non-binding interface, or a combination thereof. The term "binding interface" refers to an interface created using binding agents comprising a first ligand that specifically binds a first substrate (*e.g.,* a non-biological substrate) and a second ligand that specifically binds a second substrate (*e.g.*, a biological substrate. Thus, a binding interface mediates interaction between two or more substrates by providing an affinity for each of the two or more substrates. In one instance, the two or more substrates are all the same. In another instance, the two or more substrates are not all the same.

The term "non-binding interface" refers to an interface created using binding agents comprising a first ligand that specifically binds a first substrate (*e.g.,* a non-biological substrate) and a second ligand that shows substantially no binding to a second substrate (*e.g.,* a target biological substrate). Additionally, a non-binding Interface can be created using binding agents comprising a first ligand that shows substantially no binding to a target non-biological substrate and a second ligand that specifically binds a biological substrate. A non-binding Interface thus ensures a lack of interaction between two or more substrates.

A functional Interface can also comprise a biological array, wherein each of the plurality of binding agents is adhered to a substrate at a prescribed position, and the sum of each of the plurality of binding agents comprises a pattern. In one embodiment of a patterned interfacial biomaterial In accordance with the present invention, binding agents of the present invention are applied to a non-biological interface in a spatially restricted manner, as described further herein below.

An interfacial biomaterial of the present invention can comprise a homogeneous interfacial biomaterial, wherein each of the plurality of binding agents is identical. Alternatively, an interfacial biomaterial can be heterogeneous by constructing the interfacial biomaterial using a plurality of non-identical binding agents. In one embodiment, each of the plurality of non-identical binding agents comprises: (a) an identical ligand that specifically binds a first substrate (preferably a non-biological substrate); and (b) a variable domain. The variable domain can be selected from among any of a variety of ligands for substrates (in one embodiment, a biological substrate), so that a plurality of substrates (in one embodiment, a biological substrate) can be bound and/or not bound.

For example, a heterogeneous interfacial biomaterial can comprise a plurality of non-identical binding agents, wherein each of the plurality of non-binding agents comprises: (a) a first ligand that specifically binds polystyrene; and (b) a second ligand that specifically binds one of a variety of cell types. The plurality of binding agents can be adhered to a polystyrene substrate. A sample comprising a mixed cell population, wherein each of a different type of cell in the mixed cell population specifically binds one of the plurality of second ligands, can be provided to the polystyrene substrate. Following a time sufficient for binding of the mixed cell population to the plurality of binding agents, a heterogeneous interfacial biomaterials is formed between the polystyrene substrate and the mixed cell populations.

In one embodiment of the invention, preparation of a heterogeneous Interfacial biomaterial can comprise: (a) adhering at random positions on a non-biological substrate each of a plurality of non-identical binding agents; or (b) adhering at known positions on a non-biological substrate each of a plurality of non-identical binding agents. Thus, a heterogeneous Interfacial biomaterial can comprise a randomly heterogeneous or a patterned heterogeneous interfacial biomaterial.

A patterned interfacial biomaterial can be prepared In one embodiment by delivering each of a plurality of binding agents to a discrete position on a non-biological substrate using any technique suitable for dispensing a binding agent, including but not limited to spraying, painting, ink-jetting, dip-pen writing (Example 15), microcontact printing (U.S. Patent Nos. 6,180,239 and 6,048,623), stamping (U.S. Patent Nos. 5,512,131 and 5,776,748), or lithography (Bhatia *et al.*, 1993), PCT International Publication No. WO 00/56375.

The present invention further provides methods for preparing an interfacial biomaterial as defined in the appendant claims. In one embodiment of the invention, a method for preparing a binding interfacial biomaterial comprises:

(a) applying to a non-biological substrate a plurality of binding agents, wherein each of the plurality of binding agents comprises a first ligand that specifically binds to the non-biological substrate and a second ligand that specifically binds a target biological substrate, and wherein the applying is free of coupling; (b) contacting the non-biological substrate, wherein the plurality of binding agents are bound to the non-biological substrate, with a sample comprising the target biological substrate; and (c) allowing a time sufficient for binding of the target biological substrate to the plurality of binding agents, whereby an interfacial biomaterial is prepared.

Alternatively, binding of the plurality of binding agents to each of a non-biological substrate and a biological substrate can be performed simultaneously or in the reverse order, depending on a particular application. Thus, a method for preparing a binding interfacial biomaterial can also comprise: (a) contacting a plurality of binding agents, wherein each of the binding agents comprises a first ligand that specifically binds to the non-biological substrate and a second ligand that specifically binds a target biological substrate, and wherein the applying is free of coupling; (b) applying to a non-biological substrate a plurality of binding agents; and (c) allowing a time sufficient for binding of the non-biological substrate to the plurality of binding agents whereby an interfacial biomaterial is prepared.

Also disclosed herein is a method for preparing a non-binding interfacial biomaterial comprises: (a) applying to a non-biological substrate a plurality of binding agents, wherein each of the plurality of binding agents comprises a ligand that specifically binds to the non-biological substrate and a non-binding domain that shows substantially no binding to a target biological substrate, and wherein the applying is free of coupling and free of covalent linkage; and (b) contacting the non-biological substrate, wherein the plurality of binding agents are bound to the non-biological substrate, with a sample comprising the target biological substrate, whereby an interfacial biomaterial is prepared.

II.A. <u>Non-Biological Substrates</u>

The term "non-biological substrate" is used herein to describe a substrate that is not a quality or component of a living system. Representative non-biological substrates include but are not limited to common plastics (*e.g.*, polystyrene, polyurethane, polycarbonate), silicone, synthetic polymers, metals (including mixed metal alloys), metal oxides (*e.g.,* glass), non-metal oxides, ceramics, drugs, drug carriers, and combinations thereof.

A non-biological substrate can comprise any form suitable to its intended use including but not limited to a planar surface (*e.g.,* a culture plate), a non-planar surface (*e.g.,* a dish, an implant, or a tube), or a substrate in solution. In one embodiment, a non-biological substrate comprises a minimum dimension of at least about 20 nm. For example, a non-biological substrate can comprise a minimum dimension of about 50 nm, about 100 nm, about 200 nm, about 500 nm, about 1 $\mu$m, about 50 $\mu$m, about 100 $\mu$m, about 200 $\mu$m, about 500 $\mu$m, or about 1 mm.

Representative synthetic polymers include but are not limited to polytetrafluoroethylene, expanded polytetrafluoroethylene, GORE-TEX® (Gore & Associates, Inc. of Newark, Delaware), polytetrafluoroethylene, fluorinated ethylene propylene, hexafluroropropylene, polymethylmethacrylate (PMMA), pellethane (a commercial polyurethane, PELL), 2-hydroxyethyl methacrylate (PHEMA), polyethylene terephthalate (PEPT), polyethylene, polypropylene, nylon, polyethyleneterephthalate, polyurethane, silicone rubber, polystyrene, polysulfone, polyester, polyhydroxyacids, polycarbonate, polyimide, polyamide, polyamino acids, and combinations thereof. In one embodiment, a synthetic polymer comprises an expanded or porous polymer. In another embodiment, a synthetic polymer comprises a nylon suture.

Representative metals that can be used in accordance with the methods of the present invention include but are not limited to titanium, stainless steel, gold, silver, rhodium, zinc, platinum, rubidium, and copper. Suitable ceramic materials include but are not limited to silicone oxides, aluminum oxides, alumina, silica, hydroxyapapitites, glasses, quartz, calcium oxides, calcium phosphates, indium tin oxide (ITO), polysilanols, phosphorous oxide, and combinations thereof.

Other non-biological substrates include carbon-based materials such as graphite, carbon nanotubes, carbon "buckyballs", and metallo-carbon composites.

Preparation of an interfacial biomaterial for drug delivery can employ a non-biological substrate comprising a drug or drug carrier. The term "drug" as used herein refers to any substance having biological or detectable activity. Thus, the term "drug" includes a pharmaceutical agent, a detectable label, or a combination thereof. The term "drug" also includes any substance that is desirably delivered to a target cell.

The term "drug carrier", as used herein to describe a non-biological substrate, refers to a composition that facilitates drug preparation and/or administration. Any suitable drug delivery vehicle or carrier can be used, including but not limited to a gene therapy vector (*e.g.,* a viral vector or a plasmid), a microcapsule (for example, a microsphere or a nanosphere, Manome *et al.*, 1994; Saltzman & Fung, 1997), a fatty emulsion (U.S. Patent No. 5,651,991), a nanosuspension (U.S. Patent No. 5,858,410), a polymeric micelle or conjugate (Goldman *et al.*, 1997; U.S. Patent Nos. 4,551,482, 5,714,166, 5,510,103, 5,490,840, and 5,855,900), a liposome (U.S. Patent Nos. 6,214,375; 6,200,598; 6,197,333); and a polysome (U.S. Patent No. 5,922,545).

The term "detectable label" refers to any substance that can be detected, including, but not limited to an agent that can be detected using non-invasive methods such as scintigraphic methods, magnetic resonance imaging, ultrasound, spectroscopic, enzymatic, electrochemical, and/or fluorescence. Representative substrates useful for non-invasive imaging are described herein below.

A non-biological substrate is selected for a desired application based on a number of factors including but not limited to biocompatibility, degradability, surface area to volume ratio, and mechanical integrity. For clinical applications, a non-biological substrate can comprise a biocompatible non-biological substrate such as titanium, synthetic polymers (*e.g.,* silicone), and any other biocompatible non-biological substrate. A non-biological substrate can also be rendered biocompatible by application of a plurality of binding agents as disclosed herein. Selection of a suitable non-biological substrate is within the skill of one in the art.

II.B. Biological Substrates

The term "biological substrate" as used herein refers to a quality or component pertaining to living systems. As such, a "biological substrate" can comprise an organ, a tissue, a cell, or components thereof. Thus, a biological substrate can comprise a macromolecule including, but not limited to a protein (*e.g.,* an antibody, collagen, a receptor), a peptide, a nucleic acid *(e.g.,* an aptamer), an oligomer, a small molecule *(e.g.,* a chemical compound), a nucleic acid-protein fusion, and/or any other biological affinity agent. The term "biological substrate" also encompasses substrates that have been Isolated from a living system and substrates that have been recombinantly or synthetically produced.

III. Binding Agents

The term binding agent" refers to a composition that mediates a binding or non-binding interaction between two substrates. In the invention a binding agent mediates an Interaction between a non-biological substrate and a biological substrate. Thus, in the present invention, a binding agent comprises: (a) a ligand that specifically binds a non-biological substrate; and (b) a ligand that specifically binds a biological substrate. Also disclosed herein is, a binding agent that comprises:

(a) a ligand that specifically binds a non-biological substrate; and (b) a non-binding domain that shows substantially no binding to a target biological substrate.

A ligand that specifically binds a non-biological substrate shows specific binding In the absence of covalent linkage or coupling via a linking moiety. For example, the binding between the ligand and the non-biological substrate is free of any of the forms of linking described herein below as they pertain to, for example, linking a first and second ligand of a binding agent.

A ligand that specifically binds a biological substrate can possess additional bioactivity as a result of specific binding. For example, a ligand can additionally show kinase activity, phosphatase activity, DNA repair activity, oncogene activity, tumor suppressor activity, angiogenesis stimulatory activity, angiogenesis inhibitory activity, mitogenic activity, signaling activity, transport activity, enzyme activity, anti-fouling activity, antibacterial activity, anti-viral activity, antigenic activity, immunogenic activity, apoptosis-inducing activity, anti-apoptotic-inducing activity, cytotoxic activity, lubricant activity, and combinations thereof.

A binding agent can be constructed by linking a first and second ligand, or a ligand and a non-binding domain, to form a single molecule or complex. Linking can comprise fusing two or more peptide ligands during synthesis, as described in Examples 12 and 13. Optionally, a peptide linker region between the two domains can also be incorporated during synthesis. Alternatively, a first and second ligand can be combined via a linker by covalent bonding or chemical coupling, as described further herein below.

III.A. Peptides

In the invention, a ligand comprises a peptide ligand that specifically binds to a non-biological substrate and/or to a biological substrate. Also disclosed herein is a non-binding domain that comprises a peptide that shows substantially no binding to a target biological substrate.

The term "peptide" broadly refers to an amino acid chain that includes naturally occurring amino acids, synthetic amino acids, genetically encoded amino acids, non-genetically encoded amino acids, and combinations thereof. Peptides can include both L-form and D-form amino acids. A peptide of the present invention can be subject to various changes, substitutions, insertions, and deletions where such changes provide for certain advantages in its use. Thus, the term "peptide" encompasses any of a variety of forms of peptide derivatives including amides, conjugates with proteins, cyclone peptides, polymerized peptides, conservatively substituted variants, analogs, fragments, chemically modified peptides, and peptide mimetics.

In one embodiment of the invention, the peptide comprises an amino acid sequence comprising at least about 3 residues, in another embodiment about 3 to about 50 residues, and in yet another embodiment about 3 to about 25 residues. Any peptide ligand that shows specific binding features can be used in the practice of the present invention. In one embodiment, peptide fragments containing less than about 25 amino acid residues are employed. In another embodiment, peptide fragments less than about 20 amino acids are employed.

Representative non-genetically encoded amino acids include but are not limited to 2-aminoadipic acid; 3-aminoadipic add; β-aminopropionic acid; 2-aminobutyric acid; 4-aminobutyric acid (piperidinic acid); 6-aminocaproic acid; 2-aminoheptanoic acid; 2-aminoisobutyrlc acid; 3-aminoisobutyric acid; 2-aminopimelic acid; 2,4-diaminobutyric acid; desmosine; 2,2'-diaminopimelic acid; 2,3-diaminopropionic acid; N-ethylglycine; N-ethylasparagine; hydroxylysine; allo-hydroxylysine; 3-hydroxyproline; 4-hydroxyproline; Isodesmosine; allo-isoleucine; N-methylglycine (sarcosine); N-methyllsoleucine; N-methylvaline; norvaline; norleucine; and ornithine.

Representative derivatized amino acids include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups can be derivatized to form salts, methyl and ethyl esters or other types of esters or hydrazides. Free hydroxyl groups can be derivatized to form O-acyl or O-alkyl derivatives. The imidazole nitrogen of histidine can be derivatized to form N-im-benzylhistidine.

The term "conservatively substituted variant" refers to a peptide having an amino acid residue sequence substantially identical to a sequence of a reference peptide in which one or more residues have been conservatively substituted with a functionally similar residue. In one embodiment, a conservatively substituted variant displays a similar binding specificity when compared to the reference peptide. The phrase "conservatively substituted variant" also includes peptides

wherein a residue is replaced with a chemically derivatized residue, provided that the resulting peptide has a binding specificity as disclosed herein.

Examples of conservative substitutions include the substitution of one non-polar (hydrophobic) residue such as isoleucine, valine, leucine or methionine for another; the substitution of one polar (hydrophilic) residue for anther such as between arginine and lysine, between glutamine and asparagine, between glycine and serine; the substitution of one basic residue such as lysine, arginine or histidine for another; or the substitution of one acidic residue, such as aspartic acid or glutamic acid for another.

Peptides of the present Invention also include peptides having one or more additions and/or deletions or residues relative to the sequence of a peptide whose sequence is disclosed herein, so long as the requisite binding specificity of the peptide is maintained. The term "fragment" refers to a peptide having an amino acid residue sequence shorter than that of a peptide disclosed herein.

A peptide can be modified by terminal-$NH_2$ acylation (*e.g.*, acetylation, or thioglycolic acid amidation) or by terminal-carboxylamidabon (*e.g.*, with ammonia or methylamine). Terminal modifications are useful to reduce susceptibility by proteinase digestion, and to therefore prolong a half-iffe of peptides in solutions, particularly in biological fluids where proteases can be present.

Peptide cyclization is also a useful modification because of the stable structures formed by cyclization and in view of the biological activities observed for such cyclic peptides. Representative methods for cyclizing peptides are described by Schneider & Eberle (1993) Peptides, 1992: Proceedings of the Twenty-Second European Peptide Symposium, September 13-19, 1992. Interlaken, Switzerland, Escom, Leiden, The Natherlands. Typically, tertbutoxycarbonyl protected peptide methyl ester is dissolved in methanol, sodium hydroxide solution is added, and the admixture is reacted at 20°C to hydrolytically remove the methyl ester protecting group. After evaporating the solvent, the tertbutoxycarbonyl-protected peptide is extracted with ethyl acetate from acidified aqueous solvent. The tertbutoxycarbonyl protecting group is then removed under mildly acidic conditions in dioxane co-solvent. The unprotected linear peptide with free amino and carboxyl termini so obtained is converted to its corresponding cyclic peptide by reacting a dilute solution of the linear peptide, in a mixture of dichloromethane and dimethylformamide, with dicyclohexylcarbodiimide in the presence of 1-hydroxybenzotrlazole and N-methylmorpholine. The resultant cyclic peptide is then purified by chromatography.

Optionally, a ligand of the present invention can comprise one or more amino acids that have been modified to contain one or more halogens, such as fluorine, bromine, or iodine, to facilitate linking to a linker molecule as described further herein below.

The term "peptoid" as used herein refers to a peptide wherein one or more of the peptide bonds are replaced by pseudopeptide bonds including but not limited to a carba bond ($CH_2$-$CH_2$), a depsi bond (CO-O), a hydroxyethylene bond (CHOH-$CH_2$), a ketomethylene bond (CO-$CH_2$), a methylene-oxy bond ($CH_2$-O), a reduced bond ($CH_2$-NH), a thiomethylene bond ($CH_2$-S), an N-modified bond (-NRCO-), and a thiopeptide bond (CS-NH). See *e.g.*, Garbay-Jaureguiberry *et al.,* 1992; Tung *et al.*, 1992; Urge *et al.*, 1992; Corringer *et al.,* 1993; Pavone *et al.,* 1993.

Representative peptides that specifically bind to a non-biological substrate are set forth as SEQ ID NOs:1-71. *See* Examples 2-8.

Peptide ligands that specifically bind a biological substrate include peptides with known binding specificities, Including but not limited to: (a) cell-binding peptides listed in Table 1 (SEQ ID NOs:74-98); (b) other peptides known to specifically bind a target substrate; or (c) peptides discovered by display technology as described herein below.

Table 1

| Binding Specificity | Peptide Sequence |
| --- | --- |
| Cell-binding epitopes of fibronectin | GGWSHW (SEQ ID NO:74) |
| | RGD (SEQ ID NO:75) |
| | YIGSR (SEQ ID NO:76) |
| | GRGD (SEQ ID NO:77) |
| | GYIGSR (SEQ ID NO:78) |
| | PDSGR (SEQ ID NO:79) |
| | IKVAV (SEQ ID NO:80) |
| | GRGDY (SEQ ID NO:81) |
| | GYIGSRY (SEQ ID NO:82) |
| | RGDY (SEQ ID NO:83) |
| | YIGSRY (SEQ ID NO:84) |
| | REDV (SEQ ID NO:85) |
| | GREDV (SEQ ID NO:86) |
| | RGDF (SEQ ID NO:87) |
| | GRGDF (SEQ ID NO:88) |
| lung cells | peptides of the format $CX_3CX_3CX_3C$ where X = any amino acid (*e.g.*, CGFECVRQCPERC (SEQ ID NO:89)) |
| fibroblast | RGD (SEQ ID NO:75) |
| | KRSR (SEQ ID NO:90) |
| heparin | KRSR (SEQ ID NO:90) |
| | KRSRGGG (SEQ ID NO:91) |
| muscle (myoblasts) | ASSLNIA (SEQ ID NO:92) |
| smooth muscle cells | KQAGDV (SEQ ID NO:93) |
| endothelial cells | YIGSR (SEQ ID NO:94) |
| | CRRGDWLC (SEQ ID NO:95) |
| fibroblasts and endothelial cells | RGD (SEQ ID NO:75) |
| | RGDS (SEQ ID NO:96) |
| osteoblasts | RGD (SEQ ID NO:75) |
| | KRSK (SEQ ID NO:97) |
| | KRSRGGG (SEQ ID NO:98) |

Peptides of the present invention, including peptoids, can be synthesized by any of the techniques that are known to those skilled in the art of peptide synthesis. Synthetic chemistry techniques, such as a solid-phase Merrifield-type synthesis, are employed for reasons of purity, antigenic specificity, freedom from undesired side products, ease of production, and the like. A summary of representative techniques can be found in Stewart & Young (1969) Solid Phase Peptide Synthesis, Freeman, San Francisco, California, United States of America; Merrifield (1969) Adv Enzymol Relat Areas Mol Biol 32:221-296; Fields & Noble (1990) Int J Pept Protein Res 35:161-214; and Bodanszky (1993) Principles of Peptide Synthesis, 2nd Rev. Ed. Springer-Verlag, Berlin, New York, among other places. Representative solid phase-synthesis techniques can be found in Andersson et al. (2000) Biopolymers 55:227-250, references cited therein, and in U.S. Patent Nos. 6,015,561; 6,015,881; 6,031,071; and 4,244,946. Peptide synthesis in solution is described in Schröder & Lübke (1965) The Peptides, Academic Press, New York, New York, United States of America. Appropriate protective groups useful for peptide synthesis are described in the above texts and in McOmie (1973) Protective Groups in Organic Chemistry, Plenum Press, London, New York. In one embodiment of the invention, a peptide is produced using an automated peptide synthesizer as described in Examples 11-13.

Peptides can also be synthesized by native chemical ligation as described in U.S. Patent No. 6,184,344. Briefly, the ligation step employs a chemoselective reaction of two unprotected peptide segments to produce a transient thioester-linked intermediate. The intermediate spontaneously rearranges to generate the full length ligation product.

Peptides, including peptides comprising non-genetically encoded amino acids, can also be produced in a cell-free translation system, such as the system described by Shimizu et al. (2001) Nat Biotechnol 19:751-755. In addition, peptides having a specified amino acid sequence can be purchased from commercial sources (*e.g.*, Biopeptide Co., LLC of San Diego, California, United States of America, and PeptidoGenics of Livermore, California, United States of America).

Peptides possessing one or more tyrosine residues at an internal position or at the carboxyl terminus of the peptide can be conveniently labeled, for example, by iodination or radio-iodination.

The term "peptide mimetic" as used herein refers to a ligand that mimics the biological activity of a reference peptide, by substantially duplicating the antigenicity of the reference peptide, but it is not a peptide or peptoid. In one embodiment, a peptide mimetic has a molecular weight of less than about 700 daltons. A peptide mimetic can be designed or selected using methods known to one of skill in the art. *See* e.g., U.S. Patent Nos. 5,811,392; 5,811,512; 5,578,629; 5,817,879; 5,817,757; and 5,811,515.

Any peptide or peptide mimetic of the present invention can be used in the form of a pharmaceutically acceptable salt. Suitable acids which can be used with the peptides of the present invention include, but are not limited to inorganic acids such as trifluoroacetic acid (TFA), hydrochloric acid (HCl), hydrobromic acid, perchloric acid, nitric acid, thiocyanic acid, sulfuric acid, phosphoric acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like. In one embodiment, a pharmaceutically acceptable salt is HCl. In another embodiment, a pharmaceutically acceptable salt is TFA.

Suitable bases capable of forming salts with the peptides of the present invention include, but are not limited to inorganic bases such as sodium hydroxide, ammonium hydroxide, potassium hydroxide and the like; and organic bases such as mono-, di-, and tri-alkyl and aryl amines (*e.g.*, triethylamine, diisopropyl amine, methyl amine, dimethyl amine and the like), and optionally substituted ethanolamines (*e.g.,* ethanolamine, diethanolamine and the like).

A peptide ligand of the invention can further comprise one or more crosslinking moieties, such as a photocrosslinkable moiety, an ionically crosslinkable moiety, or terminally crosslinkable moiety. The crosslinking moieties can be used to create a two-dimensional or three-dimensional interfacial biomaterial.

III.B. Antibodies

In another instance of the disclosure, a ligand or non-binding, domain can comprise a single chain antibody. The term "single chain antibody" refers to an antibody comprising a variable heavy and a variable light chain that are joined together, either directly or via a peptide linker, to form a continuous polypeptide. Thus, the term "single chain antibody" encompasses an immunoglobulin protein or a functional portion thereof, including, but not limited to a monoclonal antibody, a chimeric antibody, a hybrid antibody, a mutagenized antibody, a humanized antibody, and antibody fragments that comprise an antigen binding site *(e.g.,* Fab and Fv antibody fragments).

Antibody ligands can be identified by the panning methods described herein below. Alternatively, known single chain antibodies having a desired binding specificity or a desired non-binding quality can be used. For example, U.S. Patent No. 5,874,542 to Rockwell et al. discloses single chain antibodies that specifically bind to vascular endothelial growth factor (VEGF) receptor. VEGF is expressed in macrophages and proliferating epidermal keratinocytes and thus can be used to promote wound healing (Brown *et al.*, 1992). A number of single chain antibodies have been identified that specifically bind to cancer cells (*e.g.*, U.S. Patent Nos. 5,977,322 and 5,837,243), to human immunodeficiency virus (U.S. Patent No. 5,840,300), and to secreted signaling molecules (*e.g.*, tumor necrosis factor (TNF); U.S. Patent No. 5,952,087). These antibody ligands can be useful, for example, drug delivery and detection methods described herein below.

III.C Other Ligands and Non-Binding Domains

A binding agent of the present disclosure can also comprise a ligand that shows specific binding other than a peptide or antibody ligand. Similarly, any suitable non-binding domain that shows substantially no binding to a target substrate can be used to prepare a binding agent. Thus, a ligand or non-binding domain of the disclosure can also comprise a protein, a synthetic polymer, a natural polymer, a polysaccharide, a nucleic acid *(e.g.,* an aptamer), a small molecule *(e.g.,* a chemical compound), a nucleic acid-protein fusion, and/or any other affinity or non-binding agent.

As also disclosed herein a non-binding domain can comprise an anionic polymer or an anionic carbohydrate. These molecules show substantially no cellular binding and thus are useful for inhibiting fibrosis, scar formation, and surgical adhesions. *See e.g.,* U.S. Patent No. 5,705,177. Representative anionic polymers include but are not limited to natural proteoglycans, glycosaminoglycan moieties of proteoglycans, dextran sulfate, pentosan polysulfate, dextran sulfate, or cellulose derivatives. Anionic polymers can be obtained from commercial sources *(e.g.,* Sigma Chemical Company of St. Louis, Missouri, United States of America), purified from a natural source, or prepared synthetically. Methods for polymer purification and synthesis can be found in Budavari (1996) The Merck Index: An Encyclopedia of Chemicals, Drugs, and Biologicals, 12th ed. Merck, Whitehouse Station, New Jersey, United States of America, among other places.

A non-binding domain disclosed herein can also comprise a polysaccharide that shows substantially no binding to platelets can be used as a calcification inhibitor as described in U.S. Patent No. 4,378,224. Suitable calcification inhibitors include natural protein polysaccharides *(e.g.,* chondroitin sulfates and hyaluronate), sulfated polysaccharides, diphosphonates, phosphoproteins, and other polyanions.

A ligand or non-binding domain as disclosed herein can also comprise a small molecule. The term "small molecule" as used herein refers to a compound, for example an organic compound, with a molecular weight in one embodiment of less than about 1,000 daltons, in another embodiment of less than about 750 daltons, in another embodiment of less than about 600 daltons, and in yet another embodiment of less than about 500 daltons. In one embodiment, a small molecule has a computed log octanol-water partition coefficient in the range of about -4 to about +14, and in another embodiment, In the range of about -2 to about +7.5.

III.D. Linkers

Binding agents useful for preparation of an interfacial biomaterial optionally further comprise a linker between a first and second ligand, or between a ligand and a non-binding region. The linker can facilitate combination of two or more ligands. In addition, the linker can comprise a spacer function to minimize potential steric hindrance between the two or more domains.

In one embodiment, the linker does not abrogate or alter ligand binding strength, ligand binding specificity, In one embodiment, the linker is substantially biologically inert except for its linking and/or spacer activities.

Suitable linkers comprise one or more straight or branched chain(s) of 2 carbon atoms to about 50 carbon atoms, wherein the chain is fully saturated, fully unsaturated, or a combination thereof. Typically, a linker comprises between 2 and about one hundred sites for ligand attachment. The methods employed for linking will vary according to the chemical nature of each of a selected ligand, non-binding domain, and linker.

Suitable reactive groups of a linker include, but are not limited to amines, carboxylic acids, alcohols, aldehydes, and thiols. An amine group in a linker can form a covalent bond with a carboxylic acid group of a ligand, such as a carboxyl terminus of a peptide ligand. A carboxylic acid group or an aldehyde in a linker can form a covalent bond with the amino terminus of a peptide ligand or other ligand amine group. An alcohol group in a linker can form a covalent bond with the carboxyl terminus of a peptide ligand or other ligand carboxylic acid group. A thiol group in a linker can form a disulfide bond with a cysteine in a peptide ligand or a ligand thiol group.

Additional reactive groups that can be used for linking reactions Include, but are not limited to a phosphate, a sulphate, a hydroxide, -SeH, an ester, a silane, urea, urethane, a thiol-urethane, a carbonate, a thio-ether, a thio-ester, a sulfate, an ether, or a combination thereof.

In one embodiment of the invention, a linker comprises a peptide. In one embodiment, a peptide linker comprises one (1) to about 40 amino acids. Sites for ligand attachment to a peptide ligand include functional groups of the amino acid side chains and the amino and carboxyl terminal groups. Representative peptide linkers with multiple reactive sites include polylysines, polyornithines, polycysteines, polyglutamic acid and polyaspartic acid. Alternatively, substantially inert peptide linkers comprise polyglycine, polyserine, polyproline, polyalanine, and other oligopeptides comprising alanyl, serinyl, prolinyl, or glycinyl amino acid residues.

Peptide linkers can be pennant or cascading. The term "pennant polypeptide" refers to a linear peptide. As with polypeptides typically found in nature, the amide bonds of a pennant polypeptide are formed between the terminal amine of one amino acid residue and the terminal carboxylic acid of the next amino acid residue. The term "cascading polypeptide" refers to a branched peptide, wherein at least some of the amide bonds are formed between the side chain functional group of one amino acid residue and the amino terminal group or carboxyl terminal group of the next amino acid residue.

In another embodiment of the invention, a linker can comprise a polymer, including a synthetic polymer or a natural polymer. Representative synthetic polymers include, but are not limited to polyethers *(e.g.,* polyethylene glycol; PEG), polyesters *(e.g.,* polylactic acid (PLA) and polyglycolic acid (PGA)), polyamides *(e.g.,* nylon), polyamines *(e.g.,*

polymethylmethacrylate; PMMA), polyacrylic acids, polyurethanes, polystyrenes, and other synthetic polymers having a molecular weight of about 200 daltons to about 1000 kilodaltons. Representative natural polymers include, but are not limited to hyaluronic acid, alginate, chondroitin sulfate, fibrinogen, fibronectin, albumin, collagen, and other natural polymers having a molecular weight of about 200 daltons to about 20,000 kilodaltons. Polymeric linkers can comprise a diblock polymer, a multi-block copolymer, a comb polymer, a star polymer, a dendritic polymer, a hybrid linear-dendritic polymer, or a random copolymer.

A linker can also comprise a mercapto(amido)carboxylic acid, an acrylamidocarboxylic acid, an acrlyamido-amidotriethylene glycolic acid, and derivatives thereof. See U.S. Patent No. 6,280,760.

Methods for linking a linker molecule to a ligand or to a non-binding domain will vary according to the reactive groups present on each molecule. Protocols for linking using the above-mentioned reactive groups and molecules are known to one of skill in the art. See Goldman *et al.,* 1997; Cheng 1996; Neri *et al.,* 1997; Nabel 1997; Park *et al.,* 1997; Pasqualini *et al.*, 1997; Bauminger & Wilchek 1980; U.S. Patent Nos. 6,280,760 and 6,071,890; and European Patent Nos. 0 439 095 and 0 712 621.

## IV. Identification of Ligands Using Phage Display

Display technology is an effective approach for the identification of ligands that specifically bind a substrate, for example phage display methods. According to this approach, a library of diverse ligands is presented to a target substrate, and ligands that specifically bind the substrate are selected. Conversely, ligands that show substantially no binding to a target substrate can also be recovered. Ligands and non-binding domains can be selected following multiple serial rounds of selection called panning.

Any one of a variety of libraries and panning methods can be employed to identify a peptide that is useful in the methods of the invention, as described further herein below.

## V.A. Libraries

As used herein, the term "library" means a collection of molecules. A library can contain a few or a large number of different molecules, varying from about ten molecules to several billion molecules or more. A molecule can comprise a naturally occurring molecule, or a synthetic molecule, which is not found in nature. Optionally, a plurality of different libraries can be employed simultaneously for *in vivo* panning.

Representative libraries include but are not limited to a peptide library (Example 1 and U.S. Patent Nos. 6,156,511, 6,107,059, 5,922,545, and 5,223,409), an oligomer library (U.S. Patent Nos. 5,650,489 and 5,858,670), an aptamer library (U.S. Patent No. 6,180,348 and 5,756,291), a small molecule library (U.S. Patent Nos. 6,168,912 and 5,738,996), a library of antibodies or antibody fragments (U.S. Patent Nos. 6,174,708, 6,057.098, 5,922,254, 5,840,479, 5,780,225, 5,702,892, and 5,667988), a library of nucleic acid-protein fusions (U.S. Patent No. 6,214,553), and a library of any other affinity agent that can potentially bind to a target substrate (*e.g.*, U.S. Patent Nos. 5,948,635, 5,747,334, and 5,498,538).

The molecules of a library can be produced *in vitro,* or they can be synthesized *in vivo,* for example by expression of a molecule *in vivo.* Also, the molecules of a library can be displayed on any relevant support, for example, on bacterial pili (Lu *et al.,* 1995) or on phage (Smith, 1985).

A library can comprise a random collection of molecules. Alternatively, a library can comprise a collection of molecules having a bias for a particular sequence, structure, or conformation. *See e.g.,* U.S. Patent Nos. 5,264,563 and 5,824,483. Methods for preparing libraries containing diverse populations of various types of molecules are known in the art, for example as described in U.S. Patents cited herein above. Numerous libraries are also commercially available.

A library to be used for the disclosed panning methods may have a complexity of at least about $1 \times 10^8$ to about $1 \times 10^9$ different molecules per library. A typical panning experiment with an input of $1 \times 10^{11}$ phage therefore samples on average 100 copies to 1000 copies of each molecule in the library.

The method for panning maybe performed using a phage library. Phage are used as a scaffold to display recombinant libraries and to also provide for recovery and amplification of ligands having a desired binding specificity.

The T7 phage has an icosahedral capsid made of 415 proteins encoded by gene 10 during its lytic phase. The T7 phage display system has the capacity to display peptides up to 15 amino acids in size at a high copy number (415 per phage). Unlike filamentous phage display systems, peptides displayed on the surface of T7 phage are not capable of peptide secretion. T7 phage also replicate more rapidly and are extremely robust when compared to other phage.

A phage library to be used with panning methods can also be constructed in a filamentous phage, for example M13 or M13-derived phage. The ligands may be displayed at the exterior surface of the phage, for example by fusion to M13 vital protein 8. Methods for preparing M13 libraries can be found in Sambrook & Russell (2001) Molecular Cloning: A Laboratory Manual, 3rd ed. Cold Spring Harbor Laboratory Press, Cold Spring Harbor, New York, United States of America, among other places. Representative peptide libraries prepared In M13 phage and that are useful in relation to of the present invention are described in Example 1.

Other suitable phage vectors include the mAEK and mACK vectors, which are derived from an M13mp18 backbone. These versatile vectors are compatible with a wide range of screening formats, including cell-based, solution phase, and solid-phase panning. The mAEK vector provides an independent peptide epitope that is useful in quantitation of peptide for binding and functional assays beyond panning. The mAEK vector also includes a thrombin cleavage site for highly efficient and selective efution of specifically bound phage. Thrombin cleavage also permits "off-phage" assays, in which the peptide module is clipped from the phage prior to conducting the assay. This panning method can be used for experiments that produce unacceptably high background binding when the complete phage particle is present.

Phage vectors typically Include a single allele of the viral coat gene *pIII*, and thus three copies to five copies of identical ligand-Plil fusion proteins are produced on the surface of each recombinant phage. This multiple valency results In increased avidity of selected ligands for target substrates. Thus, phage vectors can be used for primary screens where the goal is typically to identify one or several target-specific binding motifs for further characterization and where high affinity ligands are not essential.

A library used for panning may comprise a phagemid vector. A phagemid is a plasmid that includes both a phage f1 origin of replication, also acting as a packaging signal, and a single copy of the gene encoding Pill containing the expression cassettes described above. Useful phagemid vectors include the pAEK and pACK plasmids, which are derived from the vector pGEM-3z-f(+) (Promega Corporation, Madison, Wisconsin, United States of America).

Phagemid libraries are maintained as plasmids, and they are rescued by superinfection with a packaging-deficient helper phage. Progeny viruses preferentially package the phagemid DNA, which lacks phage genes other than the *pIII* fusion gene. The helper virus provides copies of wild type *pIII*, while the phagemid expresses a lesser amount of recombinant ligand-PIII fusion protein. Thus, most recombinant viruses that express ligand-PIII. fusion proteins express only a single copy. These monovalent libraries tend to result in higher affinity ligands because low affinity binding cannot be compensated by increased avidity. Thus, phagemid vectors can be used for secondary screens to optimize binding motifs and to produce high affinity ligands.

Plasmid expression systems can be used to generate sufficient quantities of ligands and non-binding domains for further characterization in standard binding assays. Alternatively, ligands and non-binding domains selected by panning can be synthesized to appropriate amounts.

As a precursor to chemical synthesis, it is often useful to determine activities of peptide ligands expressed as fusion proteins in standard expression cassettes such as glutathione-S-transferase (GST), green fluorescent protein (GFP), and bacterial alkaline phosphatase (BAP) (Yamabhai & Kay, 2001). These expression modules facilitate expression, stabilization, and purification of peptide ligands and can also serve as indicators of peptide binding.

Peptide Libraries. A peptide library can be used to perform the disclosed panning methods. A peptide library comprises in one instance peptides comprising three or more amino acids, In another instance at least five, six, seven, or eight amino acids, in another instance up to 50 amino acids, in another instance up to 100 amino acids, in another instance up to about 200 amino acids, and in yet another instance up to about 300 amino acids. In one instance a peptide library comprises peptides having a molecular weight of about 500 daltons to about 3500 daltons.

The peptides can be linear, branched, or cyclic, and can include non-peptidyl moieties. The peptides can comprise naturally occurring amino acids, synthetic amino acids, genetically encoded amino acids, non-genetically encoded amino acids, and combinations thereof.

A biased peptide library can also be used, a biased library comprising peptides wherein one or more (but not all) residues of the peptides are constant. For example, an internal residue can be constant, so that the peptide sequence is represented as:

$$(Xaa_1)_m - (AA)_1 - (Xaa_2)_n$$

where $Xaa_1$ and $Xaa_2$ are any amino acid, or any amino acid except cysteine, wherein $Xaa1$ and $Xaa_2$ are the same or different amino acids, $m$ and $n$ indicate a number $Xaa$ residues, wherein in one instance $m$ and $n$ are Independently chosen from the range of 2 residues to 20 residues (*e.g.*, 2, 3, 4, 5, 6, 7, 8, 9, 10, 11, 12, 13, 14, 15, 16, 17, 18, 19 and/or 20 resldues), in another instance m and n are chosen from the range of 4 residues to 9 residues (*e.g.*, 4, 5, 6, 7, 8, and/or 9), and *AA* Is the same amino acid for all peptides in the library. In one instance *AA* is located at or near the center of the peptide. In one instance $m$ and $n$ are not different by more than 2 residues; in another instance $m$ and $n$ are equal.

In one instance libraries are those in which *AA* is tryptophan, proline, or tyrosine. In another instance libraries are those in which *AA* is phenylalanine, histidine, arginine, aspartate, leucine, or isoleucine. In another instance libraries are those in which *AA* is asparagine, serine, alanine, or methionine. In yet another instance libraries are those in which *AA* is cysteine or glycine.

A representative library can be prepared using degenerate codons encoded as NNK, where N = A, C, G, or T and K = G or T. Restriction of the wobble position of the codon reduces, but does not eliminate, the codon bias intrinsic to the genetic code (*e.g.*, 6 codons each for serine, arginine, and leucine, but only one each for methionine and tryptophan) and also eliminates two of the three stop codons. Additional library formats include, but are not limited to those presented in viable 2. In one instance, an $X_6PX_5$ library is employed. In another instance, an $SCX_{16}S$ library is employed. In yet another instance an $X_6YX_6$ library is employed. Representative approaches for library synthesis are also disclosed In the Examples (*see e.g.,* Example 1).

## Table 2

| Library Format | Representation |
|---|---|
| $X_7$ | n-X-X-X-X-X-X- |
| $CX_7C$ | n-C-X-X-X ... \ / ... X ... -C-X-X-X |
| $SSX_{16}S$ | n-S-S-X-X-X-X-X-X-X-X-X-X-X-X-X-X-X-S- |
| $SCX_{16}S$ | n-S-C-X-X-X-X-X-X-X-X-X-X-X-X-X-X-X-S- |
| $SCX_{16}C$ | X-X-X-X-X-X-X / \ n-S-C X : : \| : -C X \ / X-X-X-X-X-X-X |
| $X_6CX_4CX_6$ | n-X-X-X-X-X-C-X : \ : X : \| : X : / -X-X-X-X-X-X-C-X |
| $X_6PX_6$ | n-X-X-X-X-X-X-P-X-X-X-X-X-X- |
| $X_6NX_6$ | n-X-X-X-X-X-X-N-X-X-X-X-X-X- |
| $X_6GX_6$ | n-X-X-X-X-X-X-G-X-X-X-X-X-X- |
| $X_6YX_6$ | n-X-X-X-X-X-X-Y-X-X-X-X-X-X- |
| $X_6HX_6$ | n-X-X-X-X-X-X-H-X-X-X-X-X-X- |

NOTE: X is any amino acid. Solid lines indicate peptide bonds, and dotted lines indicate cysteine-cysteine bonds.

Antibody Libraries.

Panning methods may employ an antibody library. Vectors for the construction of antibody libraries include the pCANTAB-5E or pCANTAB-6 vectors (Amersham Biosciences, Piscataway, New Jersey, United States of America). These vectors contain a constant region single chain fragment variable antibody (scFv) scaffold, and variable sequences are cloned into the vector sequences encoding antibody heavy and light chains. Antibody ligands can be displayed using, for example, an M13 phage vector as described herein above. Methods for constructing an antibody library in M13 or M13-derived phage can be found In U.S. Patent Nos. 6,225,447; 5,580,717; and 5,702,892; among other places.

An antibody library used for the disclosed panning methods can comprise a naive library or an immunized library. Naive antibody libraries can be constructed using IgG hypervariable regions derived from peripheral blood lymphocytes pooled from normal and/or immunologically deficient subjects. Naive libraries are particularly useful in screening targets comprising a poorly immunogenic epitope. Alternatively, an immunized library can be prepared, wherein IgG hypervariable regions are derived from splenocytes of mice previously immunized with the target substrate.

IV.B. Panning Methods

The panning techniques employed in relation to the present invention can comprise solid phase screening, solution phase screening, antibody-directed proximity screening, cell-based screening, tissue-based screening, or a combination thereof. Screening formats are described further herein below. *See also* Examples 2-8.

Methods for recovering of ligands that bind to a substrate are selected based on one or more characteristics common to the molecules present in the library. For example, mass spectrometry and/or gas chromatography can be used to resolve molecules sharing a common core structure. Thus, where a library comprises diverse molecules based generally on the structure of an organic molecule, determining the presence of a parent peak for the particular molecule can identify a ligand.

Alternatively, each of the diverse molecules of a library can comprise a tag that facilitates recovery and identification. For example, a representative tag is an oligonucleotide or a small molecule such as biotin. *See e.g.,* Brenner & Lerner 1992; Norris *et al.,* 1999; Paige *et al.,* 1999; U.S. Patent No. 6,068,829.

A tag can also be a support or surface to which a molecule can be attached. For example, a support can be a biological tag such as a virus or virus-like particle such as a bacteriophage ("phage"); a bacterium; or a eukaryotic cell such as yeast, an insect cell, or a mammalian cell (*e.g.*, an endothelial progenitor cell or a leukocyte); or can be a physical tag such as a liposome or a microbead. Where a molecule is linked to a support, the part of the molecule suspected of being able to interact with a substrate can be positioned so as be able to participate in the interaction.

Solid Phase Screening. Solid phase screening methods are used when the binding substrate comprises a non-biological surface. See Examples 2-8. Solid phase screening also encompasses panning methods in which a biological target is coated on a solid support (*e.g.*, in wells of a microtiter plate), as described in Example 9. This approach requires that a target biological substrate retains at least an approximation of native structure and function when immobilized on a support.

Solution Phase Screening. This approach can be used to identify a ligand that specifically binds to biological substrate in solution. In particular, the method is suited for identification of a ligand that specifically binds to a biological substrate, wherein the biological substrate is bound to other biological components as part of a complex. Solution phase screening is also appropriate in cases in which the binding capacity of a biological substrate is diminished by immobilization on a substrate. According to this approach, the biological substrate, a component complexed therewith, or the ligand is modified to include a tag that facilitates recovery of the substrate, as described herein above.

Antibody-Directed Proximity Screens. If purified target cannot be obtained, an antibody that specifically binds the target can be used to recover ligands that also bind the target. The method is' based on the observation that a ligand typically binds a target substrate, wherein the target substrate is complexed with an antibody or another protein, at sites on the target substrate that are adjacent to the regions bound by the antibody or other associated protein. A bound ligand is detected by horseradish peroxidase (HRP) activation of biotin-tyramine in the presence of hydrogen peroxide. Activated biotin-tyramine then biotinylates any molecule that it contacts. However, activated biotin-tyramine is quenched by water, so that it has an extremely limited radius of diffusion. In this way, only molecules in close proximity to HRP, including phage bound at nearby sites, are biotinylated. Biotinylated phage are partitioned from the population by affinity to streptavidin magnetic beads. Recovered phage are amplified by infection and can then be characterized or subjected to additional rounds of panning. *See* Osbourn *et al.,* 1998a; Osbourn *et al.,* 1998b.

Cell-Based Screening. Target substrates comprising receptors or other molecules present on a cell surface can be used in cell-based screening. This approach involves panning a phage library over a cell population. To select phage that bind a cell-surface molecule, the method includes steps to minimize detection of phage binding to other molecules present at the cell membrane. See Example 9.

One approach for minimizing detection of non-target binding involves differential screening using a mixed population of "labeled" cells that express the receptor and a large excess of "unlabeled" cells lacking the receptor. According to the method, any phage that bind molecules common to both cell populations are preferentially bound to the excess of unlabeled cells and are depleted over multiple rounds of selection. Phage that bind to the target receptor are thereby enriched during multiple rounds of selection.

Another approach combines antibody-directed and cell-based screening methods. If an antibody is available to a cell-surface molecule, or to an epitope-tagged version thereof, the antibody is bound to the target molecule on the cell surface. In this case, the large number of phage that bind to molecules other than the target receptor are not detected because they are biotinylated.

Tissue Screening. Identification of a peptide that specifically binds to biological substrate can also be identified by *in vivo* panning as described in U.S. Patent No. 6,086,829. According to this method, a library of diverse peptides is administered to a subject, or to an isolated target tissue or organ procured from the subject, or fraction thereof, and phage that specifically bind a target tissue or organ are recovered.

V. <u>Applications</u>

An interfacial biomaterial of the present invention can be used in any application where an interaction between two substrates, namely a non-biological substrate and a biological substrate, is desirably controlled. Representative uses of an Interfacial biomaterial of the present invention are described briefly herein below. Disclosed herein are interactions comprising a binding interaction, a non-binding interaction, or a combination thereof. The nature and quality of the interaction relies on the binding specificity, binding strength, or non-binding quality of the plurality of binding agents used to create an interfacial biomaterials.

V.A. <u>Cell Culture</u>

In one embodiment of the invention, an interfacial biomaterial comprises a coating that mediates cell adhesion to a surface for cell culture. Example 14 describes an interfacial biomaterial comprising binding agents that specifically bind to cells and to polystyrene. The interfacial biomaterial is created by adhering a plurality of binding agents to a polystyrene culture plate, and then adhering cells to the plurality of binding agents.

An interfacial biomaterial for cell culture can be used to facilitate culture of any type of cell Including, but not limited to fibroblast cells, aortic endothelial cells, stem cells, embryonic and newborn tissue cells, vertebrate endothelial cells, chondrocytes, osteoblasts, adipocytes, and myoblasts. Cells can be derived from any species including, but not limited to human, primate, porcine, murine, and insect cells.

To create a binding interface between a non-biological substrate and a biological substrate comprising cells, each of a plurality of binding agents can comprise a peptide ligand derived from a cell adhesion molecule, such as any of those listed in Table 1 (SEQ ID NOs:74-98). The term "cell adhesion molecule" refers to any of a family of proteins and peptides found to facilitate cell adhesion or cell attachment to a surface. Alternatively, the binding agents can comprise a peptide ligand that specifically binds extracellular matrix proteins. Representative methods for preparing a binding interface are described in Example 16.

An interfacial biomaterial for cell adhesion to a culture surface can comprise a heterogeneous interface comprising a plurality of non-identical cell-binding peptides. Each of the plurality of binding-agents comprises: (a) a ligand that specifically binds a culture surface substrate; and (b) a variable cell-binding ligand.

A cell culture can be maintained in contact with the interfacial biomaterial under conditions and for a period of time effective to generate a two-dimensional or three-dimensional tissue-like structure, such as a bone-like tissue or a vascularized tissue.

The present invention also encompasses *in vitro* and *ex vivo* cell culture for subsequent transplantation to a subject. Cultured cells can be separated from the interfacial biomaterial and provided to a subject. Another approach involves transplanting to a subject a composition comprising a non-biological substrate, an interfacial biomaterial, and a cellular substrate.

V.B. <u>Biological Arrays</u>

The present invention further provides a method for preparing a biological array. The term "array" generally refers to a pattern of adherent spots and a pattern of biological substrates specifically bound thereto. The term "spot" is used herein to describe a region comprising a binding agent of the present invention specifically bound to a non-biological substrate.

In one embodiment of the invention, a method for preparing a biological array comprises: (a) providing a non-biological substrate having a plurality of positions; (b) applying to each of the plurality of positions a binding agent comprising a first ligand that specifically binds the non-biological substrate and a second ligand that specifically binds a target biological substrate, wherein the applying is free of coupling; (c) contacting the non-biological substrate, wherein a plurality of binding agents are bound to the non-biological substrate, with a sample comprising the target biological substrate; and (d) allowing a time sufficient for binding of the target biological substrate to the plurality of binding agents, whereby a biological array is prepared. A representative method for applying a plurality of binding agents to a plurality of positions comprises dip-pen printing as described in Example 15.

The amount of binding agent dispensed, spot size, and spot shape can be varied by modifying the concentration and volume of dispensed ligand, the temperature at which dispensing is performed, and/or application technique. Typically, a spot dimension comprises a minimal dimension of about 0.2 $\mu$m to about 1.0 $\mu$m, but can comprise a larger minimal dimension as desired for a particular application. It is within the skill of one in the art to optimize spot size, shape, and quantity of binding agent for a particular application, after a review of the disclosure presented herein.

A spot can be any suitable size and shape as appropriate for binding to a target biological substrate. For example, a spot prepared by dispensing a binding agent comprising a cell-binding ligand can comprise a maximal dimension less than or approximately equal to the size of an adhered cell. For example, a white blood cell is approximately

20 μm in diameter, and *Xenopus laevis* oocytes are as large as 1 mm in diameter. When placed on a surface, these cells do not flatten substantially when adhered to a surface. Endothelial cells typically flatten when adhered to a surface and can have an area of approximately 250-4,000 μm$^2$. Similarly, hepatocytes can have an area of approximately 500-10,000 μm$^2$.

The term "inter-spot dimension" refers to a distance between spots of an array. In one embodiment of the invention, an inter-spot dimension is sufficient to distinguish adjacent spots and biological substrates specifically bound thereto. For example, where the patterned interfacial biomaterial is used to prepare a cellular array, the inter-spot dimension is sufficient to prevent contact between cells at adjacent spots. The inter-spot dimension can also be determined to distinguish adjacent spots while permitting interaction of substrates bound thereto.

Thus, a spot can be dimensioned for binding of a single cell. Further, a spot that is substantially smaller than a flattened cell dimension can be used to force an adhered cell to remain in a rounded form. When cell-to-cell contact is desired to affect cellular features (*e.g.*, viability, growth, proliferation, differentiation, protein processing, orientation, spreading), spots capable of adhering more than one cell can be used.

The term "border region" is used herein to describe a region exclusive of one or more spots. The border regions of the non-biological substrate can further comprise a biological substrate adsorbed to or coupled to the borders, or any other treatment desired. For example, cells can be adhered to a border region using serum to facilitate cell binding.

A heterogeneous and patterned interfacial biomaterial is useful for cellular manipulations such as cytometry. For example, a number or ratio of different cell types in a sample can be determined by: (a) applying a binding agent to each of a plurality of positions on a non-biological substrate; (b) contacting a cell suspension with the non-biological substrate; and (c) determining a number of cells bound to the non-biological substrate. A sample can comprise any cellular sample, such as blood, urine, cerebrospinal fluid, a pap smear, biopsy, soil, water, and any other application where there is a desire to determine the presence, number or relative frequency of one or more cell types. An automated detector unit can be used to determine the number of cells bound using a program designed to detect cells at the spot positions. The presence or absence of a cell can be detected using spectrophotometry, detection of a cellular label (*e.g.*, a fluorescent label), or microscopic analysis.

Cellular arrays prepared as disclosed herein are also useful for immobilizing cells for microinjection experiments.

More generally, a biological array of the present invention is useful for screening a plurality of biological substrates in the presence of a test substance. A method for identifying an interacting molecule can comprise: (a) preparing a biological array comprising a plurality of biological substrates, wherein each of the plurality of biological substrates is specifically bound to one of a plurality of positions on a non-biological substrate; (b) contacting the biological array with a candidate substance; (c) allowing a time sufficient for binding of the candidate substance to the biological array; and (d) assaying an Interaction between one or more of the biological substrates and the candidate substance, whereby an interacting molecule is identified.

For example, a biological array used for screening a test substance can comprise a cellular array. An Interacting molecule can be identified by observing a biological outcome, such as a change in cell morphology, In the presence of the test substance.

A method for screening a cellular array can further comprise contacting the cellular array with a detection agent. Representative detection agents include, but are not limited to labeled ligands and labeled nucleic acids. For example, a cell population transfected using recombinant DNA technology can be surveyed to determine a subset of cells that successfully express the transfected DNA.

V.C. Enhancement of an interaction Between Biological Materials

The present disclosure provides an interfacial biomaterial for enhancing an interaction between two or more materials. The materials disclosed herein can be the same or different, and can be biological or non-biological. Also disclosed herein is an interfacial biomaterial comprising a plurality of binding agents wherein each binding agent comprises first and second ligands that specifically bind a biological substrate, and wherein the plurality of binding agents comprise an interface between the biological substrates. In one instance the first and second ligands bind the same biological substrate. In another instance the first and second ligands bind different biological substrates.

Also disclosed herein is a method for preparing an interfacial biomaterial to enhance an interaction between biological materials. A method for preparing an interfacial biomaterial to promote an interaction between biological materials can comprise: (a) adhering to a first biological material a plurality of binding agents, wherein each of the plurality of binding agents comprises a first ligand that specifically binds the first biological material and a second ligand that specifically binds a second biological material; (b) contacting the second biological material with the first biological material with the adhered binding agents; and (c) allowing a time sufficient for binding of the second biological material to the plurality of binding agents.

V.C.1. Coated Implant Devices

The present invention further provides an Interfacial biomaterial for coating implants for improved *in vivo* use. The interfacial biomaterial can be formed prior to (*in vitro* or *ex vivo*) or following (*in vivo*) implantation of an implant device. The term "coating", as used herein to describe applying a coating to a substrate, refers to a contacting a ligand, or a binding agent comprising a ligand, with the substrate and allowing a time sufficient for binding of the ligand or binding agent to the substrate. Representative methods for coating a non-biological substrate are described in Example 14.

The term "implant" generally refers to a non-biological material that can be introduced Into a human or animal body to restore a function of a damaged tissue. An implant device can be created using any biocompatible substrate to which binding agents can specifically bind as disclosed herein. Representative implants include, but are not limited to hip endoprostheses, artificial Joints, jaw or facial implants, tendon and ligament replacements, skin replacements, bone replacements and artificial bone screws, vascular prostheses, heart pacemakers, artificial heart valves, breast implants, penile implants, stents, catheters, shunts, nerve growth guides, intraocular lenses, wound dressings, and tissue sealants.

In one embodiment, a non-biological implant substrate is biocompatible, In another embodiment biodegradable, and has a high surface are to volume ratio to permit cellular growth and transport. For example, suitable non-biological substrates include synthetic polymers and/or copolymers of polylactic acid and polyglycolic acid, which can be processed into highly porous and degradable scaffolds. *See e.g.,* Mikos *et al.*, 1994; Harris *et al.,* 1998.

In one embodiment of the invention, an interfacial biomaterial can create a binding interface that mediates cell attachment to a non-biological implant. Implant devices prepared according to the methods of the present invention control the amount and rate of cell attachment, and thus the rate of tissue integration of the device in *vivo.* Enhanced cell adhesion and tissue integration act to minimize infection by sealing the implant site with a protective layer of cells. This protective cellular layer can also reduce scarring.

Thus; the present invention relates to a method for implanting a device In a subject, wherein the coated implant promotes cell attachment, can comprise: (a) applying to an Implant a plurality of binding agents, wherein each of the plurality of binding agents comprises a first ligand that specifically binds the implant and a second ligand that specifically binds cells at an implant site, wherein the applying is free of coupling; (b) placing the implant In a subject at the implant site; and (c) allowing a time sufficient for binding of the cells to the plurality of binding agents. The term "time sufficient for binding" refers to a time in which host cells can migrate to the vicinity of the implant and bind to the implant via the binding agent.

For example, an interfacial biomaterial to promote incorporation of a silicone breast implant can be prepared using a plurality of binding agents,
wherein each binding agent comprises: (a) a ligand that specifically binds a silicone implant; and (b) a ligand that specifically binds fat cells. The plurality of binding agents is coated onto the silicone breast implant, which is then transplanted into the host. The ligand that specifically binds fat cells promotes cellular attachment to and successful incorporation of the implant.

As another example, a binding agent can comprise a ligand that specifically binds a titanium implant and a ligand that specifically binds cells near the implant site. See Example 13. Representative peptide ligands suitable for binding titanium are set forth as SEQ ID NOs:24-36. Representative cell-binding peptides are listed in Table 1 and in SEQ ID NOs:74-98.

In another embodiment of the invention, suture materials are coated with binding agents that specifically bind the suture material. A coated suture can promote tissue restoration or repair by securing proteins or cells, depending on the binding specificity of the binding agent, at the wound site. Thus a coated suture can provide mechanical strength and closure to the wound.

For wound sites that are not readily accessible or when sutureless intervention is desirable, an interfacial biomaterial comprising a tissue sealant can be used. Such therapeutic "glues" offer advantages including simplicity, rapidity of administration and cellular recovery, and safety. In one embodiment, an interfacial biomaterial for tissue sealing further comprises an adhesion force sufficient to promote tissue repair, including repair of tissues comprising necrotic cells and/or an abnormal amount of moisture. In one embodiment, an interfacial biomaterial does not substantially impair tissue function or structural integrity at the wound site.

A method for preparing an interfacial biomaterial to promote wound healing can comprise: (a) adhering to a biodegradable polymer a plurality of binding agents, wherein each of the plurality of binding agents comprises a first ligand that specifically binds the biodegradable polymer and a second ligand that specifically binds cells; (b) implanting the polymer at a wound site; and (c) allowing a time sufficient for binding of the cells to the plurality of binding agents.

An interfacial biomaterial comprising a tissue sealant is useful for promoting repair of any wound in need of sealing including but not limited to interleaking blebs; tissue severed by surgical intervention, including plastic or reconstructive surgery; bronchopleural fistula, peptic ulcer; tympanic membrane perforation; cornea perforation; corneal transplant; retinal holes; lacerated or ruptured tendons; and tissues subject to plastic and reconstructive repair.

A further embodiment of this invention relates to the use of interfacial biomaterials as an implantable template for highly ordered cellular structures, such as organs, skin, or muscles. An interfacial biomaterial is created using binding agents that specifically bind to the template material and to cells or proteins. The target cells or proteins are assembled on the template via the interfacial biomaterial and then recruit additional cells or matrix, proliferate, or differentiate to create a multicellular organ or tissue. The interfacial biomaterial can be formed *in vitro* or *ex vivo* as described herein above. Alternatively, the interfacial biomaterial can be formed in *vivo* by implantation of a non-biological substrate coated with a plurality of binding agents.

As also disclosed herein, an implant coating can be used to create a non-binding interface. A method for preparing a non-binding Implant coating comprises: (a) applying to the implant a plurality of binding agents, wherein each of the plurality of binding agents comprises a ligand that specifically binds the implant and a non-binding domain that shows substantially no binding to cells at an implant site, wherein the applying is free of coupling; and (b) placing the implant in a subject at the implant site.

A non-binding interface as disclosed herein is useful to prevent or minimize surgical adhesions. Clinically significant adhesions occur in about 5% to about 10% of surgical procedures, and up to nearly 100% for some procedures. Surgical adhesions can result in complications including obstruction, infertility, pain, and the necessity for a second operative procedure. Sea di Zerega 1993; Stangel *et al.,* 1984.

A non-binding interface can be used to prevent the formation of adhesions between Injured tissues by placement of the interfacial biomaterial between the injured tissues. For example, a barrier substrate comprising two surfaces can differentially mediate attachment of healthy cells and non-attachment of injured cells. A first surface of the barrier is coated with a plurality of binding agents, each binding agent comprising a ligand that specifically binds to a non-biological barrier substrate and a non-binding domain that shows substantially no cellular binding. A second surface of the barrier substrate Is optionally coated with a plurality of binding agents, each binding agent comprising a ligand that specifically binds to a non-biological barrier substrate and a ligand that specifically binds cells at the site of the injury. The coated barrier is placed in a subject at the site of injury. In one instance, the non-biological barrier substrate comprises a biodegradable substrate, for example a biodegradable polymer, such that healing occurs with minimal scar or adhesion Formation.

Approaches for prevention of post-surgical adhesion have included administration of linear synthetic and natural polymers (U.S. Patent No. 6,060,582; (Diamond & Dechemey, 1987; Unsky *et al.,* 1987; Leach & Henry, 1990; Steinleitner *et al.,* 1991). In contrast to the methods for preventing or minimizing post-surgical adhesions disclosed herein, these approaches do not use an interfacial biomaterial comprising a plurality of binding agents, wherein each of the plurality of binding agents comprises a ligand that specifically binds a non-biological substrate and a non-binding domain that shows substantially no binding to a biological substrate.

A non-binding interfacial biomaterial can also function as a biological lubricant. An effective boundary lubricant is important for many implant situations where excessive wear occurs between a synthetic implant and a host. Thus, an interfacial biomaterial for lubrication can be prepared using a plurality of binding agents, wherein each of the plurality of binding agents comprises: (a) a ligand that specifically binds an implant; and (b) a non-binding domain that shows substantially no binding to host cells at an implant site.

In another instance of the disclosure an interfacial biomaterial comprising a boundary lubricant can be prepared using a plurality of binding agents, wherein each of the plurality of binding agents comprises: (a) a ligand that specifically binds a first biological substrate; and (b) a non-binding domain that shows substantially no binding to a second biological substrate. For example, each of the plurality of binding agents used to create a boundary lubricant can comprise: (a) a ligand that specifically binds articular cartilage; and (b) a non-binding domain that shows substantially no binding to biological substrates present in synovial fluid. An interfacial biomaterial so prepared can be used, for example, to manage degenerative joint disease by protecting articular cartilage and restoring viscoelastic properties of synovial fluid.

In still another embodiment of the invention, an interfacial biomaterial comprising an implant coating can comprise a heterogeneous interface, wherein regions of the interface show different binding specificities and mediate different *in vivo* processes. For example, an implant coating can comprise both a binding interface and a non-binding interface as described herein above for a barrier substrate. In one embodiment, a heterogeneous interface is patterned by adhering binding agents to a non-biological substrate in a spatially restricted manner.

V.D. Coated Composition for Transplantation

The present invention further provides a method for coating donor transplant cells or tissues to elicit improved viability of the transplant. Synthetic polymer membranes can be used to encapsulate cells for transplantation. For treatment of diabetes, islet of Langerhans cells can be transplanted in a synthetic microcapsule to minimize a post-transplantation immune response in the host (Marik *et al.,* 1999). Shortcomings of this approach include limited viability of the encapsulated islet cells, possibly as a result of poor incorporation of lack of revascularization.

To promote successful transplantation of encapsulated cells or tissues, an interfacial biomaterial can be prepared comprising a plurality of binding agents, wherein each of the plurality of binding agents comprises:

(a) a first ligand that specifically binds to a non-biological microcapsule; and

(b) a second ligand that specifically binds to host cells at a transplant site. Following transplantation, the second ligand mediates cellular integration of encapsulated donor cells and host cells at the transplant site.

## V.E. Diagnosis and Drug Delivery

The present invention further relates to a method for preparing an interfacial biomaterial comprising a therapeutic or diagnostic interface, the method comprising: (a) adhering a plurality of binding agents to a non-biological substrate, wherein each of the plurality of binding agents comprises a first ligand that specifically binds a drug, a detectable label, or a drug carrier, and a second ligand that specifically binds a target cell; (b) administering the non-biological substrate to a subject; and (c) allowing a time sufficient for binding of the target cell to the plurality of binding agents, whereby an Interfacial biomaterial is formed.

Representative ligands that specifically bind a target cell and that can be used to prepare a binding agent as disclosed herein are described in U.S. Patent Nos. 6,068,829 and 6,180,084; PCT International Publication Nos. WO 98/10795 and WO 01/09611; Arap et al. (1998) Science 279:377-380; Staba et al. (2000) Cancer Gene Ther 7:13-19; Wickham et al. (1995) Gene Ther 2:750-756).

Representative non-biological drugs and drug carriers are described herein above. In one embodiment of the Invention, a drug comprises a detectable label. In another embodiment, the label can be detected *in vivo.* Additional non-biological substrates comprising imaging agents, including agents for scintigraphy, magnetic resonance imaging, ultrasound, and fluorescence, are described herein below.

Scintigraphic imaging methods include SPECT (Single Photon Emission Computed Tomography), PET (Positron Emission Tomography), gamma camera imaging, and rectilinear scanning. A non-biological substrate comprising a label for scintigraphic: imaging comprises in one embodiment a radionuclide label, and In another embodiment a radionuclide label selected from the group consisting of [18]fluorine, [64]copper, [65]copper, [67]gallium, [68]gallium, [77]bromine, [80m]bromine, [95]ruthenium, [97]ruthenium, [103]ruthenium, [105]ruthenium, [99m]technetium, [107]mercury , [203]mercury, [123]iodine, [124]iodine, [125]iodine, [126]iodine, [131]iodine, [133]iodine, [111]indium, [113]mindium, [99m]rhenium, [105]rhenium, [101]rhenium, [188]rhenium, [188]rhenium, [121m]tellurium, [122m]tellurium, [125m]tellurium, [165]thulium, [167]thulium, [168]thulium, and nitride or oxide forms derived there from.

Magnetic resonance image-based techniques create images based on the relative relaxation rates of water protons in unique chemical environments. As used herein, the term "magnetic resonance imaging" refers to magnetic source techniques including convention magnetic resonance imaging, magnetization transfer imaging (MTI), proton magnetic resonance spectroscopy (MRS), diffusion-weighted imaging (DWI) and functional MR imaging (fMRI). *See* Rovaris *et al.,* 2001; Pomper & Port 2000; and references cited therein.

Non-biological substrates comprising contrast agents for magnetic source imaging include but are not limited to paramagnetic or superparamagnetic ions, iron oxide particles (Weissleder *et al.,* 1992; Shen *et al.,* 1993), and water soluble contrast agents. Paramagnetic and superparamagnetic ions can be selected from the group of metals including iron, copper, manganese, chromium, erbium, europium, dysprosium, holmium and gadolinium. In one embodiment, the metal is iron, in another embodiment manganese, and in yet another embodiment gadolinium.

Ultrasound imaging can be used to obtain quantitative and structural information of a target tissue. Representative non-biological substrates comprising for providing microbubbles *in vivo* include but are not limited to gas-filled lipophilic or lipid-based bubbles (*e.g.*, U.S. Patent Nos. 6,245,318; 6,231,834; 6,221,018; and 5,088,499). In addition, gas or liquid can be entrapped in porous inorganic particles that facilitate microbubble release upon delivery to a subject (U.S. Patent Nos. 6,254,852 and 5,147,631).

Non-invasive imaging methods can also comprise detection of a fluorescent label. Non-biological substrates comprising fluorescent labels include, but are not limited to carbocyanine and aminostyryl dyes, particularly long chain dialkyl carbocyanines (*e.g.*, Dil, DiO, and DiD available from Molecular Probes Inc. of Eugene, Oregon, United States if America) and dialkylaminostyryl dyes. A fluorescent label can also comprise sulfonated cyanine dyes, including Cy5.5 and Cy5 (available from Amersham of Arlington Heights, Illinois, United States of America), IRD41 and IRD700 (available from Li-Cor, Inc. of Lincoln, Nebraska, United States of America), NIR-1 (available from Dejindo of Kumamoto, Japan), and LaJoila Blue (available from Diatron of Miami, Florida, United States of America). In addition, a fluorescent label can comprise an organic chelate derived from lanthanide ions, for example fluorescent chelates of terbium and europium (U.S. Patent No. 5,928,627).

## V.F. Diagnostic, Affinity Chromatography, and Filtration Applications

The present invention provides compositions and methods for using Interfacial biomaterials for detection and determination of a ligand(s) as well as the isolation of a ligand(s). The Interfacial biomaterial mediates the interaction(s) between a non-biological substrate and a biological substrate. More particularly, the present invention relates to binding agents that create a binding Interface between substrates via specific binding of each substrate. The present invention describes methods used in diagnostic applications whereby a ligand is determined in a liquid medium. The present invention also includes methods for the isolation of a ligand from a liquid medium.

V.F.1. General Considerations for Diagnostic Applications

The present disclosure provides assay methods and reagents used in homogeneous and heterogeneous specific binding type assays for determining qualitatively or quantitatively a ligand in a liquid medium. Ligand amounts in a liquid medium can be determined using a non-competitive binding process (for example, the "Sandwich" technique). In general this assay requires at least two reactive sites in order to bind to both the insoluble/substrate phase containing a specific binding substance and a biotin-labeled specific binding substance. The foregoing is not necessary when a competitive binding process is employed.

Most previous assays rely on streptavidin or avidin interactions with biotin (Hiller *et al.,* 1987). Streptavidin, a tetrameric protein produced by *Streptomyces avidinii,* forms a very strong and specific non-covalent complex with the water-soluble vitamin biotin. The binding affinity is among the highest displayed for non-covalent interactions between a ligand and protein, with an association constant (Ka) estimated to be in the range of $10^{13}$ M$^{-1}$ to $10^{15}$ M$^{-1}$. This binding affinity is such that the binding of streptavidin and biotin is essentially irreversible under most physiological conditions, and provides the basis for the usefulness of these compounds in a wide variety of clinical and industrial applications (Green, 1975).

Both streptavidin and the homologous protein avidin, which shares its high affinity for biotin, have been investigated since they show strong ligand-protein interactions. The X-ray crystal structures of streptavidin and avidin, both in their apo and holo forms, have been described. The sequences of both have also been reported, as well as the construction of several streptavidin fusion proteins. *See e.g.,* Sano and Cantor, 1991; U.S. Pat. No. 4,839,293.

Today, streptavidin/avidin plays a key role in four technological areas of commercial interest: 1) bioseparations/ cell sorting; 2) imaging; 3) drug delivery; and 4) diagnostics (Wilchek and Bayer, 1990). In the separations area, these proteins have been used extensively in cell sorting applications, where, for example, they can be used to remove contaminating cells from hematopoietic stem cells prior to marrow transplantation (Berenson *et al.,* 1992). Streptavidin has also been widely used in both research and clinical settings to test for the presence of various tumor specific biomarkers.

Before the avidin/biotin system can be used in an assay, both the biotin and the avidin need to be chemically modified to incorporate the appropriate functionalities. The preparation of the biotin labeled reagent (for example, a biotin labeled specific binding substance or biotin labeled ligand) may be accomplished by mixing the entity to be labeled with biotin N-hydroxysuccinimide ester (BNHS) in a suitable solvent such as dimethylformamide. Although BNHS is commonly used, other suitable reagents and/or methods may be employed.

Preparation of a substrate or an insoluble phase containing a specific binding substance for the ligand to be determined is accomplished by known methods. For example, the specific binding substance can be attached to a solid carrier by cross-linking, by covalent binding, or by physical coupling. Solid carriers include, but are not limited to polypropylene tubes, polystyrene microtiter plates, and nylon beads. When the ligand to be detected is an antigen, preparation of the insoluble phase can be accomplished by coating the tubes or plates with the appropriate antibody. This binding is non-specific, and consequently the antibody performs two roles: substrate binding and biotin binding. When nylon beads are used, the appropriate antibody may be covalently coupled to the beads by the method of Faulstich (Faulstich *et al.*, 1974).

Various enzymes can be used to produce an enzyme labeled avidin or streptavidin reagent. Enzymes to be conjugated to avidin or streptavidin are chosen based upon the availability of assay-systems that can be used to detect the enzyme either qualitatively or quantitatively. For example, in qualitative determination of a ligand, reagents are commercially available that allow the enzyme to be detected using an assay that produces a colored product.

Enzymes suitable for use in the instant invention include, but are not limited to those classified by the International Union of Biochemists (I.U.B.) as oxidoreductases, hydrolases, and lyases. Exemplary oxidoreductases include, but are not limited to those that act on the CHOH group, the aldehyde or keto group, the $CHNH_2$ group, and those acting on hydrogen peroxide as acceptor. In one embodiment, an oxidoreductase is glucose oxidase. In another embodiment, an oxidoreductase is horseradish peroxidase. Exemplary hydrolases include, but are not limited to those acting on ester bonds (both organic and inorganic esters) and those acting on glycosyl compounds, for example, glycoside hydrolases. In one embodiment, a hydrolase is alkaline phosphatase. In another embodiment, a hydrolase is β-galactosidase.

Other techniques for monitoring the binding of IFBMs to biological or nonbiological materials include, but are not limited to surface plasmon resonance (SPR), Fourier Transform Infrared (FTIR) spectroscopy, RAMAN spectroscopy, and mass spectrometry. *See e.g.,* U.S. Patent Nos. 6,429,015 and 6,428,955.

A general procedure for the determination of a ligand antigen using the "Sandwich" technique is described in Example 20 and is based on U.S. Patent No. 4,298,685 to Parikh et al. Briefly, an appropriately diluted antigen standard or unknown sample is added to an antibody coated polypropylene tubes, which are then incubated at room temperature to allow antigens present in the standard or sample to bind. The tubes are aspirated and washed, and biotin labeled antibody is added and allowed to bind overnight at 4°C. The tubes are then aspirated and washed again, and an appropriate dilution of HRP labeled avidin is added. The tubes are incubated at room temperature for 5-60 minutes, aspirated, and then washed. The enzyme activity in the insoluble phase is determined at timed intervals. When the color intensity of the reaction product is considered suitable, the enzymatic reaction is terminated and the absorbance is measured at an appropriate wavelength. Avidin can also be labeled with alkaline phosphatase instead of HRP.

When alkaline phosphatase-labeled avidin is used in lieu of HRP-labeled avidin, enzyme activity in the insoluble phase is determined by adding 1 ml of 0.05 M sodium carbonate buffer, pH 9.8 containing 1 mg/ml p-nitrophenylphosphate and 1 mM $MgCl_2$. Following an appropriate incubation period, the reaction is terminated with 100 μl of 1 N NaOH and the absorbance at 400 nm is determined.

Enzyme immunoassays conducted in microtiter plates are performed in essentially the same manner as described above. The enzyme assays are conducted using only 250 μl of the substrate solution and terminated with 50 μl of 1 N NaOH. The color intensity is estimated qualitatively, or determined quantitatively by transferring the solution to a 250 μl microcuvette and reading spectrophotometrically.

Other immunoassays systems that can be used with the present invention include those described in U.S. Patent Nos. 4,282,287; 4,298,685; 4,279,992; 4,253,995; 4,230,797; 4,228,237; and 4,208,479.

V.F.2 General Consideration for Affinity Chromatography Applications

The principle of the affinity chromatography separation technique is well known. The present invention describes the use of an interfacial biomaterial adhered to a support to selectively bind a species or ligand. Traditionally, the interaction between the support and the ligand is non-specific. The present invention, however, utilizes specific Interactions, the strength of which can be tuned by optimizing the specific interaction. Consequently, the other species will be carried by the flow of the reaction mixture away from the beginning portion of the column where the immobilized species is, thereby effecting inherent separation of the bound-and free-species. This technique is described in U.S. Patent No. 4,205,058 to Wagner *et al*.

Prior to the disclosure of the present invention, preparation of peptide-coated surfaces and devices has been accomplished by non-specific adsorption, by coupling of the peptide to a derivatized surface, or by coupling of the peptide to a linker molecule covalently attached to the surface. These procedures are relatively tedious and time-consuming, generally require multiple steps for effective association of the peptide and the substrate, often require chemical reactions for immobilization, and can be characterized by difficulty in achieving reproducible surface coverage and loss of maximal activity. The present invention represents a facile method to coat a substrate with a novel multifunctional interfacial biomaterial that can be used In a diagnostic or affinity chromatography application whereby specific tailored strength interactions are present.

Thus, there exists a long-felt need in the art to develop an efficient and widely applicable method for promoting specific interactions between non-biological substrates and biological substrates. In addition, there exists a continuing need to develop methods for directing interactions among molecules and/or cells, particularly in the context of diagnostic and affinity chromatography.

To meet this need, the present invention relates to interfacial biomaterials that can mediate selective interactions between biological and non-biological substrates, novel binding agents that can specifically bind a target non-biological substrate and a target biological substrate, and methods for making and using the same in diagnostic and affinity chromatography applications.

V.G. Non-Fouling Coatings

Also disclosed herein is, an interfacial biomaterial that comprises a non-fouling interface, which is a type of non-binding interface. Non-fouling coatings are useful as a protective treatment for any non-biological substrate susceptible to fouling, including, but not limited to medical equipment, medical devices, clothing, and marine machines and articles of manufacture.

Also enclosed herein are interfacial biomaterials that create a non-adhesive interface to thereby prevent fouling and corrosion. The term "fouling" refers to a process of becoming dirty, contaminated, corroded, or clogged. Conversely, the term "non-fouling" refers to a quality of preventing or minimizing fouling. Thus, a non-fouling interfacial biomaterial can be used to reduce attachment of pathogens and other organisms to a surface, and to reduce aesthetic and operational consequences of fouling.

Current anti-fouling coatings comprise toxic chemicals that are consumable and that pollute the environment Thus, there exists a need In the art for methods for treating a variety of substrates with a non-toxic and long-lasting protective coating.

Fouling includes the steps of: (1) attachment to and colonization of a surface by pathogens, (2) secretion of an extracellular matrix and formation of a biofilm, and (3) attachment of other pathogens and/or multicellular organisms to the biofilm. Thus, an interfacial biomaterial comprising a surface that shows substantially no binding to target pathogens could effectively reduce fouling.

A non-fouling interfacial biomaterial is prepared using a plurality of binding agents, wherein each of the plurality of binding agents comprises a first ligand that specifically binds a non-biological substrate susceptible to fouling and a second ligand that shows substantially no binding to a target organism that mediates fouling (*e.g.,* bacteria, fungi, or any other pathogen).

Substrates that are susceptible to fouling and that can be protected using an interfacial biomaterial Include, but are not limited to medical devices, textiles, and surfaces subjected to an aqueous environment. In each case, a first ligand that specifically binds the non-biological substrate susceptible to fouling can be identified using the panning methods disclosed herein. Similarly, a second ligand that specifically binds to a suspected pathogen or to a combination of pathogens can also be identified by panning.

An interfacial biomaterial disclosed herein can also comprise a non-fouling coating for Implantable devices. Such a coating could be useful, for example, for coating central venous catheters used for chemotherapy, antibiotics and ionotropic support, intravenous nutrition, monitoring of hemodynamic status, venous access for diagnostic blood tests, etc. The incidence of hospital-acquired infection is seven-fold higher in patients with invasive devices such as central venous catheters (Dobbins *et al.,* 1999), and catheter-related infection has a mortality rate of 35% (Collin, 1999). Thus, there exists a need for a reliable method for inhibiting fouling of catheters and other implantable devices.

Catheter-related infections can Involve *S, epidermis, S. auras, Bacillus* species, *Corynebacterium* species, *Pseudomonas aeruginosa, Acinetobacter,* fungal organisms (*e.g., Candida*), and other infectious agents. Host proteins (*e.g.,* fibronectin, fibrinogen, laminin) and qualities of the catheter surface (*e.g.,* charge, hydrophobicity) can contribute to adherence of infectious agents to the catheter surface. Thus, an interfacial biomaterial disclosed herein can comprise a plurality of binding agents, wherein each of the plurality of binding agents comprises a first ligand that specifically binds a catheter substrate and a second ligand that shows substantially no binding to one or more infectious agents. Thus, an interfacial biomaterials so prepared could prevent bacterial and/or fungal colonization of the catheter and thereby reduce catheter related infection.

A non-fouling interfacial biomaterial can also be used to coat fabric, clothing, and clothing fibers of natural or synthetic origin. For example, clothing intended for extended wear or for use in conditions that are permissive to bacterial growth could be used for a longer period of time if protected by an interfacial biomaterial having non-fouling properties.

Non-fouling interfacial biomaterials are also useful for coating surfaces subjected to an aqueous environment. Such a non-fouling coating can minimize a rate of corrosion and other detrimental effects of operation. Representative surfaces that can be treated Include, but are not limited to ship hulls, drilling platforms, pilings, cooling towers, ponds retainers, pumps, valves, oil pipes, water-conducting pipes, glass and other transparent observation windows, sonar domes, and filtration members. For example, a non-fouling coating can be used to prevent adherence of barnacles to surfaces required in a marine setting.

V.H. Modulating an Activity of a Biological Substrate

In another embodiment, the present invention relates to a method for modulating an activity of a biological substrate, the method comprising (a) coating a biodegradable, non-biological substrate with a plurality of binding agents, wherein each of the plurality of binding agents comprises a first ligand that specifically binds the biodegradable, non-biological substrate and a second ligand that specifically binds the biological substrate, wherein the coating is free of coupling; (b) placing the coated biodegradable, non-biological substrate at a target site, wherein the biological substrate is present at the target site; and (c) allowing a time sufficient for binding of the biological substrate at the target site to the binding agents, wherein the binding modulates the activity of the biological substrate.

As used herein, the terms "modulate", "modulating", and "modulated" all refer to an increase, decrease, or other alteration of any or all chemical and biological activities or properties of a biological substrate. In one embodiment, a biological substrate is selected from the group consisting of a tissue, a cell, a macromolecule, and combinations thereof. In one embodiment, a cell is a vascular endothelial cell. In another embodiment, a cell is a tumor vascular endothelial cell. In one embodiment, a macromolecule is a Tie2 receptor.

As used herein, the term "modulator" refers to a second ligand of the method that specifically binds the biological substrate. In one embodiment of the invention, a modulator is an agonist of biological substrate. As used herein, the term "agonist" means a substance that synergizes or potentiates the biological activity of a biological substrate. In another embodiment of the invention, a modulator is an antagonist of a biological substrate. As used herein, the term "antagonist" or "inhibitor" refers to a substance that blocks or mitigates the biological activity of a biological substrate. In one embodiment, a modulator specifically binds a Tie2 receptor.

As used herein, the term "target site" refers to any cell or group of cells, either *in vivo, in vitro,* or *ex vivo.* This term includes single cells and populations of cells. The term includes but is not limited to cell populations comprising glands and organs such as skin, liver, heart, kidney, brain, pancreas, lung, stomach, and reproductive organs. It also includes but is not limited to mixed cell populations such as bone marrow. Further, it includes but is not limited to such abnormal cells as neoplastic or tumor cells, whether individually or as a part of solid or metastatic tumors.

The term "target site" as used herein additionally refers to an intended site for accumulation of a ligand following administration to a subject. In one embodiment, a target site is a wound site and the modulating enhances wound healing. In another embodiment, a target site is an angiogenic site, including, but not limited to a site of tumor angiogenesis, and the modulating inhibits angiogenesis.

Example

It will be understood that not all of the Examples below fall within the scope of the invention, in relation to which reference is made to the appendant claims.

The following Examples have been included. Certain aspects of the following Examples are described in terms of techniques and procedures found or contemplated by the present co-inventors to work well in the practice of the invention. These Examples illustrate standard laboratory practices of the present co-inventors. In light of the present disclosure and the general level of skill in the art, those of skill will appreciate that the following Examples are intended to be exemplary only and that numerous changes, modifications, and alterations can be employed without departing from the scope of the invention.

Example 1

Peptide Libraries

Three phage peptide libraries were used: (a) a library encoding peptides of the format $X_6YX_6$; (b) a library encoding peptides of the format $X_6PX_6$, and (c) a library encoding peptides of the format $SCX_{16}S$.

The $X_6YX_6$ library was constructed using variable sequences comprising 39 nucleotides ligated to the 5' terminus of the *pIII* gene of filamentous phage M13. Peptides produced by the library were 13-mer peptide sequences with a fixed central tyrosine residue flanked by six random amino acids on each side.

The following is provided as an exemplary library construction scheme for the $X_6YX_6$ library. A similar strategy can be used for the other libraries, which can also be produced using techniques that are well known in the art.

To produce the $X_6YX_6$ library, an oligonucleotide of sequence AGTGTGTGC<u>CTCGAG</u>CNNKNNKNNKNN-KNNKNNK**TAT**NNKNNKNNKNN KNNKNNK<u>TCTAGA</u>CTGTGCAGT (SEQ ID NO:99)was built In which the NNKNNKNNKNNKNNKNNKTATNNKNNKNNKNNKNNKNNK module (SEQ ID NO:100) represents the library. The underlined CTCGAG and TCTAGA sequences represent the Xhol and Xbal restriction endonuclease sites used to clone the library into the phage vector. The bolded **TAT** sequence represents a tyrosine codon. N represents equilmolar mixtures of A, C, G and T. K represents equimolar G and T.

The $X_6PX_6$ library was constructed using the filamentous phage M13. Peptides produced by the library were 13-mer peptide sequences with a fixed central proline residue flanked by six random amino acids on each side.

The $SCX_{16}S$ library encoding 19-mer peptides, wherein each peptide includes 16 central random amino acids, a serine at each terminus, and a single cysteine residue. The peptides were displayed on the amino terminus of the PIII coat protein of the M13 phage.

Example 2

Isolation of Peptides that Specifically Bind Polystyrene

The $X_6PX_6$, $X_6YX_6$, and $SCX_{16}S$ libraries (described in Example 1) were screened for binding to polystyrene using a 96-well high binding microtiter plate (COSTAR® polystyrene plates available from VWR Scientific of West Chester, Pennsylvania, United States of America). Nonspecific protein binding sites were blocked using 100 μl of 5% dry milk in phosphate buffered saline plus TWEEN® (PBS-T). The plate was sealed and incubated for 1 hour at room temperature with shaking at 50 rpm. The wells were then washed 5 times with 300 μl of PBS-T, ensuring that the wells did not-dry out. The library was diluted in PBS-T and was added at a concentration of $10^{10}$ pfu/ml in a total volume of 100 μl. After another 1 hour incubation at room temperature and shaking at 50 rpm, unbound phage were removed by 5 washes of 300 μl PBS-T. Bound phage were eluted for 30 minutes at 150 rpm with 3 μg/μl thrombin. After elution, 1.5 μl mM D-phenylalanyl-L-prolyl-L-arginine chloromethylketone (PPACK) was added and serial dilutions were made for titer determination.

To ensure production of highest titer phage stocks, eluted phage were added to 5 ml of undiluted exponential phase TG1 cultures in 2X YT media. The mixture was incubated for approximately three hours in a 37°C shaker at 210 rpm. Phage supernatant was then harvested for titer determination after spinning at 8500 x g for 10 minutes. Second and third rounds of selection were performed in a similar manner to that of the first round, using the amplified phage from the previous round as input.

To detect phage that specifically bound to titanium, conventional ELISAs were performed using an anti-M13 phage antibody conjugated to HRP, followed by the addition of chromogenic agent o-phenylenediamine in 10% hydrogen peroxide. Relative binding strengths of the phage were determined by absorbance measurements at 490 nm using a microtiter plate reader.

The DNA encoding peptides that specifically bound polystyrene was sequenced by the chain terminator method using a reverse primer designed according to the *pIII* sequence. The sequence encoding the peptide insert was located in the phage genome and translated to yield the corresponding amino acid sequence displayed on the phage surface.

Representative peptides that specifically bind to polystyrene are listed in Table 3 and are set forth as SEQ ID NOs:1-22.

Table 3

| Sequence | SEQ ID NO. |
| --- | --- |
| FLSFVFPASAWGG | 1 |
| FYMPFGPTWWQHV | 2 |
| LFSWFLPTDNYPV | 3 |
| FMDIWSPWHLLGT | 4 |
| FSSLFFPHWPAQL | 5 |
| SCAMAQWFCDRAEPHHVIS | 6 |
| SCNMSHLTGVSLCDSLATS | 7 |
| SCVYSFIDGSGCNSHSLGS | 8 |
| SCSGFHLLCESRSMQRELS | 9 |
| SCGILCSAFPFNNHQVGAS | 10 |
| SCCSMFFKNVSYVGASNPS | 11 |
| SCPIWKYCDDYSRSGSIFS | 12 |
| SCLFNSMKCLVLILCFVS | 13 |
| SCYVNGHNSVWVVVFWGVS | 14 |
| SCDFVCNVLFNVNHGSNMS | 15 |
| SCLNKFFVLMSVGLRSYTS | 16 |
| SCCNHNSTSVKDVQFPTLS | 17 |
| FFPSSWYSHLGVL | 18 |
| FFGFDVYDMSNAL | 19 |
| LSFSDFYFSEGSE | 20 |
| FSYSVSYAHPEGL | 21 |
| LPHLIQYRVLLVS | 22 |
| CGSSLVGLHSYWSSPFF | |

Example 3

Isolation of Peptides that Specifically Bind Polyurethane

The SCX$_{16}$S library (described in Example 1) was screened for binding to polyurethane. Phage were detected, isolated, amplified, and sequenced as described in Example 2.

A representative peptide that specifically binds polyurethane is SCYVNGHNSVWVVVFWGVS (SEQ ID NO 23).

Example 4

Isolation of Peptides that Specifically Bind Polyglycolic Acid (PGA)

The $SCX_{16}S$ library (described in Example 1) was screened for binding to polyglycolic acid. Polyglycolic acid (PGA) mesh was washed repetitively before panning in an excess of water

Prior to adding phage to the PGA scaffold, the phage were sequentially transferred between polystyrene wells targets in order to extract polystyrene-binding and nonspecific-binding phage from the population. This step was performed at each round of panning. Nonspecific binding sites were also blocked with 1% BSA in PBS during odd-numbered rounds of panning and with 5% dry milk in PBS-T during even-numbered rounds of panning. Alternation of the BSA and dry milk blocking proteins prevented survival of peptides that specifically bind BSA and dry milk between rounds. Phage were detected, isolated, amplified, and sequenced as described in Example 2.

Representative peptides that specifically bind polyglycolic acid are listed in Table 4 and are set forth as SEQ ID NOs:37-50.

Table 4

| Sequence | SEQ ID NO. |
|---|---|
| SCNSFMFINGSFKETGGCS | 37 |
| SCFGNLGNLIYTCDRLMPS | 38 |
| SCSFFMPWCNFLNGEMAVS | 39 |
| SCFGNVFCVYNQFAAGLFS | 40 |
| SCCFINSNFSVMNHSLFKS | 41 |
| SCDYFSFLECFSNGWSGAS | 42 |
| SCWMGLFECPDAWLHDWDS | 43 |
| SCFWYSWLCSASSSDALIS | 44 |
| SCFGNFLSFGFNCESALGS | 45 |
| SCLYCHLNNQFLSWVSGNS | 46 |
| SCFGFSDCLSWFVQPSTAS | 47 |
| SCNHLGFFSSFCDRLVENS | 48 |
| SCGYFCSFYNYLDIGTASS | 49 |
| SCNSSSYSWYCWFGGSSPS | 50 |

Example 5

Isolation of Peptides that Bind Polycarbonate

The $X_6NX_6$, $SCX_{16}S$, and $X_6PX_6$ libraries (described in Example 1) were screened for binding to polycarbonate. Polycarbonate sheets were washed repetitively with ethanol and water before use.

Prior to adding phage to the polycarbonate sheets, phage were sequentially transferred between polystyrene wells targets in order to extract polystyrene-binding and nonspecific-binding phage from the population. This step was performed at each round of panning. Nonspecific binding sites were also blocked with 1% BSA in PBS during odd-numbered rounds of panning and with 5% dry milk in PBS-T during even-numbered rounds of panning. Alternation of the BSA and dry milk blocking proteins prevented survival of peptides that specifically bind BSA and dry milk between rounds. Phage were detected, isolated, amplified, and sequenced as described in Example 2.

Representative peptides that specifically bind polycarbonate are listed in Table 5 and are set forth as SEQ ID NOs:66-71.

Table 5

| Sequence | SEQ ID NO. |
|----------|------------|
| FGHGWLNTLNLGW | 66 |
| FSPFSANLWYDMF | 67 |
| VFVPFGNWLSTSV | 68 |
| FWNVNYNPWGWNY | 69 |
| FYWDRLNVGWGLL | 70 |
| LYSTMYPGMSWLV | 71 |

Example 6

Isolation of Peptides that Bind Nylon Sutures

The $X_6YX_6$ library (described in Example 1) was screened for binding to nylon sutures. Nylon sutures were washed repetitively with ethanol and water before use.

Prior to adding phage to the nylon sutures, phage were sequentially transferred between polystyrene wells targets in order to extract polystyrene-binding and nonspecific-binding phage from the population. This step was performed at each round of panning. Nonspecific binding sites were also blocked with 1 % BSA in PBS during odd-numbered rounds of panning and with 5% dry milk in PBS-T during even-numbered rounds of panning. Alternation of the BSA and dry milk blocking proteins prevented survival of peptides that specifically bind BSA and dry milk between rounds. Phage were detected, isolated, amplified, and sequenced as described in Example 2. Representative sequences are as follows (SEQ ID NOs:105-116):

ssMASMTGGQYMGHsr
ssMASMTGGQWMGHsr
ssSCFYQNVISSSFAGNPWECsr
ssSCNMLLNSLPLPSEDWSACsr
ssSCPFTHSLALNTDRASPGCsr
ssSCFESDFPNVRHHVLKQSCsr
ssSCVFDSKHFSPTHSPHDVCsr
ssSCGDHMTDKNMPNSGISGCsr
ssMASMTGGQWMGHsr
ssSCDFFNRHGYNSGCEHSVCsr
ssSCGDHMTDKNMPNSGISGCsr
ssSCYYNGLVVHHSNSGHKDCsr.

Example 7

Isolation of Peptides that Specifically Bind Titanium

The $SCX_{16}S$ library (described in Example 1) was screened for binding to titanium beads. Commercially pure titanium beads of approximately 25 $\mu$m diameter were washed repetitively before panning in an excess of hexanes and ethanol to remove any surface organics. Twenty-five titanium beads were placed in wells of 96-well polystyrene plates.

Prior to adding phage to the titanium beads, phage were sequentially transferred between polystyrene wells targets in order to extract plastic binding and nonspecific binding phage from the population. This step was performed at each round of panning. Nonspecific binding sites were also blocked with 1% bovine serum albumin (BSA) in phosphate-buffered saline (PBS) during odd-numbered rounds of panning and with 5% dry milk in PBS-T during even-numbered rounds of panning. Alternation of the BSA and dry milk blocking proteins prevented survival of peptides that specifically bind BSA and dry milk between rounds. Phage were detected, isolated, amplified, and sequenced as described in Example 2.

Representative peptides that specifically bind titanium are listed in Table 6 and are set forth as SEQ ID NOs: 24-36.

Table 6

| Sequence | SEQ ID NO. |
|---|---|
| SCFWFLRWSLFIVLFTCCS | 24 |
| SCESVDCFADSRMAKVSMS | 25 |
| SCVGFFCITGSDVASVNSS | 26 |
| SCSDCLKSVDFIPSSLASS | 27 |
| SCAFDCPSSVARSPGEWSS | 28 |
| SCVDVMHADSPGPDGLN8 | 29 |
| SCSSFEVSEMFTCAVSSYS | 30 |
| SCGLNFPLCSFVDFAQDAS | 31 |
| SCMLFSSVFDCGMLISDLS | 32 |
| SCVDYVMHADSPGPDGLNS | 33 |
| SCSENFMFNMYGTGVCTES | 34 |
| SCSSFEVSEMFTCAVSSYS | 35 |
| SCGLNFPLCSFVDFAQDAS | 36 |

Example 8

Isolation of Peptides that Bind Stainless Steel

The $X_6HX_6$, $SCX_{16}S$, $X_6YX_6$, $X_7$, and $X_6NX_6$ libraries (described in Example 1 and Table 1) were screened for binding to stainless steel. Stainless steel beads were washed repetitively with ethanol and water before use.

Prior to adding phage to the stainless steel beads, phage were sequentially transferred between polystyrene wells targets in order to extract plastic binding and nonspecific binding phage from the population. This step was performed at each round of panning. Nonspecific binding sites were also blocked with 1% BSA in PBS during odd-numbered rounds of panning and with 5% dry milk in PBS-T during even-numbered rounds of panning. Alternation of the BSA and dry milk blocking proteins prevented survival of peptides that specifically bind BSA and dry milk between rounds. Phage were detected, isolated, amplified, and sequenced as described in Example 2.

Representative peptides that specifically bind stainless steel are listed in Table 7 and are set forth as SEQ ID NOs:51-65.

Table 7

| Sequence | SEQ ID NO. |
|---|---|
| CFVLNCHLVLDRP | 51 |
| SCFGNFLSFGFNCEYALGS | 52 |
| DGFFILYKNPDVL | 53 |
| NHQNQTN | 54 |
| ATHMVGS | 55 |
| GINPNFI | 56 |
| TAISGHF | 57 |
| LYGTPEYAVQPLR | 58 |
| CFLTQDYCVLAGK | 59 |
| DGFFILYKNPDVL | 60 |
| VLHLDSYGPSVPL | 61 |
| VLHLDSYGPSVPL | 62 |

(continued)

| Sequence | SEQ ID NO. |
|---|---|
| VVDSTGYLRPVST | 63 |
| VLQNATNVAPFVT | 64 |
| WWSSMPYVGDYTS | 65 |

Example 9

Isolation of Peptides that Specifically Bind Chondrocytes

The SCX$_{16}$S library (described in Example 1) was screened for binding to chondrocytes. The peptides of the library were of the format SCX$_{16}$S, including 16 central random amino acids, terminal fixed serines and a single cysteine residue. The peptides are displayed on the amino terminus of the pIII coat protein of the M13 phage.
Human chondrocytes were obtained from Clonetics, Inc. (San Diego, California, United States of America) and grown to confluency on one well of a polystyrene 6-well plate in supplemented F-12 media (Sigma-Aldrich Corp., St. Louis, Missouri, United States of America). The entire cell panning procedure was free of detergent. The library was pre-cleared of phage that specifically or non-specifically bind polystyrene by incubating phage in polystyrene wells for two hours prior to addition to the cellular target. In each round, nonspecific binding sites were blocked using 5% dry milk in PBS. Phage were detected, isolated, amplified, and sequenced as described in Example 2. A representative peptide has the sequence SCSVYDHKIGRDSFYSGCS (SEQ ID NO:101). A representative peptide also has a preference for chondrocytes greater than 10 fold over endothelial cells.

Example 10

Isolation of Peptides that Bind Collagen

Collagen beads (bovine type I and type III collagen from BD Biosciences, Bedford, Massachusetts, United States of America) were screened in a manner as we have previously. A mixed library (X$_7$, X$_6$GX$_6$, X$_6$PX$_6$, X$_6$HX$_6$, X$_6$YX$_6$, X$_6$NX$_6$, SCX$_{16}$S, SSX$_{16}$S, and X$_6$CX$_4$CX$_9$) was used to determine if there is a peptide structural motif that possesses preferential binding to collagen. As before, the collagen sample is blocked with either milk or BSA at each round before phage are added. The collagen beads with bound phage are washed (5X) and then added to *E. coli* cells for subsequent infection and amplification. The phage are isolated from the cells and added to a new collagen sample and the procedure is repeated.
Phage were detected, isolated, amplified, and sequenced as described in Example 2.

Example 11

Synthesis of a Labeled Polystyrene-Binding Peptide

The peptide fluorescein-FLSFVFPASAWGG (SEQ ID NO:1 was synthesized using an automated peptide synthesizer according to the directions provided by the manufacturer. After cleavage from the resin, the peptides were washed, purified by high performance liquid chromatography (HPLC), and characterized by mass spectroscopy. This peptide possesses a plastic binding domain (FLSFVFPASAWGG; SEQ ID NO:1) and a fluorescent probe (fluorescein).

Example 12

Synthesis of a Binding Agent Comprising a Polystyrene-Binding Peptide and a Cell-Binding Peptide

The peptide FLSFVFPASAWGGSSGRGD (SEQ ID NO:72) was synthesized using an automated peptide synthesizer according to the directions provided by the manufacturer. After cleavage from the resin, the peptides were washed, purified by HPLC, and characterized by mass spectroscopy. This peptide possesses a cell binding domain (RGD; SEQ ID NO:75) and a plastic binding domain (FLSFVFPASAWGG; SEQ ID NO:1).

Example 13

Synthesis of a Binding Agent Comprising a Titanium-Binding Peptide and a Cell-Binding Peptide

The peptide SCSDCLKSVDFIPSSLASSRGD (SEQ ID NO:103) was synthesized using an automated peptide synthesizer according to the directions provided by the manufacturer. After cleavage from the resin, the peptides were washed, purified by HPLC, and characterized by mass spectroscopy. This peptide possesses a cell-binding domain (RGD; SEQ ID NO:75) and a titanium-binding domain (SCSDCLKSVDFIPSSLASS; SEQ ID NO:27).

Example 14

Coating of Polystyrene with a Peptide Ligand

A piece of polystyrene was dipped in an aqueous solution of a binding agent comprising a peptide that specifically binds polystyrene (for example, any one of SEQ ID NOs:1-22). The polystyrene was then washed with copious amounts of PBS pH 7.4 and then dried. A decrease in contact angle from 70° to 28° was observed, indicating that the peptide ligand was coated on the polystyrene surface.

Additional methods for applying a peptide ligand or binding agent on a non-biological substrate including brushing and spraying a solution comprising the ligand or binding agent. A non-biological substrate can also be coated with a dissolvable sacrificial material, then coated with the ligand or binding agent, followed by removal of the sacrificial material to afford a pattern. Representative methods for using a sacrificial material can be found in Clark et al. (2001) J Am Chem Soc 123:7677-7682, among other places.

Example 15

Applying a Binding Agent to Polystyrene Using Pin-Dip Technology

Peptides that specifically bind polystyrene, or binding agents comprising a peptide that specifically binds polystyrene, were diluted to a concentration of 25 mg/ml in a solution of 90 parts PBS pH 7.4 and 10 parts dimethyl sulfoxide (DMSO). Solutions were then patterned in duplicate onto distinct wells of a 12-well tissue culture polystyrene plate using a pin arrayer (Cartesian Technologies, Inc. of Irvine, California, United States of America). A 10 x 10 array of islands was prepared by applying one hundred spots, each approximately 40 $\mu$m in diameter, with vertical and horizontal spacing of 500 $\mu$m. A line pattern was applied with an array of 400 spots of horizontal spacing 70 $\mu$m and vertical spacing 750 $\mu$m.

Example 16

Preparation of an Interfacial Biomaterial for Cell Culture

A 25 mg/mL solution was prepared using a binding agent comprising the peptide sequence RGDFLSFVFPASAWGG (SEQ ID NO:72) in a mixture of 90 parts PBS pH 7.4 and 10 parts DMSO. Fifty (50) $\mu$l of the binding agent solution was to each of three wells of a 96-well polystyrene microtiter plate for a duration of 1 hour. In another 3 wells, 50 $\mu$l of a control peptide 25 mg/ml fluorescein-labeled FLSFVFPASAWGG (SEQ ID NO:1) was added. The wells were washed three times with PBS and non-specific protein binding sites were blocked with sterile-filtered BSA (3% in PBS) for 30 minutes with shaking at 25 rpm. As a negative control, 3 additional wells were blocked with the BSA solution and did not contain a polystyrene-binding peptide or a binding agent comprising a polystyrene-binding peptide. Following 4 washes with PBS, human umbilical vein endothelial cells (HUVECs) in supplemented EBM media were seeded onto each well. Cell adhesion and spreading was monitored by slight microscopy following a 1-hour, 2-hour, or overnight culture. Wells coated with the binding agent comprising SEQ ID NO:72 showed increased cell adhesion and cell spreading when compared to wells coated with a peptide that specifically binds polystyrene but lacks a cell-binding domain, or with uncoated cells.

Example 17

Isolation of a Single Chain Antibody to the Tie2 Receptor

mRNA from splenocytes of mice immunized with the extracellular domain of human Tie2 was prepared. A set of primers specific for the heavy and light chain variable regions expressed in murine B lymphocytes was used to reverse transcribe and amplify these antibody fragments. The heavy and light chain genes were joined with a flexible linker to form a single chain fragment variable (scFv) antibody. The single chain antibodies were cloned into the pCANTAB 5E phagemid vector (Amersham Biosciences Corp., Piscataway, New Jersey, Untied States of America), allowing their expression as fusion proteins on the surface of phage. Selection for phage clones binding the Tie2 receptor was carried out using the purified extracellular domain of the Tie2 receptor (ExTek). During iterative selection, binding levels of the pooled selected phage clones to the targeted ExTek protein increased with each round of selection, as measured by ELISA, and appeared to plateau by the second round. Binding of these selected phage clones to an unrelated control protein and to the blocking agent remained negligible throughout the iterative selection.

Individual clones were picked from the first and second round selected pools for evaluation of clonal heterogeneity by DNA fingerprint analysis. These studies showed that a dominant species has already begun to emerge by round 2. Therefore, subsequent analyses were restricted to the more heterogeneous clones isolated from the round 1 selected pool.

Individual clones from the Round 1 selected pool were tested for affinity to Tile2 and controls. Representative clones demonstrated specific binding to a purified extracellular domain of the Tie2. receptor (ExTek) but not to a purified extracellular domain of the closely related receptor tyrosine kinase Fms (ExFms). A non-binding clone (1C8) was carried forward as a negative control.

These clones were also tested by cellular ELISA for their ability to recognize Tie2 expressed on the surface of 293 cells. Numerous clones were identified that bind to 293 cells stably transfected to express Tie2. These clones did not bind the parental 293 cells lacking Tie2 receptor.

Soluble single chain antibodies were expressed in a non-suppressor strain and purified from periplasmic extracts using an antibody against the C-terminal E-peptide tag on the soluble scFvs. This system produces pure scFv in sufficiently high quantities for detailed molecular analysis (> 500 $\mu$g from the periplasmic extract of one liter of bacteria).

Additional experiments demonstrated that one of these scFv, 1B1, was capable of inhibiting activation of the Tie2 receptor on EC as measured by its ability to inhibit both Angiopoitin-1 (Ang1) mediated Tie2 phosphorylation and Ang1 protection of TNF-induced apoptosis. Such antibodies can be developed into function-modifying interfacial biomaterials (IFBMs). The other scfvs exhibited no effects on Tie2 physiology, suggesting that these antibodies may be useful as IFBM affinity modules.

Example 18

Adhesion of Peptide to Polystyrene

The adhesion strength and mode of binding are both IFBM and substrate dependent. To characterize and quantify the adhesion forces between IFBMs and synthetic and biological substrates, a state-of-the-art force spectrometer that employs a high precision, piezo driven flexure stage equipped with a capacitive displacement sensor with a position resolution of about 0.5 nm was used. A polystyrene binding peptide from the $X_6YX_6$ peptide library was used (a cysteine-terminated peptide containing the polystyrene-binding domain in the forward direction; CGSSLVGLHSYWSSPFF; SEQ ID NO:117). The cysteine-terminated peptides were then linked to a gold-coated atomic force microscope (AFM) cantilever by incubating the cantilever in a solution of the peptide (1 mg/ml). Pull-off force measurements were carried out in PBS buffer solution on a MultiMode AFM (Digital Instruments, now Veeco Instruments, Inc., Woodbury, New York, United States of America) by repeatedly engaging a polystyrene surface with the modified cantilever tip at a speed of 300 nm/sec. The mean adhesion force for the peptide was approximately 300 pN.

Example 19

Cytophobic Coatings

Once the peptide sequences were identified, automated solid-phase peptide synthesis following standard N-9-fluorenylmethoxycarbonyl (FMOC) protocols were used to produce a polystyrene adhesion peptide (FFPSSWYSHLGVL; SEQ ID NO:18) with a C-terminal polyethylene glycol (PEG) tag (2500 molecular weight PEG). PEG was selected as the cell-repelling segment of an interfacial biomaterial since it is well known to inhibit/prevent cell adhesion and spreading. A 4 cm$^2$ square sample of polystyrene was coated with the non-fouling interfacial biomaterial (1 mg/ml in 90%/10% PBS/DMSO at pH = 7.4; overnight). The IFBM-coated polystyrene was subsequently washed with excess PBS pH 7.4. Contact angle measurements on the corresponding treated and untreated polystyrene confirmed that the interfacial biomaterial coated the surface.

In order to demonstrate that the multi-functional peptide or interfacial biomaterial (IFBM) FFPYSHLGVLSSG-PEG (SEQ ID NO:104) can coat a surface and prevent or reduce cell adhesion, we determined whether adult human dermal fibroblasts (NHDFs) or human umbilical vein endothelial cells (HUVECs) would adhere to IFBM-coated polystyrene. First, a 1.0 mg/ml solution of FFPYSHLGVLSSG-PEG (SEQ ID NO:104) was prepared in water. The solution was added to the wells of a 96 well polystyrene culture plate and incubated at 50°C overnight. The wells were then washed twice with PBS before seeding with 300 µl of either cell type. Human fibroblast and endothelial cells were also seeded on untreated polystyrene (N=3) and peptide (non-pegylated) coated polystyrene (N=3). After overnight incubation at 37°C, the wells were washed 5 times in excess PBS, then fixed in ethanol and stained with eosin Y for cell counting and optical microscopy. Both NHDF and HUVEC cells lose their rounded morphology, spread, and adhere to the untreated control plastic. At higher magnification, marked membrane ruffling is evident. NHDF or HUVECs seeded on the treated polystyrene maintain a round morphology and are not tightly adhered to the surface. Cell counting studies show that adhesion is substantially lessened and the cell number is dramatically reduced when the polystyrene is coated with FFPYSHLGVLSSG-PEG (SEQ ID NO:104).

Example 20

Determination of a Ligand Antigen using the "Sandwich" Technique

Antibody-coated polypropylene tubes (12 mm x 75 mm) are washed three times with 0.9% NaCl containing 0.5% TWEEN®-20 prior to use. To each tube, 200 µl of appropriately diluted antigen standard or unknown sample is added. The tubes are capped and incubated at room temperature for 3 hours. Thereafter, the tubes are aspirated and then washed 3 times with 0.9% NaCl containing 0.5% TWEEN-20® as before. 200 µl of the appropriately diluted biotin labeled antibody is added to each tube, and the tubes are incubated overnight at 4°C. After incubation, the tubes are aspirated and washed 3 times with 0.9% NaCl containing 0.5% TWEEN-20® solution. After washing, 200 µl of an appropriate dilution of HRP labeled avidin is added to each tube, and the tubes are incubated at room temperature for 5-60 minutes, aspirated, and then washed as before. The enzyme activity in the insoluble phase is determined by adding 1 ml of 0.033 M sodium phosphate buffer pH 6.6 containing 5.4 mM o-phenylenediamine dihydrochloride and 0.03% $H_2O_2$ to each tube at timed intervals. When the color intensity is considered suitable (15 to 30 minutes), the enzymatic reaction is terminated and the absorbance is measured at an appropriate wavelength.
When alkaline phosphatase labeled avidin is used in lieu of HRP-labeled avidin, enzyme activity In the insoluble phase Is determined by adding 1 ml of 0.05 M sodium carbonate buffer pH 9.8 containing 1 mg/ml p-nitrophenylphosphate and 1 mM $MgCl_2$. Following an appropriate incubation period, the reaction is terminated with 100 µl 1N NaOH and the absorbance at 400 nm is measured.
Enzyme immunoassays conducted In microtiter plates are performed in essentially the same manner as described above. The enzyme assays are performed using 250 µl of the substrate solution and terminated with 50 µl of 1N NaOH. The color intensity can be estimated qualitatively or determined quantitatively by and spectrophotometric analysis of the contents of each well of the microtiter plate using a 250 µl microcuvette.

References

Andersson L, Blomberg L, Flegel M, Lepsa L, Nilsson B & Verlander M (2000) Large-Scale Synthesis of Peptides. Biopolymers 55:227-250.

Arap W, Pasqualini R & Ruoslahti E (1998) Cancer Treatment by Targeted Drug Delivery to Tumor Vasculature in a Mouse Model. Science 279:377-380.

Ballinger MD, Shyamala V, Forrest LD, Deuter-Reinhard M, Doyle LV, Wang JX, Panganiban-Lustan L, Stratton JR, Apell G, Winter JA, Doyle MV, Rosenberg S & Kavanaugh WM (1999) Semirational Design of a Potent, Artificial Agonist of Fibroblast Growth Factor Receptors. Nat Biotechnol 17:1199-1204.

Bauminger S & Wlichek M (1980) The Use of Carbodiimides in the Preparation of Immunizing Conjugates. Methods Enzymol 70:151-159.

Berenson RJ, Bensinger WI, Kalamasz DF, Heimfeld S, Goffe RA, Berninger RW, Peterson DR, Thompson P & Strong DM (1992) Transplantation of Stem Cells Enriched by Immunoadsorption. Prog Clin Biol Res 377:449-457; discussion 458-449.

Bhatia SK, Teixeira JL, Anderson M, Shriver-Lake LC, Calvert JM, Georger JH, Hickman JJ, Dulcey CS, Schoen PE & Ligler FS (1993) Fabrication of Surfaces Resistant to Protein Adsorption and Application to Two-Dimensional Protein Patterning. Anal Biochem 208:197-205.

Bodanszky M (1993) Principles of Peptide Synthesis, 2nd rev. ed. Springer-Verlag, Berlin; New York.

Bolin DR, Swain AL, Sarabu R, Berthel SJ, Gillespie P, Huby NJ, Makofske R, Orzechowski L, Perrotta A, Toth K, Cooper JP, Jiang N, Falcioni F, Campbell R, Cox D, Gaizband D, Belunis CJ, Vidovic D, Ito K, Crowther R, Kammlott U, Zhang X, Palermo R, Weber D, Guenot J, Nagy Z & Olson GL (2000) Peptide and Peptide Mimetic Inhibitors of Antigen Presentation by HLA- Dr Class II Mhc Molecules. Design, Structure-Activity Relationships, and X-Ray Crystal Structures. J Med Chem 43:2135-2148.

Brenner S & Lerner RA (1992) Encoded Combinatorial Chemistry. Proc Natl Acad Sci U S A 89:5381-5383.

Brown LF, Yeo KT, Berse B, Yeo TK, Senger DR, Dvorak HF & van de Water L (1992) Expression of Vascular Permeability Factor (Vascular Endothelial Growth Factor) by Epidermal Keratinocytes During Wound Healing. J Exp Med 176:1375-1379.

Budavari S (1996) The Merck Index : An Encyclopedia of Chemicals, Drugs, and Biologicals, 12th ed. Merck, Whitehouse Station, New Jersey, United States of America.

Cheng PW (1996) Receptor Ligand-Facilitated Gene Transfer: Enhancement of Liposome-Mediated Gene Transfer and Expression by Transferrin. of Liposome-Mediated Gene Transfer and Expression by Transferrin. Hum Gene Ther 7:275-282.

Collin GR (1999) Decreasing Catheter Colonization through the Use of an Antiseptic- Impregnated Catheter: A Continuous Quality Improvement Project. Chest 115:1632-1640.

Corringer PJ, Weng JH, Ducos B, Durieux C, Boudeau P, Bohme A & Roques BP (1993) Cck-B Agonist or Antagonist Activities of Structurally Hindered and Peptidase-Resistant Boc-Cck4 Derivatives. J Med Chem 36:166-172.

Diamond MP & Decherney AH (1987) Pathogenesis of Adhesion Formation/Reformation: Application to Reproductive Pelvic Surgery. Microsurgery 8:103-107.

di Zerega GS (1993) The Cause and Prevention of Postsurgical Adhesions: A Contemporary Update. Prog Clin Biol Res 381:1-18.

Dobbins BM, Kite P & Wilcox MH (1999) Diagnosis of Central Venous Catheter Related Sepsis - a Critical Look Inside. J Clin Pathol 52:165-172.


European Patent No. 0 439 095

European Patent No. 0 712 621

Faulstich H, Schafer A & Weckauf-Bloching M (1974) Alpha- and Beta-Galactosidases Bound to Nylon Nets. FEBS Lett 48:226-229.

Fields GB & Noble RL (1990) Solid Phase Peptide Synthesis Utilizing 9-Fluorenylmethoxycarbonyl Amino Acids. Int J Pept Protein Res 35:161-214. Garbay-Jaureguiberry C, Ficheux D & Roques BP (1992) Solid Phase Synthesis of Peptides Containing the Non-Hydrolysable Analog of (O)Phosphotyrosine, P(CH2PO3H2)Phe. Application to the Synthesis of 344-357 Sequences of the Beta 2 Adrenergic Receptor. Int J Pept Protein Res 39:523-527.

Goldman CK, Rogers BE, Douglas JT, Sosnowski BA, Ying W, Siegal GP, Baird A, Campain JA & Curiel DT (1997) Targeted Gene Delivery to Kaposi's Sarcoma Cells Via the Fibroblast Growth Factor Receptor. Cancer Res 57: 1447-1451.

Green NM (1975) Avidin. Adv Protein Chem 29:85-133.

Harris LD, Kim BS & Mooney DJ (1998) Open Pore Biodegradable Matrices Formed with Gas Foaming. J Biomed Mater Res 42:396-402.

Hiller Y, Gershoni JM, Bayer EA & Wilchek M (1987) Biotin Binding to Avidin. Oligosaccharide Side Chain Not Required for Ligand Association. Biochem J 248:167-171.

Leach RE & Henry RL (1990) Reduction of Postoperative Adhesions in the Rat Uterine Horn Model with Poloxamer 407. Am J Obstet Gynecol 162:1317-1319.

Linsky CB, Diamond MP, Cunningham T, Constantine B, DeCherney AH & di Zerega GS (1987) Adhesion Reduction in the Rabbit Uterine Horn Model Using an Absorbable Barrier, Tc-7. J Reprod Med 32:17-20.

Lu Z, Murray KS, Van Cleave V, LaVallie ER, Stahl ML & McCoy JM (1995) Expression of Thioredoxin Random Peptide Libraries on the Escherichia Coli Cell Surface as Functional Fusions to Flagellin: A System Designed for Exploring Protein-Protein Interactions. Biotechnology (N Y) 13:366-372.

Manome Y, Abe M, Hagen MF, Fine HA & Kufe DW (1994) Enhancer Sequences of the Df3 Gene Regulate Expression of the Herpes Simplex Virus Thymidine Kinase Gene and Confer Sensitivity of Human Breast Cancer Cells to Ganciclovir. Cancer Res 54:5408-5413.

Marik PE, Abraham G, Careau P, Varon J & Fromm RE, Jr. (1999) The Ex Vivo Antimicrobial Activity and Colonization Rate of Two Antimicrobial-Bonded Central Venous Catheters. Crit Care Med 27:1128-1131.

McOmie JFW (1973) Protective Groups in Organic Chemistry. Plenum Press, London, New York.

Merrifield RB (1969) Solid-Phase Peptide Synthesis. Adv Enzymol Relat Areas Mol Biol 32:221-296.

Mikos AG, Lyman MD, Freed LE & Langer R (1994) Wetting of Poly(L-Lactic Acid) and Poly(Dl-Lactic-Co-Glycolic Acid) Foams for Tissue Culture. Biomaterials 15:55-58.

Mourez M, Kane RS, Mogridge J, Metallo S, Deschatelets P, Sellman BR, Whitesides GM & Collier RJ (2001) Designing a Polyvalent Inhibitor of Anthrax Toxin. Nat Biotechnol 19:958-961.

Nabel (1997), Current Protocols in Human Genetics. John Wiley & Sons, New York, Vol. on CD-ROM.

Neri D, Carnemolla B, Nissim A, Leprini A, Querze G, Balza E, Pini A, Tarli L, Halin C, Neri P, Zardi L & Winter G (1997) Targeting by Affinity-Matured Recombinant Antibody Fragments of an Angiogenesis Associated Fibronectin Isoform. Nat Biotechnol 15:1271-1275.

Nilsson F, Tarli L, Viti F & Neri D (2000) The Use of Phage Display for the Development of Tumour Targeting Agents. Adv Drug Deliv Rev 43:165-196.

Norris JD, Paige LA, Christensen DJ, Chang CY, Huacani MR, Fan D, Hamilton PT, Fowlkes DM & McDonnell DP (1999) Peptide Antagonists of the Human Estrogen Receptor. Science 285:744-746.

Osbourn JK, Derbyshire EJ, Vaughan TJ, Field AW & Johnson KS (1998a) Pathfinder Selection: In Situ Isolation of Novel Antibodies. Immunotechnology 3:293-302.

Osbourn JK, Earnshaw JC, Johnson KS, Parmentier M, Timmermans V & McCafferty J (1998b) Directed Selection of Mip-1 Alpha Neutralizing Ccr5 Antibodies from a Phage Display Human Antibody Library. Nat Biotechnol 16: 778-781.

Paige LA, Christensen DJ, Gron H, Norris JD, Gottlin EB, Padilla KM, Chang CY, Ballas LM, Hamilton PT, McDonnell DP & Fowlkes DM (1999) Estrogen Receptor (ER) Modulators Each Induce Distinct Conformational Changes in ER Alpha and ER Beta. Proc Natl Acad Sci U S A 96:3999-4004.

Park JW, Hong K, Kirpotin DB, Papahadjopoulos D & Benz CC (1997) Immunoiiposomes for Cancer Treatment. Adv Pharmacol 40:399-435.

Pasqualini R, Koivunen E & Ruoslahti- E (1997) Alpha V Integrins as Receptors for Tumor Targeting by Circulating Ligands. Nat Biotechnol 15:542-546.

Pavone V, Di Blasio B, Lombardi A, Maglio O, Isernia C, Pedone C, Benedetti E, Altmann E & Mutter M (1993) Non Coded C Alpha, Alpha-Disubstituted Amino Acids. X-Ray Diffraction Analysis of a Dipeptide Containing (S)-Alpha-Methylserine. Int J Pept Protein Res 41:15-20.

Pomper MG & Port JD (2000) New Techniques in MR Imaging of Brain Tumors. Magn Reson Imaging Clin N Am 8:691-713. Raum T, Gruber R, Riethmuller G & Kufer P (2001) Anti-Self Antibodies Selected from a Human IgD Heavy Chain Repertoire: A Novel Approach to Generate Therapeutic Human Antibodies against Tumor-Associated Differentiation Antigens. Cancer Immunol Immunother 50:141-150.

Rovaris M & Filippi M (2000) The Role of Magnetic Resonance in the Assessment of Multiple Sclerosis. J Neurol Sci 172 Suppl 1:S3-S12.

Rudgers GW & Palzkill T (2001) Protein Minimization by Random Fragmentation and Selection. Protein Eng 14: 487-492.

Ruoslahti E (2000) Targeting Tumor Vasculature with Homing Peptides from Phage Display. Semin Cancer Biol 10: 435-442.

Saltzman WM & Fung LK (1997) Polymeric Implants for Cancer Chemotherapy. Adv Drug Deliv Rev 26:209-230.

Sambrook J & Russell DW (2001) Molecular Cloning : A Laboratory Manual, 3rd ed. Cold Spring Harbor Laboratory Press, Cold Spring Harbor, New York, United States of America.

Sano T & Cantor CR (1991) Expression Vectors for Streptavidin-Containing Chimeric Proteins. Biochem Biophys Res Commun 176:571-577.Saltzman WM & Fung LK (1997) Polymeric Implants for Cancer Chemotherapy. Adv Drug Deliv Rev 26:209-230. Sambrook J & Russell DW (2001) Molecular Cloning : A Laboratory Manual, 3rd ed. Cold Spring Harbor Laboratory Press, Cold Spring Harbor, New York, United States of America. Sano T & Cantor CR (1991) Expression Vectors for Streptavidin-Containing Chimeric Proteins. Biochem Biophys Res Commun 176: 571-577. Schneider CH & Eberle AN (1993) Peptides, 1992 : Proceedings of the Twenty-Second European Peptide Symposium, September 13-19, 1992, Interlaken, Switzerland. Escom, Leiden, The Netherlands. Schröder E & Lübke K (1965) The Peptides. Academic Press, New York, United States of America. Shen T, Weissleder R, Papisov M, Bogdanov A, Jr. & Brady TJ (1993) Monocrystalline Iron Oxide Nanocompounds (Mion): Physicochemical Properties. Magn Reson Med 29:599-604. Shimizu Y, Inoue A, Tomari Y, Suzuki T, Yokogawa T, Nishikawa K & Ueda T (2001) Cell-Free Translation Reconstituted with Purified Components. Nat Biotechnol 19:751-755. Sidhu SS (2000) Phage Display in Pharmaceutical Biotechnology. Curr Opin Biotechnol 11:610-616. Smith GP (1985) Filamentous Fusion Phage: Novel Expression Vectors That Display Cloned Antigens on the Virion Surface. Science 228:1315-1317. Staba MJ, Wickham TJ, Kovesdi I & Hallahan DE (2000) Modifications of the Fiber in Adenovirus Vectors Increase Tropism for Malignant Glioma Models. Cancer Gene Ther7:13-19. Stangel JJ, Nisbet JD, 2nd & Settles H (1984) Formation and Prevention of Postoperative Abdominal Adhesions. J Reprod Med 29:143-156.

Schneider CH & Eberle AN (1993) Peptides, 1992 : Proceedings of the Twenty-Second European Peptide Symposium, September 13-19, 1992, Interlaken, Switzerland. Escom, Leiden, The Netherlands.

Schröder E & Lübke K (1965) The Peptides. Academic Press, New York, United States of America.

Shen T, Weissleder R, Papisov M, Bogdanov A, Jr. & Brady TJ (1993) Monocrystalline Iron Oxide Nanocompounds (Mion): Physicochemical Properties. Magn Reson Med 29:599-604.

Shimizu Y, Inoue A, Tomari Y, Suzuki T, Yokogawa T, Nishikawa K & Ueda T (2001) Cell-Free Translation Reconstituted with Purified Components. Nat Biotechnol 19:751-755.

Sidhu SS (2000) Phage Display in Pharmaceutical Biotechnology. Curr Opin Biotechnol 11:610-616.

Smith GP (1985) Filamentous Fusion Phage: Novel Expression Vectors That Display Cloned Antigens on the Virion Surface. Science 228:1315-1317.

Staba MJ, Wickham TJ, Kovesdi I & Hallahan DE (2000) Modifications of the Fiber in Adenovirus Vectors Increase Tropism for Malignant Glioma Models. Cancer Gene Ther7:13-19.

Stangel JJ, Nisbet JD, 2nd & Settles H (1984) Formation and Prevention of Postoperative Abdominal Adhesions. J Reprod Med 29:143-156.

Steinleitner A, Lambert H, Kazensky C & Cantor B (1991) Poloxamer 407 as an Intraperitoneal Barrier Material for the Prevention of Postsurgical Adhesion Formation and Reformation in Rodent Models for Reproductive Surgery. Obstet Gynecol77:48-52.

Stewart JM & Young JD (1969) Solid Phase Peptide Synthesis. Freeman, San Francisco, California, United States of America. Tung CH, Zhu T, Lackland H & Stein S (1992) An Acridine Amino Acid Derivative for Use in FMOC Peptide Synthesis. Pept Res 5:115-118.

Urge L, Otvos L, Jr., Lang E, Wroblewski K, Laczko I & Hollosi M (1992) FMOC-Protected, Glycosylated Asparagines Potentially Useful as Reagents in the Solid-Phase Synthesis of N-Glycopeptides. Carbohydr Res 235:83-93.

U.S. Patent No. 4,205,058
U.S. Patent No. 4,208,479
U.S. Patent No. 4,228,237
U.S. Patent No. 4,230,797
U.S. Patent No. 4,244,946
U.S. Patent No. 4,253,995
U.S. Patent No. 4,279,992
U.S. Patent No. 4,282,287
U.S. Patent No. 4,298,685
U.S. Patent No. 4,378,224
U.S. Patent No. 4,551,482
U.S. Patent No. 4,839,293
U.S. Patent No. 4,960,423
U.S. Patent No. 5,147,631
U.S. Patent No. 5,088,499
U.S. Patent No. 5,223,409
U.S. Patent No. 5,292,362
U.S. Patent No. 5,490,840
U.S. Patent No. 5,498,538
U.S. Patent No. 5,510,103
U.S. Patent No. 5,512,131
U.S. Patent No. 5,650,489
U.S. Patent No. 5,667,988
U.S. Patent No. 5,578,629
U.S. Patent No. 5,580,717
U.S. Patent No. 5,635,482
U.S. Patent No. 5,651,991
U.S. Patent No. 5,702,892
U.S. Patent No. 5,705,177
U.S. Patent No. 5,714,166
U.S. Patent No. 5,738,996
U.S. Patent No. 5,747,334
U.S. Patent No. 5,756,291
U.S. Patent No. 5,776,748
U.S. Patent No. 5,780,225
U.S. Patent No. 5,811,392
U.S. Patent No. 5,811,512
U.S. Patent No. 5,811,515
U.S. Patent No. 5,817,757
U.S. Patent No. 5,817,879
U.S. Patent No. 5,837,243
U.S. Patent No. 5,840,300
U.S. Patent No. 5,840,479
U.S. Patent No. 5,855,900
U.S. Patent No. 5,856,308

U.S. Patent No. 5,858,410
U.S. Patent No. 5,858,670
U.S. Patent No. 5,874,542
U.S. Patent No. 5,922,254
U.S. Patent No. 5,922,545
U.S. Patent No. 5,948,635
U.S. Patent No. 5,952,087
U.S. Patent No. 5,977,322
U.S. Patent No. 6,015,561
U.S. Patent No. 6,015,881
U.S. Patent No. 6,031,071
U.S. Patent No. 6,048,623
U.S. Patent No. 6,057,098
U.S. Patent No. 6,060,582
U.S. Patent No. 6,068,829
U.S. Patent No. 6,071,890
U.S. Patent No. 6,107,059
U.S. Patent No. 6,140,127
U.S. Patent No. 6,156,511
U.S. Patent No. 6,168,912
U.S. Patent No. 6,174,708
Us. Patent No. 6,180,084
U.S. Patent No. 6,180,239
U.S. Patent No. 6,180,348
U.S. Patent No. 6,180,610
U.S. Patent No. 6,184,344
U.S. Patent No. 6,197,333
U.S. Patent No. 6,200,598
U.S. Patent No. 6,214,375
U.S. Patent No. 6,214,553
U.S. Patent No. 6,221,018
U.S. Patent No. 6,225,447
U.S. Patent No. 6,231,834
U.S. Patent No. 6,245,318
U.S. Patent No. 6,254,852
U.S. Patent No. 6,280,760
Weissleder R, Bogdanov A & Papisov M (1992) Drug Targeting in Magnetic Resonance Imaging. Magn Reson Q 8:55-63.
Whaley SR, English DS, Hu EL, Barbara PF & Belcher AM (2000) Selection of Peptides with Semiconductor Binding Specificity for Directed Nanocrystal Assembly. Nature 405:665-668.
Wickham TJ, Carrion ME & Kovesdi I (1995) Targeting of Adenovirus Penton Base to New Receptors through Replacement of Its Rgd Motif with Other Receptor-Specific Peptide Motifs. Gene Ther 2:750-756.
Wilchek M & Bayer EA (1990) Introduction to Avidin-Biotin Technology. Methods Enzymol 184:5-13.
WO 00/56375
WO 01/09611
WO 98/10795 Wolfe SA, Ramm EI & Pabo CO (2000) Combining Structure-Based Design with Phage Display to Create New Cys(2)His(2) Zinc Finger Dimers. Structure Fold Des 8:739-750.
Yamabhai M & Kay BK (2001) Mapping Protein-Protein Interactions with Alkaline Phosphatase Fusion Proteins. Methods Enzymol 332:88-102.

**Claims**

1. An interfacial biomaterial comprising a plurality of binding agents, wherein each binding agent comprises a first ligand that specifically binds a target non-biological substrate, and a second ligand that specifically binds a target biological substrate, wherein each binding agent comprises two or more binding specificities, wherein the plurality of binding agents are capable of defining an interface between the target non-biological substrate and the target biological substrate, and wherein the first ligand comprises a peptide and the second ligand comprises a peptide.

2. The interfacial biomaterial of claim 1, wherein the plurality of binding agents comprises a plurality of identical binding agents or non-identical binding agents.

3. The interfacial bio-material of claim 1, further comprising a linker, wherein the linker links a first ligand and a second ligand.

4. The interfacial biomaterial of claim 1, wherein the target non-biological substrate comprises a substrate selected from a synthetic polymer, a plastic, a metal, a metal oxide, a non-metal oxide, a silicone material, a ceramic material, a drug, a drug carrier, and combinations thereof.

5. The interfacial biomaterial of claim 1, wherein the target biological substrate comprises a substrate being selected from a tissue, a cell, a macromolecule, and combinations thereof.

6. The interfacial biomaterial of claim 1, wherein the first ligand comprises a peptide comprising an amino acid sequence selected from any one of SEQ ID NOs: 1-71.

7. The interfacial biomaterial of claim 1, wherein the first ligand binds to a metal comprising titanium, stainless steel, and a combination thereof.

8. The interfacial biomaterial of claim 1, wherein the first ligand binds to a plastic comprising polystyrene, polycarbonate, polyurethane, and a combination thereof.

9. A method of preparing an interfacial biomaterial according to claim 1, said method comprising:

   (a) contacting a non-biological substrate with a plurality of binding agents, wherein each of the plurality of binding agents comprises a first ligand that specifically binds to the non-biological substrate and a second ligand that specifically binds a target biological substrate, and wherein the plurality of binding agents become bound to the non-biological substrate via a binding specificity of the plurality of binding agents for the non-biological substrate;
   (b) contacting the non-biological substrate, having a plurality of binding agents bound thereto, with a sample comprising the target biological substrate; and
   (c) allowing a time sufficient for binding of the target biological substrate to the plurality of binding agents via a binding specificity of the plurality of binding agents for the target biological substrate.

10. A method of applying an interfacial biomaterial of any one of claims 1 to 8, the method comprising:

    (a) contacting a non-biological substrate with a plurality of binding agents, wherein each of the plurality of binding agents comprises a first ligand that specifically binds to the non-biological substrate and a second ligand that specifically binds a target biological substrate, and wherein the plurality of binding agents become bound to the non-biological substrate via a binding specificity of the plurality of binding agents for the non-biological substrate;
    (b) contacting the non-biological substrate, having a plurality of binding agents bound thereto, with a sample comprising the target biological substrate; and
    (c) allowing a time sufficient for binding of the target biological substrate to the plurality of binding agents via a binding specificity of the plurality of binding agents for the target biological substrate.

11. A method according to claim 10, wherein said method is for cell culture, wherein said target biological substrate is cells, and which method further comprises (d) culturing the cells.

12. The method according to claim 10, wherein the interfacial material is applied in a pattern to form a bioarray.

13. The method according to claim 12, wherein the applying comprises a dip-pen printing.

**14.** A biological array prepared by a method according to any one of claim 12, and claim 13.

**15.** An interfacial biomaterial of any one of claims 1 to 8, for use in a method comprising:

>    (a) contacting a non-biological substrate with a plurality of binding agents, wherein each of the plurality of binding agents comprises a first ligand that specifically binds to the non-biological substrate and a second ligand that specifically binds a target biological substrate, and wherein the plurality of binding agents become bound to the non-biological substrate via a binding specificity of the plurality of binding agents for the non-biological substrate;
>    (b) contactinq the non-biological substrate, having a plurality of binding agents bound thereto, with a sample comprising the target biological substrate; and
>    (c) allowing a time sufficient for binding of the target biological substrate to the plurality of binding agents via a binding specificity of the plurality of binding agents for the target biological substrate;

>    wherein said method is for applying an interfacial biomaterial to a device comprising an implant, wherein said non-biological substrate is comprised in an implant, and wherein said target biological substrate is cells; and wherein in step (b) the implant, having a plurality of binding agents bound thereto, is contacted with cells, promoting the attachment of cells to the plurality binding agents on the implant.

**16.** A non-biological substrate comprising a plurality of binding agents, wherein each of the plurality of binding agents comprises a first ligand that specifically binds the non-biological substrate and a second ligand that specifically binds cells, wherein the first ligand comprises a peptide and the second ligand comprises a peptide, wherein the plurality of binding agents become bound to the non-biological substrate via a binding specificity of the plurality of binding agents for the non-biological substrate in forming a coated non-biological substrate; for use in a method of modulating activity of a biological substrate, said use comprising:

>    (a) placing the coated non-biological substrate at a target site, wherein biological substrate is present at the target site; and
>    (b) allowing a time sufficient for binding of biological substrate to a plurality of binding agents on the coated non-biological substrate at a target site, wherein the binding modulates the activity of the biological substrate.

**17.** The substrate of claim 16, wherein the activity modulated comprises angiogenesis or wound healing.

**18.** The method or substrate according to any one of claims 10, 12, 13, 16 or 17, wherein the biological substrate is selected from the group consisting of a tissue, a cell, a macromolécule, and combinations thereof.

**19.** The method or substrate according to any one of claims 9 to 17, wherein a linker is used to link a first ligand to a second ligand.

**20.** A non-biological drug, or a non-biological carrier of the drug comprising a plurality of binding agents, wherein each of the plurality of binding agents comprises a first ligand that specifically binds the drug or the drug carrier and a second ligand that specifically binds a target cell, wherein the first ligand comprises a peptide and the second ligand comprises a peptide,
for use in a method for drug administration to a subject, the use comprising:

>    (a) administering the drug to a subject; and
>    (b) allowing a sufficient time for binding of the plurality of binding agents to the target cell.

**Patentansprüche**

**1.** Grenzflächen-Biomaterial, umfassend eine Mehrzahl von Bindemitteln, wobei jedes Bindemittel einen ersten Liganden, der spezifisch an ein nicht-biologisches Zielsubstrat bindet, und einen zweiten Liganden, der spezifisch an ein biologisches Zielsubstrat bindet, umfasst, wobei jedes Bindemittel zwei oder mehr Bindungsspezifitäten aufweist, wobei die Mehrzahl von Bindemitteln zur Definition einer Grenzfläche zwischen dem nicht-biologischen Zielsubstrat und dem biologischen Zielsubstrat befähigt ist und wobei der erste Ligand ein Peptid umfasst und der zweite Ligand ein Peptid umfasst.

**2.** Grenzflächen-Biomaterial nach Anspruch 1, wobei die Mehrzahl von Bindemitteln eine Mehrzahl von identischen Bindemitteln oder nichtidentischen Bindemitteln umfasst.

3. Grenzflächen-Biomaterial nach Anspruch 1, ferner umfassend einen Linker, wobei der Linker einen ersten Liganden und einen zweiten Liganden verknüpft.

4. Grenzflächen-Biomaterial nach Anspruch 1, wobei das nicht-biologische Zielsubstrat ein Substrat umfasst, das aus einem synthetischen Polymeren, einem Kunststoff, einem Metall, einem Metalloxid, einem Nichtmetalloxid, einem Siliconmaterial, einem Keramikmaterial, einem Arzneistoff, einem Arzneistoffträger und Kombinationen davon ausgewählt ist.

5. Grenzflächen-Biomaterial nach Anspruch 1, wobei das biologische Zielsubstrat ein Substrat umfasst, das aus einem Gewebe, einer Zelle, einem Makromolekül und Kombinationen davon ausgewählt ist.

6. Grenzflächen-Biomaterial nach Anspruch 1, wobei der erste Ligand ein Peptid umfasst, das eine Aminosäuresequenz umfasst, die aus einer der Sequenzen SEQ ID NOs:1-71 ausgewählt ist.

7. Grenzflächen-Biomaterial nach Anspruch 1, wobei der erste Ligand an ein Metall bindet, das Titan, rostfreien Stahl und eine Kombination davon umfasst.

8. Grenzflächen-Biomaterial nach Anspruch 1, wobei der erste Ligand an einen Kunststoff bindet, der Polystyrol, Polycarbonat, Polyurethan und eine Kombination davon umfasst.

9. Verfahren zur Herstellung eines Grenzflächen-Biomaterials nach Anspruch 1, wobei das Verfahren folgendes umfasst:

(a) man bringt ein nicht-biologisches Substrat mit einer Mehrzahl von Bindemitteln in Kontakt, wobei jedes der Mehrzahl von Bindemitteln einen ersten Liganden, der spezifisch an das nicht-biologische Substrat bindet, und einen zweiten Liganden, der spezifisch an ein biologisches Zielsubstrat bindet, umfasst und wobei die Mehrzahl von Bindemitteln über eine Bindungsspezifität der Mehrzahl von Bindemitteln für das nicht-biologische Substrat an das nicht-biologische Substrat gebunden wird;
(b) man bringt das nicht-biologische Substrat, an das eine Mehrzahl von Bindemitteln gebunden ist, mit einer Probe in Kontakt, die das biologische Zielsubstrat umfasst; und
(c) man läßt eine ausreichende Zeitspanne zur Bindung des biologischen Zielsubstrats an die Mehrzahl von Bindemitteln über die Bindungsspezifität der Mehrzahl von Bindemitteln für das biologische Zielsubstrat verstreichen.

10. Verfahren zum Auftragen eines Grenzflächen-Biomaterials nach einem der Ansprüche 1 bis 8, wobei das Verfahren folgendes umfasst:

(a) man bringt ein nicht-biologisches Substrat mit einer Mehrzahl von Bindemitteln in Kontakt, wobei jedes der Mehrzahl von Bindemitteln einen ersten Liganden, der spezifisch an das nicht-biologische Substrat bindet, und einen zweiten Liganden, der spezifisch an ein biologisches Zielsubstrat bindet, umfasst und wobei die Mehrzahl von Bindemitteln über eine Bindungsspezifität der Mehrzahl von Bindemitteln für das nicht-biologische Substrat an das nicht-biologische Substrat gebunden wird;
(b) man bringt das nicht-biologische Substrat, an das eine Mehrzahl von Bindemitteln gebunden ist, mit einer Probe in Kontakt, die das biologische Zielsubstrat umfasst; und
(c) man läßt eine ausreichende Zeitspanne zur Bindung des biologischen Zielsubstrats an die Mehrzahl von Bindemitteln über die Bindungsspezifität der Mehrzahl von Bindemitteln für das biologische Zielsubstrat verstreichen.

11. Verfahren nach Anspruch 10, wobei das Verfahren für die Zellkultur dient, wobei es sich beim biologischen Zielsubstrat um Zellen handelt, und wobei das Verfahren ferner (d) das Züchten der Zellen umfasst.

12. Verfahren nach Anspruch 10, wobei das Grenzflächen-Material in einem Muster aufgetragen wird, um ein Bioarray zu bilden.

13. Verfahren nach Anspruch 12, wobei das Auftragen einen Dip-Pen-Druckvorgang umfasst.

**14.** Biologisches Array, hergestellt nach einem Verfahren gemäß einem der Ansprüche 12 und 13.

**15.** Grenzflächen-Biomaterial nach einem der Ansprüche 1 bis 8 zur Verwendung in einem Verfahren, das folgendes umfasst:

(a) man bringt ein nicht-biologisches Substrat mit einer Mehrzahl von Bindemitteln in Kontakt, wobei jedes der Mehrzahl von Bindemitteln einen ersten Liganden, der spezifisch an das nicht-biologische Substrat bindet, und einen zweiten Liganden, der spezifisch an ein biologisches Zielsubstrat bindet, umfasst und wobei die Mehrzahl von Bindemitteln über eine Bindungsspezifität der Mehrzahl von Bindemitteln für das nicht-biologische Substrat an das nicht-biologische Substrat gebunden wird;
(b) man bringt das nicht-biologische Substrat, an das eine Mehrzahl von Bindemitteln gebunden ist, mit einer Probe in Kontakt, die das biologische Zielsubstrat umfasst; und
(c) man läßt eine ausreichende Zeitspanne zur Bindung des biologischen Zielsubstrats an die Mehrzahl von Bindemitteln über die Bindungsspezifität der Mehrzahl von Bindemitteln für das biologische Zielsubstrat verstreichen;

wobei das Verfahren zum Auftragen eines Grenzflächen-Biomaterials auf eine Vorrichtung, die ein Implantat umfasst, dient, wobei das nicht-biologische Substrat in einem Implantat enthalten ist, und wobei es sich beim biologischen Zielsubstrat um Zellen handelt; und wobei in Stufe (b) das Implantat, an das eine Mehrzahl von Bindemitteln gebunden ist, mit Zellen in Kontakt gebracht wird, wodurch das Anhaften der Zellen an der Mehrzahl von Bindemitteln am Implantat gefördert wird.

**16.** Nicht-biologisches Substrat, umfassend eine Mehrzahl von Bindemitteln, wobei jedes der Mehrzahl von Bindemitteln einen ersten Liganden, der spezifisch das nicht-biologische Substrat bindet und einen zweiten Liganden, der spezifische Zellen bindet, umfasst, wobei der erste Ligand ein Peptid umfasst und der zweite Ligand ein Peptid umfasst, wobei die Mehrzahl von Bindemitteln an das nicht-biologische Substrat über eine Bindungsspezifität der Mehrzahl von Bindemitteln für das nicht-biologische Substrat bei der Bildung eines beschichteten, nicht-biologischen Substrats gebunden wird;
zur Verwendung in einem Verfahren zur Modulation der Aktivität eines biologischen Substrats, wobei die Verwendung folgendes umfasst:

(a) man bringt das beschichtete, nicht-biologische Substrat an eine Zielstelle, wobei das biologische Substrat an der Zielstelle vorhanden ist; und
(b) man läßt eine ausreichende Zeitspanne verstreichen, um das biologische Substrat an eine Mehrzahl von Bindemitteln am beschichteten, nicht-biologischen Substrat an einer Zielstelle zu binden, wobei die Bindung die Aktivität des biologischen Substrats moduliert.

**17.** Substrat nach Anspruch 16, wobei die modulierte Aktivität Angiogenese oder Wundheilung umfasst.

**18.** Verfahren oder Substrat nach einem der Ansprüche 10, 12, 13, 16 oder 17, wobei das biologische Substrat aus der Gruppe ausgewählt wird, die aus einem Gewebe, einer Zelle, einem Makromolekül und Kombinationen davon besteht.

**19.** Verfahren oder Substrat nach einem der Ansprüche 9 bis 17, wobei ein Linker verwendet wird, um einen ersten Liganden mit einem zweiten Liganden zu verknüpfen.

**20.** Nicht-biologischer Arzneistoff oder nicht-biologischer Träger des Arzneistoffes, umfassend eine Mehrzahl von Bindemitteln, wobei jedes der Mehrzahl von Bindemitteln einen ersten Liganden, der spezifisch den Arzneistoff oder den Arzneistoffträger bindet, und einen zweiten Liganden, der spezifisch eine Zielzelle bindet, umfasst, wobei der erste Ligand ein Peptid umfasst und der zweite Ligand ein Peptid umfasst, zur Verwendung in einem Verfahren zur Arzneistoffverabreichung an ein Subjekt, wobei das Verfahren folgendes umfasst:

(a) man verabreicht den Arzneistoff einem Subjekt; und
(b) man läßt eine ausreichende Zeitspanne verstreichen, um die Mehrzahl von Bindemitteln an die Zielzelle zu binden.

**EP 1 527 341 B1**

**Revendications**

1. Biomatériau interfacial, comprenant une pluralité d'agents liants, dans lequel chaque agent liant comprend un premier ligand qui se lie de manière spécifique à un substrat non biologique cible, et un second ligand qui se lie de manière spécifique à un substrat biologique cible, dans lequel chaque agent liant comprend deux spécificités de liaison ou plus, dans lequel la pluralité d'agents liants est capable de définir une interface entre le substrat non biologique cible et le substrat biologique cible, et dans lequel le premier ligand comprend un peptide et le second ligand comprend un peptide.

2. Biomatériau interfacial selon la revendication 1, dans lequel la pluralité d'agents liants comprend une pluralité d'agents liants identiques ou d'agents liants non identiques.

3. Biomatériau interfacial selon la revendication 1, comprenant en outre un lieur, dans lequel le lieur lie un premier ligand et un second ligand.

4. Biomatériau interfacial selon la revendication 1, dans lequel le substrat non biologique cible comprend un substrat sélectionné parmi un polymère synthétique, un plastique, un métal, un oxyde métallique, un oxyde non métallique, un matériau siliconé, un matériau céramique, un médicament, un véhicule de médicament et des combinaisons de ceux-ci.

5. Biomatériau interfacial selon la revendication 1, dans lequel le substrat biologique cible comprend un substrat qui est sélectionné parmi un tissu, une cellule, une macromolécule et des combinaisons de ceux-ci.

6. Biomatériau interfacial selon la revendication 1, dans lequel le premier ligand comprend un peptide comprenant une séquence d'acides aminés sélectionnée parmi l'une quelconque des séquences SEQ ID N° 1 à 71.

7. Biomatériau interfacial selon la revendication 1, dans lequel le premier ligand se lie à un métal comprenant le titane, l'acier inoxydable et une combinaison de ceux-ci.

8. Biomatériau interfacial selon la revendication 1, dans lequel le premier ligand se lie à un plastique comprenant le polystyrène, le polycarbonate, le polyuréthanne et une combinaison de ceux-ci.

9. Procédé de préparation d'un biomatériau interfacial selon la revendication 1, ledit procédé comprenant les étapes consistant à :

(a) mettre en contact un substrat non biologique avec une pluralité d'agents liants, dans lequel chacun de la pluralité d'agents liants comprend un premier ligand qui se lie de manière spécifique au substrat non biologique et un second ligand qui se lie de manière spécifique à un substrat biologique cible, et dans lequel la pluralité d'agents liants devient liée au substrat non biologique via une spécificité de liaison de la pluralité d'agents liants pour le substrat non biologique ;
(b) mettre en contact le substrat non biologique, auquel une pluralité d'agents liants est liée, avec un échantillon comprenant le substrat biologique cible ; et
(c) accorder un temps suffisant pour la liaison du substrat biologique cible avec la pluralité d'agents liants via une spécificité de liaison de la pluralité d'agents liants pour le substrat biologique cible.

10. Procédé d'application d'un biomatériau interfacial selon l'une quelconque des revendications 1 à 8, le procédé comprenant les étapes consistant à :

(a) mettre en contact un substrat non biologique avec une pluralité d'agents liants, dans lequel chaque agent de la pluralité d'agents liants comprend un premier ligand qui se lie de manière spécifique au substrat non biologique et un second ligand qui se lie de manière spécifique à un substrat biologique cible et dans lequel la pluralité d'agents de liaison devient liée au substrat non biologique via une spécificité de liaison de la pluralité d'agents liants pour le substrat non biologique ;
(b) mettre en contact le substrat non biologique, auquel une pluralité d'agents liants est liée, avec un échantillon comprenant le substrat biologique cible ; et
(c) accorder un temps suffisant à la liaison du substrat biologique cible avec la pluralité d'agents liants via une spécificité de liaison de la pluralité d'agents liants pour le substrat biologique cible.

**11.** Procédé selon la revendication 10, dans lequel ledit procédé sert à la culture de cellules, dans lequel ledit substrat biologique cible est constitué de cellules, ledit procédé comprenant en outre (d) la culture des cellules.

**12.** Procédé selon la revendication 10, dans lequel le matériau interfacial est appliqué dans une configuration pour former un réseau biologique.

**13.** Procédé selon la revendication 12, dans lequel l'application comprend une nano-impression (lithographie "dip-pen").

**14.** Réseau biologique préparé par un procédé selon l'une quelconque des revendications 12 et 13.

**15.** Biomatériau interfacial selon l'une quelconque des revendications 1 à 8 pour usage dans un procédé comprenant les étapes consistant à :

(a) mettre en contact un substrat non biologique avec une pluralité d'agents liants, dans lequel chacun de la pluralité d'agents liants comprend un premier ligand qui se lie de manière spécifique au substrat non biologique et un second ligand qui se lie de manière spécifique à un substrat biologique cible, et dans lequel la pluralité d'agents de liaison devient liée au substrat non biologique via une spécificité de liaison de la pluralité d'agents liants pour le substrat non biologique ;
(b) mettre en contact le substrat non biologique, auquel une pluralité d'agents liants est liée, avec un échantillon comprenant le substrat biologique cible ; et
(c) accorder un temps suffisant pour la liaison du substrat biologique cible avec la pluralité d'agents liants via une spécificité de liaison de la pluralité d'agents liants pour le substrat biologique cible ;

dans lequel ledit procédé sert à appliquer un biomatériau interfacial à un dispositif comprenant un implant, dans lequel ledit substrat non biologique est compris dans un implant, et dans lequel ledit substrat biologique cible est constitué de cellules ; et dans lequel, à l'étape (b), l'implant, auquel est liée une pluralité d'agents liants, est mis en contact avec des cellules, favorisant la fixation de cellules à la pluralité d'agents liants sur l'implant.

**16.** Substrat non biologique comprenant une pluralité d'agents liants, dans lequel chacun de la pluralité d'agents liants comprend un premier ligand qui se lie de manière spécifique au substrat non biologique et un second ligand qui se lie de manière spécifique à des cellules, dans lequel le premier ligand comprend un peptide et le second ligand comprend un peptide, dans lequel la pluralité d'agents liants devient liée au substrat non biologique via une spécificité de liaison de la pluralité d'agents liants pour le substrat non biologique en formant un substrat non biologique revêtu ; pour usage dans un procédé de modulation d'activité d'un substrat biologique, ladite utilisation comprenant les étapes consistant à :

(a) placer le substrat non biologique revêtu sur un site cible, dans lequel le substrat biologique est présent sur le site cible ; et
(b) accorder un temps suffisant pour la liaison du substrat biologique avec une pluralité d'agents liants sur le substrat non biologique revêtu sur le site cible, dans lequel la liaison module l'activité du substrat biologique.

**17.** Substrat selon la revendication 16, dans lequel l'activité modulée comprend l'angiogenèse ou la cicatrisation de blessures.

**18.** Procédé ou substrat selon l'une quelconque des revendications 10, 12, 13, 16 ou 17, dans lequel le substrat biologique est sélectionné dans le groupe constitué d'un tissu, d'une cellule, d'une macromolécule et de leurs combinaisons.

**19.** Procédé ou substrat selon l'une quelconque des revendications 9 à 17, dans lequel on utilise un lieur pour lier un premier ligand à un second ligand.

**20.** Médicament non biologique ou véhicule non biologique du médicament comprenant une pluralité d'agents liants, dans lequel chacun de la pluralité d'agents liants comprend un premier ligand qui se lie de manière spécifique au médicament ou au véhicule de médicament et un second ligand qui se lie de manière spécifique à une cellule cible, dans lequel le premier ligand comprend un peptide et le second ligand comprend un peptide, pour usage dans un procédé d'administration d'un médicament à un sujet, l'utilisation comprenant les étapes consistant à :

(a) administrer le médicament à un sujet ; et

(b) accorder un temps suffisant pour la liaison de la pluralité d'agents liants à la cellule cible.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5856308 A
- US 5635482 A
- US 5292362 A
- US 6280760 B
- US 6140127 B
- US 4960423 B
- US 4378224 B
- US 5948635 A, Kay
- US 5948DI35 A, Kay
- US 6180239 B
- US 6048623 B
- US 5512131 A
- US 5776748 A
- WO 0056375 A
- US 5651991 A
- US 5858410 A
- US 4551482 A
- US 5714166 A
- US 5510103 A
- US 5490840 A
- US 5855900 A
- US 6214375 B
- US 6200598 B
- US 6197333 B
- US 5922545 A
- US 6015561 A
- US 6015881 A
- US 6031071 A
- US 4244946 A
- US 6184344 B
- US 5811392 A
- US 5811512 A
- US 5578629 A
- US 5817879 A
- US 5817757 A
- US 5811515 A
- US 5874542 A, Rockwell
- US 5977322 A
- US 5837243 A
- US 5840300 A
- US 5952087 A
- US 5705177 A
- US 4378224 A
- US 6071890 A
- EP 0439095 A
- EP 0712621 A
- US 6156511 A
- US 6107059 A
- US 5223409 A

- US 5650489 A
- US 5858670 A
- US 6180348 B
- US 5756291 B
- US 6168912 B
- US 5738996 B
- US 6174708 B
- US 6057098 A
- US 5922254 A
- US 5840479 A
- US 5780225 A
- US 5702892 A
- US 5667988 A
- US 6214553 B
- US 5747334 A
- US 5498538 A
- US 5264563 A
- US 5824483 A
- US 6225447 B
- US 5580717 B
- US 5702892 B
- US 6068829 A
- US 6086829 A
- US 6060582 A
- US 6180084 A
- WO 9810795 A
- WO 0109611 A
- US 6245318 B
- US 6231834 B
- US 6221018 B
- US 5088499 B
- US 6254852 B
- US 5147631 B
- US 5928627 A
- US 4839293 A
- US 6429015 B
- US 6428955 B
- US 4298685 A, Parikh
- US 4282287 A
- US 4279992 A
- US 4253995 A
- US 4230797 A
- US 4228237 A
- US 4208479 A
- US 4205058 A
- US 4960423 A
- US 5147631 A
- US 5088499 A
- US 5580717 A

- US 5738996 A
- US 5756291 A
- US 6048623 A
- US 6140127 A

- US 6180084 B
- US 6180610 B
- US 60331843 B

**Non-patent literature cited in the description**

- **Schneider ; Eberle.** *Peptides,* 1992
- *Proceedings of the Twenty-Second European Peptide Symposium,* 13 September 1992
- **Stewart ; Young.** Solid Phase Peptide Synthesis. Freeman, San Francisco, 1969
- **Merrifield.** *Adv Enzymol Relat Areas Mol Biol,* 1969, vol. 32, 221-296
- **Fields ; Noble.** *Int J Pept Protein Res,* 1990, vol. 35, 161-214
- **Bodanszky.** Principles of Peptide Synthesis. Springer-Verlag, 1993
- **Andersson et al.** *Biopolymers,* 2000, vol. 55, 227-250
- **Schröder ; Lübke.** The Peptides. Academic Press, 1965
- **McOmie.** Protective Groups in Organic Chemistry. Plenum Press, 1973
- **Shimizu et al.** *Nat Biotechnol,* 2001, vol. 19, 751-755
- **Budavari.** The Merck Index: An Encyclopedia of Chemicals, Drugs, and Biologicals. 1996
- **Sambrook ; Russell.** Molecular Cloning: A Laboratory Manual. Cold Spring Harbor Laboratory Press, Cold Spring Harbor, 2001
- **Arap et al.** *Science,* 1998, vol. 279, 377-380
- **Staba et al.** *Cancer Gene Ther,* 2000, vol. 7, 13-19
- **Wickham et al.** *Gene Ther,* 1995, vol. 2, 750-756
- **Clark et al.** *J Am Chem Soc,* 2001, vol. 123, 7677-7682
- **Andersson L ; Blomberg L ; Flegel M ; Lepsa L ; Nilsson B ; Verlander M.** Large-Scale Synthesis of Peptides. *Biopolymers,* 2000, vol. 55, 227-250
- **Arap W ; Pasqualini R ; Ruoslahti E.** Cancer Treatment by Targeted Drug Delivery to Tumor Vasculature in a Mouse Model. *Science,* 1998, vol. 279, 377-380
- **Ballinger MD ; Shyamala V ; Forrest LD ; Deuter-Reinhard M ; Doyle LV ; Wang JX ; Panganiban-Lustan L ; Stratton JR ; Apell G ; Winter JA.** Semirational Design of a Potent, Artificial Agonist of Fibroblast Growth Factor Receptors. *Nat Biotechnol,* 1999, vol. 17, 1199-1204
- **Bauminger S ; Wlichek M.** The Use of Carbodiimides in the Preparation of Immunizing Conjugates. *Methods Enzymol,* 1980, vol. 70, 151-159

- **Berenson RJ ; Bensinger WI ; Kalamasz DF ; Heimfeld S ; Goffe RA ; Berninger RW ; Peterson DR ; Thompson P ; Strong DM.** Transplantation of Stem Cells Enriched by Immunoadsorption. *Prog Clin Biol Res,* 1992, vol. 377, 449-457458-449
- **Bhatia SK ; Teixeira JL ; Anderson M ; Shriver-Lake LC ; Calvert JM ; Georger JH ; Hickman JJ ; Dulcey CS ; Schoen PE ; Ligler FS.** Fabrication of Surfaces Resistant to Protein Adsorption and Application to Two-Dimensional Protein Patterning. *Anal Biochem,* 1993, vol. 208, 197-205
- **Bodanszky M.** Principles of Peptide Synthesis. Springer-Verlag, 1993
- **Bolin DR ; Swain AL ; Sarabu R ; Berthel SJ ; Gillespie P ; Huby NJ ; Makofske R ; Orzechowski L ; Perrotta A ; Toth K.** Peptide and Peptide Mimetic Inhibitors of Antigen Presentation by HLA- Dr Class II Mhc Molecules. Design, Structure-Activity Relationships, and X-Ray Crystal Structures. *J Med Chem,* 2000, vol. 43, 2135-2148
- **Brenner S ; Lerner RA.** Encoded Combinatorial Chemistry. *Proc Natl Acad Sci U S A,* 1992, vol. 89, 5381-5383
- **Brown LF ; Yeo KT ; Berse B ; Yeo TK ; Senger DR ; Dvorak HF ; van de Water L.** Expression of Vascular Permeability Factor (Vascular Endothelial Growth Factor) by Epidermal Keratinocytes During Wound Healing. *J Exp Med,* 1992, vol. 176, 1375-1379
- **Budavari S.** *The Merck Index : An Encyclopedia of Chemicals, Drugs, and Biologicals,* 1996
- **Cheng PW.** Receptor Ligand-Facilitated Gene Transfer: Enhancement of Liposome-Mediated Gene Transfer and Expression by Transferrin. of Liposome-Mediated Gene Transfer and Expression by Transferrin. *Hum Gene Ther,* 1996, vol. 7, 275-282
- **Collin GR.** Decreasing Catheter Colonization through the Use of an Antiseptic- Impregnated Catheter: A Continuous Quality Improvement Project. *Chest,* 1990, vol. 115, 1632-1640
- **Corringer PJ ; Weng JH ; Ducos B ; Durieux C ; Boudeau P ; Bohme A ; Roques BP.** Cck-B Agonist or Antagonist Activities of Structurally Hindered and Peptidase-Resistant Boc-Cck4 Derivatives. *J Med Chem,* 1993, vol. 36, 166-172
- **Diamond MP ; Decherney AH.** Pathogenesis of Adhesion Formation/Reformation: Application to Reproductive Pelvic Surgery. *Microsurgery,* 1987, vol. 8, 103-107

- **Zerega GS.** The Cause and Prevention of Postsurgical Adhesions: A Contemporary Update. *Prog Clin Biol Res,* 1993, vol. 381, 1-18
- **Dobbins BM ; Kite P ; Wilcox MH.** Diagnosis of Central Venous Catheter Related Sepsis - a Critical Look Inside. *J Clin Pathol,* 1999, vol. 52, 165-172
- **Faulstich H ; Schafer A.** Weckauf-Bloching M (1974) Alpha- and Beta-Galactosidases Bound to Nylon Nets. *FEBS Lett,* 1974, vol. 48, 226-229
- **Fields GB ; Noble RL.** Solid Phase Peptide Synthesis Utilizing 9-Fluorenylmethoxycarbonyl Amino Acids. *Int J Pept Protein Res,* 1990, vol. 35, 161-214
- **Garbay-Jaureguiberry C ; Ficheux D ; Roques BP.** Solid Phase Synthesis of Peptides Containing the Non-Hydrolysable Analog of (O)Phosphotyrosine, P(CH2PO3H2)Phe. Application to the Synthesis of 344-357 Sequences of the Beta 2 Adrenergic Receptor. *Int J Pept Protein Res,* 1992, vol. 39, 523-527
- **Goldman CK ; Rogers BE ; Douglas JT ; Sosnowski BA ; Ying W ; Siegal GP ; Baird A ; Campain JA ; Curiel DT.** Targeted Gene Delivery to Kaposi's Sarcoma Cells Via the Fibroblast Growth Factor Receptor. *Cancer Res,* 1997, vol. 57, 1447-1451
- **Green NM.** Avidin. *Adv Protein Chem,* 1975, vol. 29, 85-133
- **Harris LD ; Kim BS ; Mooney DJ.** Open Pore Biodegradable Matrices Formed with Gas Foaming. *J Biomed Mater Res,* 1998, vol. 42, 396-402
- **Hiller Y ; Gershoni JM ; Bayer EA ; Wilchek M.** Biotin Binding to Avidin. Oligosaccharide Side Chain Not Required for Ligand Association. *Biochem J,* 1987, vol. 248, 167-171
- **Leach RE ; Henry RL.** Reduction of Postoperative Adhesions in the Rat Uterine Horn Model with Poloxamer 407. *Am J Obstet Gynecol,* 1990, vol. 162, 1317-1319
- **Linsky CB ; Diamond MP ; Cunningham T ; Constantine B ; DeCherney AH ; Zerega GS.** Adhesion Reduction in the Rabbit Uterine Horn Model Using an Absorbable Barrier, Tc-7. *J Reprod Med,* 1987, vol. 32, 17-20
- **Lu Z ; Murray KS ; Van Cleave V ; LaVallie ER ; Stahl ML ; McCoy JM.** Expression of Thioredoxin Random Peptide Libraries on the Escherichia Coli Cell Surface as Functional Fusions to Flagellin: A System Designed for Exploring Protein-Protein Interactions. *Biotechnology (N Y),* 1995, vol. 13, 366-372
- **Manome Y ; Abe M ; Hagen MF ; Fine HA ; Kufe DW.** Enhancer Sequences of the Df3 Gene Regulate Expression of the Herpes Simplex Virus Thymidine Kinase Gene and Confer Sensitivity of Human Breast Cancer Cells to Ganciclovir. *Cancer Res,* 1994, vol. 54, 5408-5413
- **Marik PE ; Abraham G ; Careau P ; Varon J ; Fromm RE ; Jr.** The Ex Vivo Antimicrobial Activity and Colonization Rate of Two Antimicrobial-Bonded Central Venous Catheters. *Crit Care Med,* 1999, vol. 27, 1128-1131
- **McOmie JFW.** Protective Groups in Organic Chemistry. Plenum Press, 1973
- **Merrifield RB.** Solid-Phase Peptide Synthesis. *Adv Enzymol Relat Areas Mol Biol,* 1969, vol. 32, 221-296
- **Mikos AG ; Lyman MD ; Freed LE ; Langer R.** Wetting of Poly(L-Lactic Acid) and Poly(DI-Lactic-Co-Glycolic Acid) Foams for Tissue Culture. *Biomaterials,* 1994, vol. 15, 55-58
- **Mourez M ; Kane RS ; Mogridge J ; Metallo S ; Deschatelets P ; Sellman BR ; Whitesides GM ; Collier RJ.** Designing a Polyvalent Inhibitor of Anthrax Toxin. *Nat Biotechnol,* 2001, vol. 19, 958-961
- **Nabel.** Current Protocols in Human Genetics. John Wiley & Sons, 1997
- **Neri D ; Carnemolla B ; Nissim A ; Leprini A ; Querze G ; Balza E ; Pini A ; Tarli L ; Halin C ; Neri P.** Targeting by Affinity-Matured Recombinant Antibody Fragments of an Angiogenesis Associated Fibronectin Isoform. *Nat Biotechnol,* 1997, vol. 15, 1271-1275
- **Nilsson F ; Tarli L ; Viti F ; Neri D.** The Use of Phage Display for the Development of Tumour Targeting Agents. *Adv Drug Deliv Rev,* 2000, vol. 43, 165-196
- **Norris JD ; Paige LA ; Christensen DJ ; Chang CY ; Huacani MR ; Fan D ; Hamilton PT ; Fowlkes DM ; McDonnell DP.** Peptide Antagonists of the Human Estrogen Receptor. *Science,* 1999, vol. 285, 744-746
- **Osbourn JK ; Derbyshire EJ ; Vaughan TJ ; Field AW ; Johnson KS.** Pathfinder Selection: In Situ Isolation of Novel Antibodies. *Immunotechnology,* 1998, vol. 3, 293-302
- **Osbourn JK ; Earnshaw JC ; Johnson KS ; Parmentier M ; Timmermans V ; McCafferty J.** Directed Selection of Mip-1 Alpha Neutralizing Ccr5 Antibodies from a Phage Display Human Antibody Library. *Nat Biotechnol,* 1998, vol. 16, 778-781
- **Paige LA ; Christensen DJ ; Gron H ; Norris JD ; Gottlin EB ; Padilla KM ; Chang CY ; Ballas LM ; Hamilton PT ; McDonnell DP.** Estrogen Receptor (ER) Modulators Each Induce Distinct Conformational Changes in ER Alpha and ER Beta. *Proc Natl Acad Sci U S A,* 1999, vol. 96, 3999-4004
- **Park JW ; Hong K ; Kirpotin DB ; Papahadjopoulos D ; Benz CC.** Immunoiiposomes for Cancer Treatment. *Adv Pharmacol,* 1997, vol. 40, 399-435
- **Pasqualini R ; Koivunen E ; Ruoslahti- E.** Alpha V Integrins as Receptors for Tumor Targeting by Circulating Ligands. *Nat Biotechnol,* 1997, vol. 15, 542-546

- **Pavone V ; Di Blasio B ; Lombardi A ; Maglio O ; Isernia C ; Pedone C ; Benedetti E ; Altmann E ; Mutter M.** Non Coded C Alpha, Alpha-Disubstituted Amino Acids. X-Ray Diffraction Analysis of a Dipeptide Containing (S)-Alpha-Methylserine. *Int J Pept Protein Res,* 1993, vol. 41, 15-20
- **Pomper MG ; Port JD.** New Techniques in MR Imaging of Brain Tumors. *Magn Reson Imaging Clin N Am,* 2000, vol. 8, 691-713
- **Raum T ; Gruber R ; Riethmuller G ; Kufer P.** Anti-Self Antibodies Selected from a Human IgD Heavy Chain Repertoire: A Novel Approach to Generate Therapeutic Human Antibodies against Tumor-Associated Differentiation Antigens. *Cancer Immunol Immunother,* 2001, vol. 50, 141-150
- **Rovaris M ; Filippi M.** The Role of Magnetic Resonance in the Assessment of Multiple Sclerosis. *J Neurol Sci,* 2000, vol. 172 (1), S3-S12
- **Rudgers GW ; Palzkill T.** Protein Minimization by Random Fragmentation and Selection. *Protein Eng,* 2001, vol. 14, 487-492
- **Ruoslahti E.** Targeting Tumor Vasculature with Homing Peptides from Phage Display. *Semin Cancer Biol,* 2000, vol. 10, 435-442
- **Saltzman WM ; Fung LK.** Polymeric Implants for Cancer Chemotherapy. *Adv Drug Deliv Rev,* 1997, vol. 26, 209-230
- **Sambrook J ; Russell DW.** Molecular Cloning : A Laboratory Manual. Cold Spring Harbor Laboratory Press, Cold Spring Harbor, 2001
- **Sano T ; Cantor CR.** Expression Vectors for Streptavidin-Containing Chimeric Proteins. *Biochem Biophys Res Commun,* 1991, vol. 176, 571-577
- **Schneider CH ; Eberle AN.** *Peptides,* 1992
- **Schröder E ; Lübke K.** The Peptides. Academic Press, 1965
- **Shen T ; Weissleder R ; Papisov M ; Bogdanov A ; Jr. ; Brady TJ.** Monocrystalline Iron Oxide Nanocompounds (Mion): Physicochemical Properties. *Magn Reson Med,* 1993, vol. 29, 599-604
- **Shimizu Y ; Inoue A ; Tomari Y ; Suzuki T ; Yokogawa T ; Nishikawa K ; Ueda T.** Cell-Free Translation Reconstituted with Purified Components. *Nat Biotechnol,* 2001, vol. 19, 751-755
- **Sidhu SS.** Phage Display in Pharmaceutical Biotechnology. *Curr Opin Biotechnol,* 2000, vol. 11, 610-616
- **Smith GP.** Filamentous Fusion Phage: Novel Expression Vectors That Display Cloned Antigens on the Virion Surface. *Science,* 1985, vol. 228, 1315-1317
- **Staba MJ ; Wickham TJ ; Kovesdi I ; Hallahan DE.** Modifications of the Fiber in Adenovirus Vectors Increase Tropism for Malignant Glioma Models. *Cancer Gene Ther,* 2000, vol. 7, 13-19
- **Stangel JJ ; Nisbet JD ; Settles H.** Formation and Prevention of Postoperative Abdominal Adhesions. *J Reprod Med,* 1984, vol. 29, 143-156
- **Steinleitner A ; Lambert H ; Kazensky C ; Cantor B.** Poloxamer 407 as an Intraperitoneal Barrier Material for the Prevention of Postsurgical Adhesion Formation and Reformation in Rodent Models for Reproductive Surgery. *Obstet Gynecol,* 1991, vol. 77, 48-52
- **Stewart JM ; Young JD.** Solid Phase Peptide Synthesis. Freeman, San Francisco, 1969
- **Tung CH ; Zhu T ; Lackland H ; Stein S.** An Acridine Amino Acid Derivative for Use in FMOC Peptide Synthesis. *Pept Res,* 1992, vol. 5, 115-118
- **Urge L ; Otvos L ; Jr. ; Lang E ; Wroblewski K ; Laczko I ; Hollosi M.** FMOC-Protected, Glycosylated Asparagines Potentially Useful as Reagents in the Solid-Phase Synthesis of N-Glycopeptides. *Carbohydr Res,* 1992, vol. 235, 83-93
- **Weissleder R ; Bogdanov A ; Papisov M.** Drug Targeting in Magnetic Resonance Imaging. *Magn Reson Q,* 1992, vol. 8, 55-63
- **Whaley SR ; English DS ; Hu EL ; Barbara PF ; Belcher AM.** Selection of Peptides with Semiconductor Binding Specificity for Directed Nanocrystal Assembly. *Nature,* 2000, vol. 405, 665-668
- **Wickham TJ ; Carrion ME ; Kovesdi I.** Targeting of Adenovirus Penton Base to New Receptors through Replacement of Its Rgd Motif with Other Receptor-Specific Peptide Motif. *Gene Ther,* 1995, vol. 2, 750-756
- **Wilchek M ; Bayer EA.** Introduction to Avidin-Biotin Technology. *Methods Enzymol,* 1990, vol. 184, 5-13
- **Wolfe SA ; Ramm EI ; Pabo CO.** Combining Structure-Based Design with Phage Display to Create New Cys(2)His(2) Zinc Finger Dimers. *Structure Fold Des,* 2000, vol. 8, 739-750
- **Yamabhai M ; Kay BK.** Mapping Protein-Protein Interactions with Alkaline Phosphatase Fusion Proteins. *Methods Enzymol,* 2001, vol. 332, 88-102